# EUROPEAN PATENT SPECIFICATION

(11) **EP 2 695 940 B1**
(45) Date of publication and mention of the grant of the patent: **30.11.2016**
(21) Application number: 12767953.8
(22) Date of filing: 30.03.2012
(51) Int. Cl.: C12P 13/12, C12N 9/02, C12N 9/10

(54) **METHOD FOR PRODUCING L-CYSTEINE**
VERFAHREN ZUR HERSTELLUNG VON L-CYSTEIN
PROCÉDÉ POUR LA PRODUCTION DE L-CYSTÉINE

(30) Priority: 01.04.2011 US 201161470573 P; 01.04.2011 JP 2011081981
(43) Date of publication of application: 12.02.2014
(73) Proprietor: Ajinomoto Co., Inc., Tokyo 104-8315 (JP)
(72) Inventor: YAMAZAKI, Shunsuke, Kawasaki-shi Kanagawa 210-8681 (JP); NONAKA, Gen, Kawasaki-shi Kanagawa 210-8681 (JP); TAKUMI, Kazuhiro, Kawasaki-shi Kanagawa 210-8681 (JP)
(74) Representative: Strehl Schübel-Hopf & Partner
(86) International application number: PCT/JP2012/058696
(87) International publication number: WO 2012/137689

(56) References cited:
- EP-A1- 2 479 279
- WO-A1-2007/012078
- WO-A1-2009/104731
- DE-C1- 19 949 579
- JP-A- 2003 511 086
- JP-A- 2009 501 550
- JP-A- 2010 022 215
- JP-A- 2010 187 552
- JP-A- 2010 193 788
- JP-A- 2010 207 194
- US-A1- 2007 026 505
- WARREN M J ET AL: "THE ESCHERICHIA COLI CYSG GENE ENCODES S-ADENOSYLMETHIONINE-DEPENDENT UROPORPHYRINOGEN III METHYLASE", BIOCHEMICAL JOURNAL, PORTLAND PRESS LTD, GB, vol. 265, no. 3, 1 February 1990 (1990-02-01), pages 725-729, XP008014987, ISSN: 0264-6021

### SEQUENCE LISTING

<110> Ajinomoto Co., Inc.
<120> A method for producing L-cysteine
<130> D282-11149
<150> JP2011-081981
   <151> 2011-04-01
<150> US61/470,573
   <151> 2011-04-01
<160> 74
<170> PatentIn version 3.1
<210> 1
   <211> 313
   <212> DNA
   <213> Escherichia coli
<400> 1
   gcatgcttcc aactgcgcta atgacgcagc tggacgaagg cgggattctc gtcttacccg     60
   taggggagga gcaccagtat ttgaaacggg tgcgtcgtcg gggaggcgaa tttattatcg    120
   ataccgtgga ggccgtgcgc tttgtccctt tagtgaaggg tgagctggct taaaacgtga    180
   ggaaatacct ggatttttcc tggttatttt gccgcaggtc agcgtatcgt gaacatcttt    240
   tccagtgttc agtagggtgc cttgcacggt aattatgtca ctggttatta accaattttt    300
   cctgggggtc gac                                                       313
<210> 2
   <211> 313
   <212> DNA
   <213> Escherichia coli
<400> 2
   gcatgcttcc aactgcgcta atgacgcagc tggacgaagg cgggattctc gtcttacccg      60
   taggggagga gcaccagtat ttgaaacggg tgcgtcgtcg gggaggcgaa tttattatcg     120
   ataccgtgga ggccgtgcgc tttgtccctt tagtgaaggg tgagctggct taaaacgtga     180
   ggaaatacct ggatttttcc tggttatttt gccgcaggtc agcgtataat gaagatcttt     240
   tccagtgttc agtagggtgc cttgcacggt tataatgtca ctggttatta accaattttt     300
   cctgggggtc gac                                                        313
<210> 3
   <211> 30
   <212> DNA
   <213> Artificial Sequence
<220>
   <223> CysG Fw(XhoI)
<400> 3
   acgcctcgag atggattatt tgcctctttt 30
<210> 4
   <211> 28
   <212> DNA
   <213> Artificial Sequence
<220>
   <223> CysG Rv(XbaI)
<400> 4
   gctctagatc aagccagatt gacaacgg 28
<210> 5
   <211> 30
   <212> DNA
   <213> Artificial Sequence
<220>
   <223> CysJ Fw(SalI)
<400> 5
   acgcgtcgac atgacgactc aggcaccagg 30
<210> 6
   <211> 28
   <212> DNA
   <213> Artificial Sequence
<220>
   <223> CysI Rv(XbaI)
<400> 6
   gctctagatc attttgcctc ctgccaga 28
<210> 7
   <211> 60
   <212> DNA
   <213> Artificial Sequence
<220>
   <223> CysG-JI Rv
<400> 7
   ctggtgcctg agtcgtcatc gtttttcctc tcaagccaga ttgacaacgg cggactcgcg 60
<210> 8
   <211> 30
   <212> DNA
   <213> Artificial Sequence
<220>
   <223> SalI-cysG(Ec)Fw
<400> 8
   acgcgtcgac atggatcatt tgcctatatt 30
<210> 9
   <211> 30
   <212> DNA
   <213> Artificial Sequence
<220>
   <223> XbaI-cysG(Ec)Rv
<400> 9
   gctctagatt aatggttgga gaaccagttc 30
<210> 10
   <211> 30
   <212> DNA
   <213> Artificial Sequence
<220>
   <223> SalI-cysJ(Ec)Fw
<400> 10
   acgcgtcgac atgacgacac aggtcccacc 30
<210> 11
   <211> 30
   <212> DNA
   <213> Artificial Sequence
<220>
   <223> XbaI-cysI(Ec)Rv
<400> 11
   gctctagatt aatcccacaa atcacgcgcc 30
<210> 12
   <211> 78
   <212> DNA
   <213> Artificial Sequence
<220>
   <223> cysG-JI(Ec)Rv
<400> 12
   caacgcggaa ggtgggacct gtgtcgtcat gcgtcgttat gttccagttt aatggttgga     60
   gaaccagttc agtttatc                                                   78
<210> 13
   <211> 36
   <212> DNA
   <213> Artificial Sequence
<220>
   <223> primer P1
<400> 13
   agctgagtcg acccccagga aaaattggtt aataac 36
<210> 14
   <211> 33
   <212> DNA
   <213> Artificial Sequence
<220>
   <223> primer P2
<400> 14
   agctgagcat gcttccaact gcgctaatga cgc 33
<210> 15
   <211> 33
   <212> DNA
   <213> Artificial Sequence
<220>
   <223> primer P3
<400> 15
   agctgatcta gaaaacagaa tttgcctggc ggc 33
<210> 16
   <211> 33
   <212> DNA
   <213> Artificial Sequence
<220>
   <223> primer P4
<400> 16
   agctgaggat ccaggaagag tttgtagaaa cgc 33
<210> 17
   <211> 32
   <212> DNA
   <213> Artificial Sequence
<220>
   <223> primer P5
<400> 17
   agctgagtcg acgtgttcgc tgaatacggg gt 32
<210> 18
   <211> 32
   <212> DNA
   <213> Artificial Sequence
<220>
   <223> primer P6
<400> 18
   agctgatcta gagaaagcat caggattgca gc 32
<210> 19
   <211> 59
   <212> DNA
   <213> Artificial Sequence
<220>
   <223> primer P7
<220>
   <221> misc_feature
   <222> (25)..(25)
   <223> n is a, c, g, or t
<220>
   <221> misc_feature
   <222> (27)..(28) <223> n is a, c, g, or t
<220>
   <221> misc_feature
   <222> (46).. (46)
   <223> n is a, c, g, or t
<220>
   <221> misc_feature
   <222> (49).. (49)
   <223> n is a, c, g, or t
<220>
   <221> misc_feature
   <222> (51)..(51)
   <223> n is a, c, g, or t
<400> 19
   atcgtgaaga tcttttccag tgttnannag ggtgccttgc acggtnatna ngtcactgg 59
<210> 20
   <211> 32
   <212> DNA
   <213> Artificial Sequence
<220>
   <223> primer P8
<220>
   <221> misc_feature
   <222> (16)..(20)
   <223> n is a, c, g, or t
<400> 20
   tggaaaagat cttcannnnn cgctgacctg cg 32
<210> 21
   <211> 312
   <212> DNA
   <213> Escherichia coli
<400> 21
   gcatgcttcc aactgcgcta atgacgcagc tggacgaagg cgggattctc gtcttacccg 60
   taggggagga gcaccagtat ttgaaacggg tgcgtcgtcg gggaggcgaa tttattatcg 120
   ataccgtgga ggccgtgcgc tttgtccctt tagtgaaggg tgagctggct taaaacgtga 180
   ggaaatacct ggatttttcc tggttatttt gccgcaggtc agcgtataat gaagatcttt 240
   tccagtgttg acaagggtcc ttgcacggtt ataatgtcac tggttattaa ccaatttttc 300
   ctgggggtcg ac 312
<210> 22
   <211> 33
   <212> DNA
   <213> Artificial Sequence
<220>
   <223> ydeD299F
<400> 22
   agctgagtcg acatgtcgcg aaaagatggg gtg 33
<210> 23
   <211> 33
   <212> DNA
   <213> Artificial Sequence
<220>
   <223> ydeD299R
<400> 23
   agctgatcta gagtttgttc tggccccgac atc 33
<210> 24
   <211> 33
   <212> DNA
   <213> Artificial Sequence
<220>
   <223> cysEF
<400> 24
   agctgagtcg acatgtcgtg tgaagaactg gaa 33
<210> 25
   <211> 21
   <212> DNA
   <213> Artificial Sequence
<220>
   <223> cysEX-1
<400> 25
   atcaccgccg cttcaccaac g 21
<210> 26
   <211> 21
   <212> DNA
   <213> Artificial Sequence
<220>
   <223> cysEX-2
<400> 26
   cgttggtgaa gcggcggtga t 21
<210> 27
   <211> 33
   <212> DNA
   <213> Artificial Sequence
<220>
   <223> cysER
<400> 27
   agctgatcta gaatagatga ttacatcgca tcc 33
<210> 28
   <211> 33
   <212> DNA
   <213> Artificial Sequence
<220>
   <223> serA5F
<400> 28
   agctgagtcg acatggcaaa ggtatcgctg gag 33
<210> 29
   <211> 35
   <212> DNA
   <213> Artificial Sequence
<220>
   <223> serA5R
<400> 29
   agctgatcta gattacagca gacgggcgcg aatgg 35
<210> 30
   <211> 34
   <212> DNA
   <213> Artificial Sequence
<220>
   <223> PrOMPAF
<400> 30
   agctgagtcg accgcctcgt tatcatccaa aatc 34
<210> 31
   <211> 33
   <212> DNA
   <213> Artificial Sequence
<220>
   <223> PrOMPAR
<400> 31
   agctgagcat gcactaattt tccttgcgga ggc 33
<210> 32
   <211> 43
   <212> DNA
   <213> Artificial Sequence
<220>
   <223> primer P9
<400> 32
   agctgaaagc ttgcatgcac gcgtggcgat ctggcctgac tgc 43
<210> 33
   <211> 39
   <212> DNA
   <213> Artificial Sequence
<220>
   <223> primer P10
<400> 33
   agctgagtcg accccgtggt ggcaaccttt aaaaaactg 39
<210> 34
   <211> 192
   <212> DNA
   <213> Pantoea ananatis
<400> 34
   gcatgcacgc gtggcgatct ggcctgactg ccttgttagc atttcttcat aactgtttca     60
   tggaatcagg tagttgatat tgctactatc cagttcattc aacgaaaatc cagcgtttaa    120
   cgtgccgcac agtgtattgt gctggtgaga cgagtaagtc agttttttaa aggttgccac    180
   cacggggtcg ac                                                        192
<210> 35
   <211> 912
   <212> DNA
   <213> Escherichia coli
<220>
   <221> CDS
   <222> (1)..(912)
<400> 35
   gtg agt aca tta gaa caa aca ata ggc aat acg cct ctg gtg aag ttg      48
   Met Ser Thr Leu Glu Gln Thr Ile Gly Asn Thr Pro Leu Val Lys Leu
   1               5                   10                  15
   cag cga atg ggg ccg gat aac ggc agt gaa gtg tgg tta aaa ctg gaa      96
   Gln Arg Met Gly Pro Asp Asn Gly Ser Glu Val Trp Leu Lys Leu Glu
               20                  25                  30
   ggc aat aac ccg gca ggt tcg gtg aaa gat cgt gcg gca ctt tcg atg      144
   Gly Asn Asn Pro Ala Gly Ser Val Lys Asp Arg Ala Ala Leu Ser Met
           35                  40                  45
   atc gtc gag gcg gaa aag cgc ggg gaa att aaa ccg ggt gat gtc tta      192
   Ile Val Glu Ala Glu Lys Arg Gly Glu Ile Lys Pro Gly Asp Val Leu
       50                  55                  60
   atc gaa gcc acc agt ggt aac acc ggc att gcg ctg gca atg att gcc      240
   Ile Glu Ala Thr Ser Gly Asn Thr Gly Ile Ala Leu Ala Met Ile Ala
   65                  70                  75                  80
   gcg ctg aaa ggc tat cgc atg aaa ttg ctg atg ccc gac aac atg agc      288
   Ala Leu Lys Gly Tyr Arg Met Lys Leu Leu Met Pro Asp Asn Met Ser
                   85                  90                  95
   cag gaa cgc cgt gcg gcg atg cgt gct tat ggt gcg gaa ctg att ctt      336
   Gln Glu Arg Arg Ala Ala Met Arg Ala Tyr Gly Ala Glu Leu Ile Leu
               100                 105                 110
   gtc acc aaa gag cag ggc atg gaa ggt gcg cgc gat ctg gcg ctg gag      384
   Val Thr Lys Glu Gln Gly Met Glu Gly Ala Arg Asp Leu Ala Leu Glu
           115                 120                 125
   atg gcg aat cgt ggc gaa gga aag ctg ctc gat cag ttc aat aat ccc      432
   Met Ala Asn Arg Gly Glu Gly Lys Leu Leu Asp Gln Phe Asn Asn Pro
       130                 135                 140
   gat aac cct tat gcg cat tac acc acc act ggg ccg gaa atc tgg cag      480
   Asp Asn Pro Tyr Ala His Tyr Thr Thr Thr Gly Pro Glu Ile Trp Gln
   145                 150                 155                 160
   caa acc ggc ggg cgc atc act cat ttt gtc tcc agc atg ggg acg acc      528
   Gln Thr Gly Gly Arg Ile Thr His Phe Val Ser Ser Met Gly Thr Thr
                   165                 170                 175
   ggc act atc acc ggc gtc tca cgc ttt atg cgc gaa caa tcc aaa ccg      576
   Gly Thr Ile Thr Gly Val Ser Arg Phe Met Arg Glu Gln Ser Lys Pro
               180                 185                 190
   gtg acc att gtc ggc ctg caa ccg gaa gag ggc agc agc att ccc ggc      624
   Val Thr Ile Val Gly Leu Gln Pro Glu Glu Gly Ser Ser Ile Pro Gly
           195                 200                 205
   att cgc cgc tgg cct acg gaa tat ctg ccg ggg att ttc aac gct tct      672
   Ile Arg Arg Trp Pro Thr Glu Tyr Leu Pro Gly Ile Phe Asn Ala Ser
       210                 215                 220
   ctg gtg gat gag gtg ctg gat att cat cag cgc gat gcg gaa aac acc      720
   Leu Val Asp Glu Val Leu Asp Ile His Gln Arg Asp Ala Glu Asn Thr
   225                 230                 235                 240
   atg cgc gaa ctg gcg gtg cgg gaa gga ata ttc tgt ggc gtc agc tcc      768
   Met Arg Glu Leu Ala Val Arg Glu Gly Ile Phe Cys Gly Val Ser Ser
                   245                 250                 255
   ggc ggc gcg gtt gcc gga gca ctg cgg gtg gca aaa gct aac cct gac      816
   Gly Gly Ala Val Ala Gly Ala Leu Arg Val Ala Lys Ala Asn Pro Asp
               260                 265                 270
   gcg gtg gtg gtg gcg atc atc tgc gat cgt ggc gat cgc tac ctt tct      864
   Ala Val Val Val Ala Ile Ile Cys Asp Arg Gly Asp Arg Tyr Leu Ser
           275                 280                 285
   acc ggg gtg ttt ggg gaa gag cat ttt agc cag ggg gcg ggg att taa      912
   Thr Gly Val Phe Gly Glu Glu His Phe Ser Gln Gly Ala Gly Ile
       290                 295                 300
<210> 36
   <211> 303
   <212> PRT
   <213> Escherichia coli
<400> 36
   Met Ser Thr Leu Glu Gln Thr Ile Gly Asn Thr Pro Leu Val Lys Leu
   1               5                   10                  15
   Gln Arg Met Gly Pro Asp Asn Gly Ser Glu Val Trp Leu Lys Leu Glu
               20                  25                  30
   Gly Asn Asn Pro Ala Gly Ser Val Lys Asp Arg Ala Ala Leu Ser Met
           35                  40                  45
   Ile Val Glu Ala Glu Lys Arg Gly Glu Ile Lys Pro Gly Asp Val Leu
       50                  55                  60
   Ile Glu Ala Thr Ser Gly Asn Thr Gly Ile Ala Leu Ala Met Ile Ala
   65                  70                  75                  80
   Ala Leu Lys Gly Tyr Arg Met Lys Leu Leu Met Pro Asp Asn Met Ser
                   85                  90                  95
   Gln Glu Arg Arg Ala Ala Met Arg Ala Tyr Gly Ala Glu Leu Ile Leu
               100                 105                 110
   Val Thr Lys Glu Gln Gly Met Glu Gly Ala Arg Asp Leu Ala Leu Glu
           115                 120                 125
   Met Ala Asn Arg Gly Glu Gly Lys Leu Leu Asp Gln Phe Asn Asn Pro
       130                 135                 140
   Asp Asn Pro Tyr Ala His Tyr Thr Thr Thr Gly Pro Glu Ile Trp Gln
   145                 150                 155                 160
   Gln Thr Gly Gly Arg Ile Thr His Phe Val Ser Ser Met Gly Thr Thr
                   165                 170                 175
   Gly Thr Ile Thr Gly Val Ser Arg Phe Met Arg Glu Gln Ser Lys Pro
               180                 185                 190
   Val Thr Ile Val Gly Leu Gln Pro Glu Glu Gly Ser Ser Ile Pro Gly
           195                 200                 205
   Ile Arg Arg Trp Pro Thr Glu Tyr Leu Pro Gly Ile Phe Asn Ala Ser
       210                 215                 220
   Leu Val Asp Glu Val Leu Asp Ile His Gln Arg Asp Ala Glu Asn Thr
   225                 230                 235                 240
   Met Arg Glu Leu Ala Val Arg Glu Gly Ile Phe Cys Gly Val Ser Ser
                   245                 250                 255
   Gly Gly Ala Val Ala Gly Ala Leu Arg Val Ala Lys Ala Asn Pro Asp
               260                 265                 270
   Ala Val Val Val Ala Ile Ile Cys Asp Arg Gly Asp Arg Tyr Leu Ser
           275                 280                 285
   Thr Gly Val Phe Gly Glu Glu His Phe Ser Gln Gly Ala Gly Ile
       290                 295                 300
<210> 37
   <211> 35
   <212> DNA
   <213> Artificial Sequence
<220>
   <223> primer P11
<400> 37
   agctgagtcg acgtgagtac attagaacaa acaat 35
<210> 38
   <211> 33
   <212> DNA
   <213> Artificial Sequence
<220>
   <223> primer P12
<400> 38
   agctgatcta gaagtctccg atgctattaa tcc 33
<210> 39
   <211> 60
   <212> DNA
   <213> Artificial Sequence
<220>
   <223> primer P13
<400> 39
   tccgctcacg atttttttca tcgctggtaa ggtcatttat cccccaggaa aaattggtta 60
<210> 40
   <211> 63
   <212> DNA
   <213> Artificial Sequence
<220>
   <223> primer P14
<400> 40
   tttcacaccg ctcaaccgca gggcataacc ggcccttgaa gcctgctttt ttatactaag 60
   ttg 63
<210> 41
   <211> 4403
   <212> DNA
   <213> Escherichia coli
<220>
   <221> CDS
   <222> (301)..(1311)
<220>
   <221> CDS
   <222> (1317)..(2147)
<220>
   <221> CDS
   <222> (2150)..(3022)
<400> 41
   tacagcggaa cctggcacgg gccagaaggg ttgatgccgt cggatgacac actcaagagc     60
   tggacgctca gcaaaattgt ctggcagcgc taagtctttt ttcacaccgc tcaaccgcag    120
   ggcataaccg gccctgcgcg tccaattctg tttttcgtct gtcttttccc gccgccttat    180
   gcctttttcg actttgaaat cagcaaacga tatataaaac cgttacgggt ttacgctgag   240
   ttataaataa actgctgtat ctgcagatga gatctgcatc aaatttcctc agggtgaacc    300
   atg acc tta cca gcg atg aaa aaa atc gtg agc gga ctc gca ctg tcg      348
   Met Thr Leu Pro Ala Met Lys Lys Ile Val Ser Gly Leu Ala Leu Ser
   1               5                   10                  15
   ctg agt ctg gcc ggt gcc gca aac gcg acc gag ctg ttg aac agc tct      396
   Leu Ser Leu Ala Gly Ala Ala Asn Ala Thr Glu Leu Leu Asn Ser Ser
               20                  25                  30
   tac gat gtc gca cgt gaa tta ttt gtc gcc ctg aat gcg cct ttt gtc      444
   Tyr Asp Val Ala Arg Glu Leu Phe Val Ala Leu Asn Ala Pro Phe Val
           35                  40                  45
   agc cag tgg gat gcc agc cat cct gac gac aag ctg acc att aag atg      492
   Ser Gln Trp Asp Ala Ser His Pro Asp Asp Lys Leu Thr Ile Lys Met
       50                  55                  60
   tcc cat gcc ggg tca tcc aaa cag gcg ctg gcg atc ctg caa ggc ctg      540
   Ser His Ala Gly Ser Ser Lys Gln Ala Leu Ala Ile Leu Gln Gly Leu
   65                  70                  75                  80
   cgt gcc gat gtg gtg acc tat aac cag gtc acc gat gtg cag gtg ctg      588
   Arg Ala Asp Val Val Thr Tyr Asn Gln Val Thr Asp Val Gln Val Leu
                   85                  90                  95
   cac gat aaa ggc aaa ctg atc cct gcc gac tgg caa acc cgc ctg ccg      636
   His Asp Lys Gly Lys Leu Ile Pro Ala Asp Trp Gln Thr Arg Leu Pro
               100                 105                 110
   aat aac agt tcg ccg ttt tac tcc acc atg gcg ttc ctg gtg cgc aag      684
   Asn Asn Ser Ser Pro Phe Tyr Ser Thr Met Ala Phe Leu Val Arg Lys
           115                 120                 125
   gga aac cca aag cag att cac gac tgg tcc gat tta acc cgt gac gat      732
   Gly Asn Pro Lys Gln Ile His Asp Trp Ser Asp Leu Thr Arg Asp Asp
       130                 135                 140
   gtg aag ctg att ttt cct aat ccc aaa acc tcg ggc aac gga cgt tat      780
   Val Lys Leu Ile Phe Pro Asn Pro Lys Thr Ser Gly Asn Gly Arg Tyr
   145                 150                 155                 160
   acc tat ctt gct gcc tgg ggc gcc gcc agc aac act gac ggg ggc gat      828
   Thr Tyr Leu Ala Ala Trp Gly Ala Ala Ser Asn Thr Asp Gly Gly Asp
                   165                 170                 175
   cag gct aaa acc cgc gct ttt atg aca aaa ttt ctg aaa aat gtt gaa      876
   Gln Ala Lys Thr Arg Ala Phe Met Thr Lys Phe Leu Lys Asn Val Glu
               180                 185                 190
   gtc ttc gat acc ggt ggc cga ggt gct acg acc acc ttt gct gaa cgc      924
   Val Phe Asp Thr Gly Gly Arg Gly Ala Thr Thr Thr Phe Ala Glu Arg
           195                 200                 205
   ggt ctg ggc gat gtg ttg atc agt ttt gag tct gaa gtg aat aac atc      972
   Gly Leu Gly Asp Val Leu Ile Ser Phe Glu Ser Glu Val Asn Asn Ile
       210                 215                 220
   cgc aac cag tac ggc aaa gac gac tac gaa gtc gtg gtg cct aaa acc     1020
   Arg Asn Gln Tyr Gly Lys Asp Asp Tyr Glu Val Val Val Pro Lys Thr
   225                 230                 235                 240
   gat att ctc gcg gag ttt ccc gtt gcc tgg gta gat aaa aac gtc gag     1068
   Asp Ile Leu Ala Glu Phe Pro Val Ala Trp Val Asp Lys Asn Val Glu
                       245                     250                     255
   cag aat aaa aca gcc gat gca gcg aaa gcc tat ctg acc tgg ctg tat        1116
   Gln Asn Lys Thr Ala Asp Ala Ala Lys Ala Tyr Leu Thr Trp Leu Tyr
                   260                     265                     270
   tct cct gcg gcg cag aaa att att acg gat ttc tat tac cgc gtg aac        1164
   Ser Pro Ala Ala Gln Lys Ile Ile Thr Asp Phe Tyr Tyr Arg Val Asn
               275                     280                     285
   aat ccg cag tta atg gcg cag caa aaa gcc cgt ttt cct gcc acg aac        1212
   Asn Pro Gln Leu Met Ala Gln Gln Lys Ala Arg Phe Pro Ala Thr Asn
       290                     295                     300
   ctg ttt cgt gtt gaa gac att ttt ggc ggc tgg gat aac gtg atg aaa        1260
   Leu Phe Arg Val Glu Asp Ile Phe Gly Gly Trp Asp Asn Val Met Lys
   305                     310                     315                     320
   acc cat ttc gcc agc ggt ggc gag cta gac cag tta tta gcg gcg ggg        1308
   Thr His Phe Ala Ser Gly Gly Glu Leu Asp Gln Leu Leu Ala Ala Gly
                       325                     330                     335
   cgg tgatc atg ttt gca gcc agc caa aaa cgc gtc ctg ccc ggt ttc ggt      1358
   Arg       Met Phe Ala Ala Ser Gln Lys Arg Val Leu Pro Gly Phe Gly
                         340                     345                     350
   ctc agc ctg ggc acc agc ctg ctc ttt acc tgt ctg gtg ctg ctg ctg        1406
   Leu Ser Leu Gly Thr Ser Leu Leu Phe Thr Cys Leu Val Leu Leu Leu
                   355                     360                     365
   cca atc agc gca ctg att atg cag ctg tcg cag atg acg ttg cag caa        1454
   Pro Ile Ser Ala Leu Ile Met Gln Leu Ser Gln Met Thr Leu Gln Gln
               370                     375                     380
   tac tgg gac gtg gtc acc aat ccg cag ctc atc gcg gcc tat aag gtc        1502
   Tyr Trp Asp Val Val Thr Asn Pro Gln Leu Ile Ala Ala Tyr Lys Val
       385                     390                     395
   acg ctg ctg tcg gcc ggt gtg gcc tca ctg ttt aat gcc gta ttc ggc        1550
   Thr Leu Leu Ser Ala Gly Val Ala Ser Leu Phe Asn Ala Val Phe Gly
   400                     405                     410                     415
   atg tta atg gcg tgg atc tta acg cgt tac cgt ttt ccg ggc cgc acg        1598
   Met Leu Met Ala Trp Ile Leu Thr Arg Tyr Arg Phe Pro Gly Arg Thr
                       420                     425                     430
   ctg ctc gat ggt ctg atg gat ctg ccg ttt gcg ctg ccg acc gcg gtt        1646
   Leu Leu Asp Gly Leu Met Asp Leu Pro Phe Ala Leu Pro Thr Ala Val
                   435                     440                     445
   gct ggc ctg acg ctg gcc ggt ctg ttt tcc gtg aac ggc tgg tac gga        1694
   Ala Gly Leu Thr Leu Ala Gly Leu Phe Ser Val Asn Gly Trp Tyr Gly
               450                     455                     460
   caa tgg ttc gcg cat ttt gat atc aag atc tcc tat acc tgg atc ggt        1742
   Gln Trp Phe Ala His Phe Asp Ile Lys Ile Ser Tyr Thr Trp Ile Gly
       465                     470                     475
   atc gcg ctc gcg atg gcc ttc acc agt att ccg ttt gtg gtg cgt acc        1790
   Ile Ala Leu Ala Met Ala Phe Thr Ser Ile Pro Phe Val Val Arg Thr
   480                     485                     490                     495
   gtg cag ccg gtg ctg gaa gag ctg ggg cct gaa tat gag gaa gcg gct        1838
   Val Gln Pro Val Leu Glu Glu Leu Gly Pro Glu Tyr Glu Glu Ala Ala
                       500                     505                     510
   caa acg ctg ggc gcc acg ccc tgg cag agc ttc cgc cgg gtc gtt ctg        1886
   Gln Thr Leu Gly Ala Thr Pro Trp Gln Ser Phe Arg Arg Val Val Leu
                   515                     520                     525
   cct gaa gtg gca ccg gcc tta ctt gcg ggc acc gcg ctg tcg ttt acc        1934
   Pro Glu Val Ala Pro Ala Leu Leu Ala Gly Thr Ala Leu Ser Phe Thr
               530                     535                     540
   cgc agc ctg ggc gag ttt ggt gcg gta atc ttt att gcc ggc aac atc        1982
   Arg Ser Leu Gly Glu Phe Gly Ala Val Ile Phe Ile Ala Gly Asn Ile
       545                     550                     555
   gct tgg aaa acc gaa gtg acc tcg ctg atg atc ttc gtg cgc ctg cag        2030
   Ala Trp Lys Thr Glu Val Thr Ser Leu Met Ile Phe Val Arg Leu Gln
   560                     565                     570                     575
   gag ttt gac tat ccg gca gcc agc gcc att gcc tcg gtc att ctg gcg        2078
   Glu Phe Asp Tyr Pro Ala Ala Ser Ala Ile Ala Ser Val Ile Leu Ala
                   580                 585                 590
   gca tca ctg ctg tta ctt ttc gct atc aat acc tta caa agc cgc ttt      2126
   Ala Ser Leu Leu Leu Leu Phe Ala Ile Asn Thr Leu Gln Ser Arg Phe
                   595                 600                 605
   ggt cgt cgt ctg gga ggc cat ta atg gca gag att tcg caa ctc aat       2173
   Gly Arg Arg Leu Gly Gly His    Met Ala Glu Ile Ser Gln Leu Asn
               610                 615                 620
   cat gcc gac cgc cag cct gtt aac tgg gcc aag tgg ctg ctt att ggt      2221
   His Ala Asp Arg Gln Pro Val Asn Trp Ala Lys Trp Leu Leu Ile Gly
               625                 630                 635
   att ggt gcg ctg ata tcc ttg ctg ctg ctg gtc gtg ccg atg gtg tcc      2269
   Ile Gly Ala Leu Ile Ser Leu Leu Leu Leu Val Val Pro Met Val Ser
       640                 645                 650
   atc ttc tgg gag gcc ctg cat aaa gga ctg ggc gtc acc tta agt aat      2317
   Ile Phe Trp Glu Ala Leu His Lys Gly Leu Gly Val Thr Leu Ser Asn
   655                 660                 665                 670
   ctg acc gac agc gac atg ctc cat gcc ata tgg ctc acg gtg ctg gtc      2365
   Leu Thr Asp Ser Asp Met Leu His Ala Ile Trp Leu Thr Val Leu Val
                   675                 680                 685
   gca ttg att acc gtg ccg gtg aat tta gtg ttc ggc acg ctg ctg gcc      2413
   Ala Leu Ile Thr Val Pro Val Asn Leu Val Phe Gly Thr Leu Leu Ala
                   690                 695                 700
   tgg ctg gtg aca cgc ttt acc ttt ccg gga cgt cag ctg ctt ttg acg      2461
   Trp Leu Val Thr Arg Phe Thr Phe Pro Gly Arg Gln Leu Leu Leu Thr
               705                 710                 715
   ctg ttc gat att ccc ttt gcg gta tcg cct gtg gtc gcc ggt ctg atg      2509
   Leu Phe Asp Ile Pro Phe Ala Val Ser Pro Val Val Ala Gly Leu Met
       720                 725                 730
   tat ctc ctg ttc tgg ggc att aac ggc ccg gcg ggc ggc tgg ctg gat      2557
   Tyr Leu Leu Phe Trp Gly Ile Asn Gly Pro Ala Gly Gly Trp Leu Asp
   735                 740                 745                 750
   gcc cat aat att cag gtg atg ttc tcc tgg cct ggc atg gtg ctg gtc      2605
   Ala His Asn Ile Gln Val Met Phe Ser Trp Pro Gly Met Val Leu Val
                   755                 760                 765
   acc gtc ttc gtt acc tgt ccg ttt gtg gtg cgc gaa ctg gtg ccg gtg      2653
   Thr Val Phe Val Thr Cys Pro Phe Val Val Arg Glu Leu Val Pro Val
                   770                 775                 780
   atg ctg agc cag ggc agt cat gaa gat gaa gcc gcg gtg ctg tta ggt      2701
   Met Leu Ser Gln Gly Ser His Glu Asp Glu Ala Ala Val Leu Leu Gly
               785                 790                 795
   gcc tcg ggc tgg cag atg ttc cgt cgc gtg acg ctg ccg aat att cgc      2749
   Ala Ser Gly Trp Gln Met Phe Arg Arg Val Thr Leu Pro Asn Ile Arg
       800                 805                 810
   tgg gcc atg ctg tat ggc gtc gtg ctg acc aac gcc cgc gcg att ggt      2797
   Trp Ala Met Leu Tyr Gly Val Val Leu Thr Asn Ala Arg Ala Ile Gly
   815                 820                 825                 830
   gag ttt ggc gcg gtt tcc gtg gtt tcg ggt tct att cgc ggt gaa acc      2845
   Glu Phe Gly Ala Val Ser Val Val Ser Gly Ser Ile Arg Gly Glu Thr
                   835                 840                 845
   tac act tta ccg ctt cag gtt gaa tta ctg cat cag gat tac aac acg      2893
   Tyr Thr Leu Pro Leu Gln Val Glu Leu Leu His Gln Asp Tyr Asn Thr
                   850                 855                 860
   gtg ggc gcc ttt act gcc gca gcc tta ctg acc gtg atg gca atc gtg      2941
   Val Gly Ala Phe Thr Ala Ala Ala Leu Leu Thr Val Met Ala Ile Val
               865                 870                 875
   acg ctg ttt ctg aaa agc att gtg caa tgg cgt tta gag caa cag cac      2989
   Thr Leu Phe Leu Lys Ser Ile Val Gln Trp Arg Leu Glu Gln Gln His
       880                 885                 890
   aaa cgc ctg caa ctg gag gac aat cat gag cat tgagattaac cagatcaaca    3042
   Lys Arg Leu Gln Leu Glu Asp Asn His Glu His
   895                 900                 905
   aatcctttgg tcgcacagcg gtgctgaacg atatctcact ggatattcct tctggccaga    3102
   tggtggcctt actggggccg tccggttccg gtaaaaccac gctgctgcgc atcattgctg    3162
   gactggaaca tcagaacagc ggtcagattc gttttcacga ccacgatgtc agccgcctgc   3222
   acgcccgcga tcgccaggtc ggatttgtct tccagcacta tgcgctgttc cgtcatatga   3282
   cggtcttcga caatattgcc tttggcctga ccgtgctgcc gcgccgtgag cgtccgtcca   3342
   gtgcggaaat taaaaaacgc gtcacgcgcc tgctggagat ggtgcagctt tcccatctgg   3402
   cgaaccgttt cccggcccag ctttcgggag ggcagaagca gcgcgtcgcg ctggcaagag   3462
   ccctggccgt ggaaccgcaa atcctgttgc tggatgagcc ctttggtgcg ctggacgctc   3522
   aggtgcgtaa agagctgcgc cgttggttac gtcagctgca cgaagaattg aagttcacca   3582
   gcgtgttcgt cacccacgat caggaagagg cgatggaagt ggccgatcgc gtggtggtga   3642
   tgagccaggg cagcatcgaa caggtgggga cgccggatga agtctggcgc gatcccgcca   3702
   cgcgcttcgt gctggaattc ctgggtgagg ttaaccgctt cgacggtgaa gtgcatggtt   3762
   ctcagttcca tgtcggggcg caccactggc cgttaggcta tacctctgca catcagggcg   3822
   cggtcgatct gttcctgcgc ccgtgggaaa tcgacgtttc gcgcagaagt agcctggaaa   3882
   cgccgctgcc cgttcaggtc ttagaagtga gtcctcgtgg tcacttctgg cagctggtgg   3942
   tgcagccaac gggatggcag agcgagccct tctcgctggt ctttgacggt gaacagaccg   4002
   cgccgttgcg cggcgagcgc ctgttcgtgg ggctgcagca ggccagactg taccagggcg   4062
   cgacaccgtt acgggcggtt gcctttgcac acagcgcctg ataggttgag tgaatgttaa   4122
   acgcccggag gcgcttcccg cgatccgggc tttttaatgg caaggtttgt aacctgtaga   4182
   cctgataaga cgcgcaagcg tcgcatcagg caacaccacg tatggataga gatcgtgagt   4242
   acattagaac aaacaatagg caatacgcct ctggtgaagt tgcagcgaat ggggccggat   4302
   aacggcagtg aagtgtggtt aaaactggaa ggcaataacc cggcaggttc ggtgaaagat   4362
   cgtgcggcac tttcgatgat cgtcgaggcg gaaaagcgcg g                        4403
<210> 42
   <211> 337
   <212> PRT
   <213> Escherichia coli
<400> 42
   Met Thr Leu Pro Ala Met Lys Lys Ile Val Ser Gly Leu Ala Leu Ser
   1               5                   10                  15
   Leu Ser Leu Ala Gly Ala Ala Asn Ala Thr Glu Leu Leu Asn Ser Ser
               20                  25                  30
   Tyr Asp Val Ala Arg Glu Leu Phe Val Ala Leu Asn Ala Pro Phe Val
           35                  40                  45
   Ser Gln Trp Asp Ala Ser His Pro Asp Asp Lys Leu Thr Ile Lys Met
       50                  55                  60
   Ser His Ala Gly Ser Ser Lys Gln Ala Leu Ala Ile Leu Gln Gly Leu
   65                  70                  75                  80
   Arg Ala Asp Val Val Thr Tyr Asn Gln Val Thr Asp Val Gln Val Leu
                   85                  90                  95
   His Asp Lys Gly Lys Leu Ile Pro Ala Asp Trp Gln Thr Arg Leu Pro
               100                 105                 110
   Asn Asn Ser Ser Pro Phe Tyr Ser Thr Met Ala Phe Leu Val Arg Lys
           115                 120                 125
   Gly Asn Pro Lys Gln Ile His Asp Trp Ser Asp Leu Thr Arg Asp Asp
       130                 135                 140
   Val Lys Leu Ile Phe Pro Asn Pro Lys Thr Ser Gly Asn Gly Arg Tyr
   145                 150                 155                 160
   Thr Tyr Leu Ala Ala Trp Gly Ala Ala Ser Asn Thr Asp Gly Gly Asp
                   165                 170                 175
   Gln Ala Lys Thr Arg Ala Phe Met Thr Lys Phe Leu Lys Asn Val Glu
               180                 185                 190
   Val Phe Asp Thr Gly Gly Arg Gly Ala Thr Thr Thr Phe Ala Glu Arg
           195                 200                 205
   Gly Leu Gly Asp Val Leu Ile Ser Phe Glu Ser Glu Val Asn Asn Ile
       210                 215                 220
   Arg Asn Gln Tyr Gly Lys Asp Asp Tyr Glu Val Val Val Pro Lys Thr
   225                 230                 235                 240
   Asp Ile Leu Ala Glu Phe Pro Val Ala Trp Val Asp Lys Asn Val Glu
                   245                 250                 255
   Gln Asn Lys Thr Ala Asp Ala Ala Lys Ala Tyr Leu Thr Trp Leu Tyr
               260                 265                 270
   Ser Pro Ala Ala Gln Lys Ile Ile Thr Asp Phe Tyr Tyr Arg Val Asn
           275                 280                 285
   Asn Pro Gln Leu Met Ala Gln Gln Lys Ala Arg Phe Pro Ala Thr Asn
       290                 295                 300
   Leu Phe Arg Val Glu Asp Ile Phe Gly Gly Trp Asp Asn Val Met Lys
   305                 310                 315                 320
   Thr His Phe Ala Ser Gly Gly Glu Leu Asp Gln Leu Leu Ala Ala Gly
                   325                 330                 335
   Arg
<210> 43
   <211> 277
   <212> PRT
   <213> Escherichia coli
<400> 43
   Met Phe Ala Ala Ser Gln Lys Arg Val Leu Pro Gly Phe Gly Leu Ser
   1               5                   10                  15
   Leu Gly Thr Ser Leu Leu Phe Thr Cys Leu Val Leu Leu Leu Pro Ile
               20                  25                  30
   Ser Ala Leu Ile Met Gln Leu Ser Gln Met Thr Leu Gln Gln Tyr Trp
           35                  40                  45
   Asp Val Val Thr Asn Pro Gln Leu Ile Ala Ala Tyr Lys Val Thr Leu
       50                  55                  60
   Leu Ser Ala Gly Val Ala Ser Leu Phe Asn Ala Val Phe Gly Met Leu
   65                  70                  75                  80
   Met Ala Trp Ile Leu Thr Arg Tyr Arg Phe Pro Gly Arg Thr Leu Leu
                   85                  90                  95
   Asp Gly Leu Met Asp Leu Pro Phe Ala Leu Pro Thr Ala Val Ala Gly
               100                 105                 110
   Leu Thr Leu Ala Gly Leu Phe Ser Val Asn Gly Trp Tyr Gly Gln Trp
           115                 120                 125
   Phe Ala His Phe Asp Ile Lys Ile Ser Tyr Thr Trp Ile Gly Ile Ala
       130                 135                 140
   Leu Ala Met Ala Phe Thr Ser Ile Pro Phe Val Val Arg Thr Val Gln
   145                 150                 155                 160
   Pro Val Leu Glu Glu Leu Gly Pro Glu Tyr Glu Glu Ala Ala Gln Thr
                   165                 170                 175
   Leu Gly Ala Thr Pro Trp Gln Ser Phe Arg Arg Val Val Leu Pro Glu
               180                 185                 190
   Val Ala Pro Ala Leu Leu Ala Gly Thr Ala Leu Ser Phe Thr Arg Ser
           195                 200                 205
   Leu Gly Glu Phe Gly Ala Val Ile Phe Ile Ala Gly Asn Ile Ala Trp
       210                 215                 220
   Lys Thr Glu Val Thr Ser Leu Met Ile Phe Val Arg Leu Gln Glu Phe
   225                 230                 235                 240
   Asp Tyr Pro Ala Ala Ser Ala Ile Ala Ser Val Ile Leu Ala Ala Ser
                   245                 250                 255
   Leu Leu Leu Leu Phe Ala Ile Asn Thr Leu Gln Ser Arg Phe Gly Arg
               260                 265                 270
   Arg Leu Gly Gly His
           275
<210> 44
   <211> 291
   <212> PRT
   <213> Escherichia coli
<400> 44
   Met Ala Glu Ile Ser Gln Leu Asn His Ala Asp Arg Gln Pro Val Asn
   1               5                   10                  15
   Trp Ala Lys Trp Leu Leu Ile Gly Ile Gly Ala Leu Ile Ser Leu Leu
               20                  25                  30
   Leu Leu Val Val Pro Met Val Ser Ile Phe Trp Glu Ala Leu His Lys
           35                  40                  45
   Gly Leu Gly Val Thr Leu Ser Asn Leu Thr Asp Ser Asp Met Leu His
       50                  55                  60
   Ala Ile Trp Leu Thr Val Leu Val Ala Leu Ile Thr Val Pro Val Asn
   65                  70                  75                  80
   Leu Val Phe Gly Thr Leu Leu Ala Trp Leu Val Thr Arg Phe Thr Phe
                   85                  90                  95
   Pro Gly Arg Gln Leu Leu Leu Thr Leu Phe Asp Ile Pro Phe Ala Val
               100                 105                 110
   Ser Pro Val Val Ala Gly Leu Met Tyr Leu Leu Phe Trp Gly Ile Asn
           115                 120                 125
   Gly Pro Ala Gly Gly Trp Leu Asp Ala His Asn Ile Gln Val Met Phe
       130                 135                 140
   Ser Trp Pro Gly Met Val Leu Val Thr Val Phe Val Thr Cys Pro Phe
   145                 150                 155                 160
   Val Val Arg Glu Leu Val Pro Val Met Leu Ser Gln Gly Ser His Glu
                   165                 170                 175
   Asp Glu Ala Ala Val Leu Leu Gly Ala Ser Gly Trp Gln Met Phe Arg
               180                 185                 190
   Arg Val Thr Leu Pro Asn Ile Arg Trp Ala Met Leu Tyr Gly Val Val
           195                 200                 205
   Leu Thr Asn Ala Arg Ala Ile Gly Glu Phe Gly Ala Val Ser Val Val
       210                 215                 220
   Ser Gly Ser Ile Arg Gly Glu Thr Tyr Thr Leu Pro Leu Gln Val Glu
   225                 230                 235                 240
   Leu Leu His Gln Asp Tyr Asn Thr Val Gly Ala Phe Thr Ala Ala Ala
                   245                 250                 255
   Leu Leu Thr Val Met Ala Ile Val Thr Leu Phe Leu Lys Ser Ile Val
               260                 265                 270
   Gln Trp Arg Leu Glu Gln Gln His Lys Arg Leu Gln Leu Glu Asp Asn
           275                 280                 285
   His Glu His
       290
<210> 45
   <211> 1089
   <212> DNA
   <213> Escherichia coli
<220>
   <221> CDS
   <222> (1)..(1089)
<400> 45
   atg agc att gag att aac cag atc aac aaa tcc ttt ggt cgc aca gcg        48
   Met Ser Ile Glu Ile Asn Gln Ile Asn Lys Ser Phe Gly Arg Thr Ala
   1               5                   10                  15
   gtg ctg aac gat atc tca ctg gat att cct tct ggc cag atg gtg gcc        96
   Val Leu Asn Asp Ile Ser Leu Asp Ile Pro Ser Gly Gln Met Val Ala
               20                  25                  30
   tta ctg ggg ccg tcc ggt tcc ggt aaa acc acg ctg ctg cgc atc att       144
   Leu Leu Gly Pro Ser Gly Ser Gly Lys Thr Thr Leu Leu Arg Ile Ile
           35                  40                  45
   gct gga ctg gaa cat cag aac agc ggt cag att cgt ttt cac gac cac       192
   Ala Gly Leu Glu His Gln Asn Ser Gly Gln Ile Arg Phe His Asp His
       50                  55                  60
   gat gtc agc cgc ctg cac gcc cgc gat cgc cag gtc gga ttt gtc ttc       240
   Asp Val Ser Arg Leu His Ala Arg Asp Arg Gln Val Gly Phe Val Phe
   65                  70                  75                  80
   cag cac tat gcg ctg ttc cgt cat atg acg gtc ttc gac aat att gcc      288
   Gln His Tyr Ala Leu Phe Arg His Met Thr Val Phe Asp Asn Ile Ala
                   85                  90                  95
   ttt ggc ctg acc gtg ctg ccg cgc cgt gag cgt ccg tcc agt gcg gaa      336
   Phe Gly Leu Thr Val Leu Pro Arg Arg Glu Arg Pro Ser Ser Ala Glu
               100                 105                 110
   att aaa aaa cgc gtc acg cgc ctg ctg gag atg gtg cag ctt tcc cat      384
   Ile Lys Lys Arg Val Thr Arg Leu Leu Glu Met Val Gln Leu Ser His
           115                 120                 125
   ctg gcg aac cgt ttc ccg gcc cag ctt tcg gga ggg cag aag cag cgc      432
   Leu Ala Asn Arg Phe Pro Ala Gln Leu Ser Gly Gly Gln Lys Gln Arg
       130                 135                 140
   gtc gcg ctg gca aga gcc ctg gcc gtg gaa ccg caa atc ctg ttg ctg      480
   Val Ala Leu Ala Arg Ala Leu Ala Val Glu Pro Gln Ile Leu Leu Leu
   145                 150                 155                 160
   gat gag ccc ttt ggt gcg ctg gac gct cag gtg cgt aaa gag ctg cgc      528
   Asp Glu Pro Phe Gly Ala Leu Asp Ala Gln Val Arg Lys Glu Leu Arg
                   165                 170                 175
   cgt tgg tta cgt cag ctg cac gaa gaa ttg aag ttc acc agc gtg ttc      576
   Arg Trp Leu Arg Gln Leu His Glu Glu Leu Lys Phe Thr Ser Val Phe
               180                 185                 190
   gtc acc cac gat cag gaa gag gcg atg gaa gtg gcc gat cgc gtg gtg      624
   Val Thr His Asp Gln Glu Glu Ala Met Glu Val Ala Asp Arg Val Val
           195                 200                 205
   gtg atg agc cag ggc agc atc gaa cag gtg ggg acg ccg gat gaa gtc      672
   Val Met Ser Gln Gly Ser Ile Glu Gln Val Gly Thr Pro Asp Glu Val
       210                 215                 220
   tgg cgc gat ccc gcc acg cgc ttc gtg ctg gaa ttc ctg ggt gag gtt      720
   Trp Arg Asp Pro Ala Thr Arg Phe Val Leu Glu Phe Leu Gly Glu Val
   225                 230                 235                 240
   aac cgc ttc gac ggt gaa gtg cat ggt tct cag ttc cat gtc ggg gcg      768
   Asn Arg Phe Asp Gly Glu Val His Gly Ser Gln Phe His Val Gly Ala
                   245                 250                 255
   cac cac tgg ccg tta ggc tat acc tct gca cat cag ggc gcg gtc gat      816
   His His Trp Pro Leu Gly Tyr Thr Ser Ala His Gln Gly Ala Val Asp
               260                 265                 270
   ctg ttc ctg cgc ccg tgg gaa atc gac gtt tcg cgc aga agt agc ctg      864
   Leu Phe Leu Arg Pro Trp Glu Ile Asp Val Ser Arg Arg Ser Ser Leu
           275                 280                 285
   gaa acg ccg ctg ccc gtt cag gtc tta gaa gtg agt cct cgt ggt cac      912
   Glu Thr Pro Leu Pro Val Gln Val Leu Glu Val Ser Pro Arg Gly His
       290                 295                 300
   ttc tgg cag ctg gtg gtg cag cca acg gga tgg cag agc gag ccc ttc      960
   Phe Trp Gln Leu Val Val Gln Pro Thr Gly Trp Gln Ser Glu Pro Phe
   305                 310                 315                 320
   tcg ctg gtc ttt gac ggt gaa cag acc gcg ccg ttg cgc ggc gag cgc     1008
   Ser Leu Val Phe Asp Gly Glu Gln Thr Ala Pro Leu Arg Gly Glu Arg
                   325                 330                 335
   ctg ttc gtg ggg ctg cag cag gcc aga ctg tac cag ggc gcg aca ccg     1056
   Leu Phe Val Gly Leu Gln Gln Ala Arg Leu Tyr Gln Gly Ala Thr Pro
               340                 345                 350
   tta cgg gcg gtt gcc ttt gca cac agc gcc tga                         1089
   Leu Arg Ala Val Ala Phe Ala His Ser Ala
           355                 360
<210> 46
   <211> 362
   <212> PRT
   <213> Escherichia coli
<400> 46
   Met Ser Ile Glu Ile Asn Gln Ile Asn Lys Ser Phe Gly Arg Thr Ala
   1               5                   10                  15
   Val Leu Asn Asp Ile Ser Leu Asp Ile Pro Ser Gly Gln Met Val Ala
               20                  25                  30
   Leu Leu Gly Pro Ser Gly Ser Gly Lys Thr Thr Leu Leu Arg Ile Ile
           35                  40                  45
   Ala Gly Leu Glu His Gln Asn Ser Gly Gln Ile Arg Phe His Asp His
       50                  55                  60
   Asp Val Ser Arg Leu His Ala Arg Asp Arg Gln Val Gly Phe Val Phe
   65                  70                  75                  80
   Gln His Tyr Ala Leu Phe Arg His Met Thr Val Phe Asp Asn Ile Ala
                   85                  90                  95
   Phe Gly Leu Thr Val Leu Pro Arg Arg Glu Arg Pro Ser Ser Ala Glu
               100                 105                 110
   Ile Lys Lys Arg Val Thr Arg Leu Leu Glu Met Val Gln Leu Ser His
           115                 120                 125
   Leu Ala Asn Arg Phe Pro Ala Gln Leu Ser Gly Gly Gln Lys Gln Arg
       130                 135                 140
   Val Ala Leu Ala Arg Ala Leu Ala Val Glu Pro Gln Ile Leu Leu Leu
   145                 150                 155                 160
   Asp Glu Pro Phe Gly Ala Leu Asp Ala Gln Val Arg Lys Glu Leu Arg
                   165                 170                 175
   Arg Trp Leu Arg Gln Leu His Glu Glu Leu Lys Phe Thr Ser Val Phe
               180                 185                 190
   Val Thr His Asp Gln Glu Glu Ala Met Glu Val Ala Asp Arg Val Val
           195                 200                 205
   Val Met Ser Gln Gly Ser Ile Glu Gln Val Gly Thr Pro Asp Glu Val
       210                 215                 220
   Trp Arg Asp Pro Ala Thr Arg Phe Val Leu Glu Phe Leu Gly Glu Val
   225                 230                 235                 240
   Asn Arg Phe Asp Gly Glu Val His Gly Ser Gln Phe His Val Gly Ala
                   245                 250                 255
   His His Trp Pro Leu Gly Tyr Thr Ser Ala His Gln Gly Ala Val Asp
               260                 265                 270
   Leu Phe Leu Arg Pro Trp Glu Ile Asp Val Ser Arg Arg Ser Ser Leu
           275                 280                 285
   Glu Thr Pro Leu Pro Val Gln Val Leu Glu Val Ser Pro Arg Gly His
       290                 295                 300
   Phe Trp Gln Leu Val Val Gln Pro Thr Gly Trp Gln Ser Glu Pro Phe
   305                 310                 315                 320
   Ser Leu Val Phe Asp Gly Glu Gln Thr Ala Pro Leu Arg Gly Glu Arg
                   325                 330                 335
   Leu Phe Val Gly Leu Gln Gln Ala Arg Leu Tyr Gln Gly Ala Thr Pro
               340                 345                 350
   Leu Arg Ala Val Ala Phe Ala His Ser Ala
           355                 360
<210> 47
   <211> 20
   <212> DNA
   <213> Artificial Sequence
<220>
   <223> primer P15
<400> 47
   ctttgtccct ttagtgaagg 20
<210> 48
   <211> 44
   <212> DNA
   <213> Artificial Sequence
<220>
   <223> primer P16
<400> 48
   agctgatcta gaagctgact cgagttaatg gcctcccaga cgac 44
<210> 49
   <211> 1239
   <212> DNA
   <213> Pantoea ananatis
<220>
   <221> CDS
   <222> (1)..(1239)
<400> 49
   atg gca aag gta tca ctg gaa aaa gac aaa att aag ttc ctg ctg gtg        48
   Met Ala Lys Val Ser Leu Glu Lys Asp Lys Ile Lys Phe Leu Leu Val
   1               5                   10                  15
   gaa ggt gtc cat cag agc gcg ctg gaa aat ctt cgt gct gca ggt tac        96
   Glu Gly Val His Gln Ser Ala Leu Glu Asn Leu Arg Ala Ala Gly Tyr
               20                  25                  30
   acc aat att gaa ttc cac aaa ggc gca ctg gat gcc gag gcg tta aaa       144
   Thr Asn Ile Glu Phe His Lys Gly Ala Leu Asp Ala Glu Ala Leu Lys
           35                  40                  45
   gct tcc gct cgc gat gcg cat ttt atc ggt atc cgt tcc cgt tcc caa       192
   Ala Ser Ala Arg Asp Ala His Phe Ile Gly Ile Arg Ser Arg Ser Gln
       50                  55                  60
   ctg acc gaa gag att ttt gcc gct gca gaa aaa ctg gta gcg gtg ggc       240
   Leu Thr Glu Glu Ile Phe Ala Ala Ala Glu Lys Leu Val Ala Val Gly
   65                  70                  75                  80
   tgt ttc tgt atc gga acg aat cag gtt gat tta aat gcc gca gcg aaa       288
   Cys Phe Cys Ile Gly Thr Asn Gln Val Asp Leu Asn Ala Ala Ala Lys
                   85                  90                  95
   cgc ggt atc ccg gtt ttt aac gca cct ttc tca aat acg cgc tct gtg       336
   Arg Gly Ile Pro Val Phe Asn Ala Pro Phe Ser Asn Thr Arg Ser Val
               100                 105                 110
   gcc gag ctg gtt att ggc gag atg ctg ctg atg ctg cgc ggt gtt ccg       384
   Ala Glu Leu Val Ile Gly Glu Met Leu Leu Met Leu Arg Gly Val Pro
           115                 120                 125
   gaa gcg aat gcc aaa gcg cac cgt ggt atc tgg aat aaa atc gcc aaa       432
   Glu Ala Asn Ala Lys Ala His Arg Gly Ile Trp Asn Lys Ile Ala Lys
       130                 135                 140
   ggc tct ttt gaa gcg cgc ggt aaa aag ctg ggt atc att ggc tat ggc       480
   Gly Ser Phe Glu Ala Arg Gly Lys Lys Leu Gly Ile Ile Gly Tyr Gly
   145                 150                 155                 160
   cat atc ggt atg caa ctg ggc gtg ctg gca gaa agt ctg ggc atg cac       528
   His Ile Gly Met Gln Leu Gly Val Leu Ala Glu Ser Leu Gly Met His
                   165                 170                 175
   gtt tac ttc tat gac atc gaa aac aag ctg ccg ttg ggc aac gca tca       576
   Val Tyr Phe Tyr Asp Ile Glu Asn Lys Leu Pro Leu Gly Asn Ala Ser
               180                 185                 190
   cag gtt cgt agc ctg acg cag ttg cta aat atg agt gac gtt gtc agc       624
   Gln Val Arg Ser Leu Thr Gln Leu Leu Asn Met Ser Asp Val Val Ser
           195                 200                 205
   ctg cat gtc ccg gaa acc gcc tct acg caa aat atg att tct gcc aat       672
   Leu His Val Pro Glu Thr Ala Ser Thr Gln Asn Met Ile Ser Ala Asn
       210                 215                 220
   gag ctg gct cag atg aag cct ggc ggc ctg ctg ata aat gcc tca cgc       720
   Glu Leu Ala Gln Met Lys Pro Gly Gly Leu Leu Ile Asn Ala Ser Arg
   225                 230                 235                 240
   ggc acc gtg gta gat att cct gct ttg tgc gaa gcg ctg gcc agc aag      768
   Gly Thr Val Val Asp Ile Pro Ala Leu Cys Glu Ala Leu Ala Ser Lys
                   245                 250                 255
   cag gtt ggt ggc gct gcg att gat gtg ttc cct gta gag ccg gcg acc      816
   Gln Val Gly Gly Ala Ala Ile Asp Val Phe Pro Val Glu Pro Ala Thr
               260                 265                 270
   aac agc gat ccg ttt gtt tcc cca ctg agc gaa ttc gac aac gtt atc      864
   Asn Ser Asp Pro Phe Val Ser Pro Leu Ser Glu Phe Asp Asn Val Ile
           275                 280                 285
   ctg acg ccg cac atc ggg gga tcg acg gaa gaa gct cag gag aat atc      912
   Leu Thr Pro His Ile Gly Gly Ser Thr Glu Glu Ala Gln Glu Asn Ile
       290                 295                 300
   ggg att gaa gtc gcg ggc aag ctg gcg aaa tat tcg gat aac ggt tca      960
   Gly Ile Glu Val Ala Gly Lys Leu Ala Lys Tyr Ser Asp Asn Gly Ser
   305                 310                 315                 320
   acg ctg tcc gcc gtc aat ttc ccg gaa gtg tca ttg ccg atg cac ggc     1008
   Thr Leu Ser Ala Val Asn Phe Pro Glu Val Ser Leu Pro Met His Gly
                   325                 330                 335
   att agc gcc agt cgt ctg ctg cat att cac gaa aac cgt ccg ggc gtt     1056
   Ile Ser Ala Ser Arg Leu Leu His Ile His Glu Asn Arg Pro Gly Val
               340                 345                 350
   ctc acc gcg atc aac cag att ttc gct gaa caa ggc atc aac att gcc     1104
   Leu Thr Ala Ile Asn Gln Ile Phe Ala Glu Gln Gly Ile Asn Ile Ala
           355                 360                 365
   gct cag tac ctg caa acc tct ccg atg atg ggt tat gtg gtc atc gac     1152
   Ala Gln Tyr Leu Gln Thr Ser Pro Met Met Gly Tyr Val Val Ile Asp
       370                 375                 380
   att gat gct gag cac gaa ctg gca gag aaa gct ctg caa ctg atg aag     1200
   Ile Asp Ala Glu His Glu Leu Ala Glu Lys Ala Leu Gln Leu Met Lys
   385                 390                 395                 400
   gcg att ccg gga acg att cgc gcc cgc ctg ctt tac tga                 1239
   Ala Ile Pro Gly Thr Ile Arg Ala Arg Leu Leu Tyr
                   405                 410
<210> 50
   <211> 412
   <212> PRT
   <213> Pantoea ananatis
<400> 50
   Met Ala Lys Val Ser Leu Glu Lys Asp Lys Ile Lys Phe Leu Leu Val
   1               5                   10                  15
   Glu Gly Val His Gln Ser Ala Leu Glu Asn Leu Arg Ala Ala Gly Tyr
               20                  25                  30
   Thr Asn Ile Glu Phe His Lys Gly Ala Leu Asp Ala Glu Ala Leu Lys
           35                  40                  45
   Ala Ser Ala Arg Asp Ala His Phe Ile Gly Ile Arg Ser Arg Ser Gln
       50                  55                  60
   Leu Thr Glu Glu Ile Phe Ala Ala Ala Glu Lys Leu Val Ala Val Gly
   65                  70                  75                  80
   Cys Phe Cys Ile Gly Thr Asn Gln Val Asp Leu Asn Ala Ala Ala Lys
                   85                  90                  95
   Arg Gly Ile Pro Val Phe Asn Ala Pro Phe Ser Asn Thr Arg Ser Val
               100                 105                 110
   Ala Glu Leu Val Ile Gly Glu Met Leu Leu Met Leu Arg Gly Val Pro
           115                 120                 125
   Glu Ala Asn Ala Lys Ala His Arg Gly Ile Trp Asn Lys Ile Ala Lys
       130                 135                 140
   Gly Ser Phe Glu Ala Arg Gly Lys Lys Leu Gly Ile Ile Gly Tyr Gly
   145                 150                 155                 160
   His Ile Gly Met Gln Leu Gly Val Leu Ala Glu Ser Leu Gly Met His
                   165                 170                 175
   Val Tyr Phe Tyr Asp Ile Glu Asn Lys Leu Pro Leu Gly Asn Ala Ser
               180                 185                 190
   Gln Val Arg Ser Leu Thr Gln Leu Leu Asn Met Ser Asp Val Val Ser
           195                 200                 205
   Leu His Val Pro Glu Thr Ala Ser Thr Gln Asn Met Ile Ser Ala Asn
       210                 215                 220
   Glu Leu Ala Gln Met Lys Pro Gly Gly Leu Leu Ile Asn Ala Ser Arg
   225                 230                 235                 240
   Gly Thr Val Val Asp Ile Pro Ala Leu Cys Glu Ala Leu Ala Ser Lys
                   245                 250                 255
   Gln Val Gly Gly Ala Ala Ile Asp Val Phe Pro Val Glu Pro Ala Thr
               260                 265                 270
   Asn Ser Asp Pro Phe Val Ser Pro Leu Ser Glu Phe Asp Asn Val Ile
           275                 280                 285
   Leu Thr Pro His Ile Gly Gly Ser Thr Glu Glu Ala Gln Glu Asn Ile
       290                 295                 300
   Gly Ile Glu Val Ala Gly Lys Leu Ala Lys Tyr Ser Asp Asn Gly Ser
   305                 310                 315                 320
   Thr Leu Ser Ala Val Asn Phe Pro Glu Val Ser Leu Pro Met His Gly
                   325                 330                 335
   Ile Ser Ala Ser Arg Leu Leu His Ile His Glu Asn Arg Pro Gly Val
               340                 345                 350
   Leu Thr Ala Ile Asn Gln Ile Phe Ala Glu Gln Gly Ile Asn Ile Ala
           355                 360                 365
   Ala Gln Tyr Leu Gln Thr Ser Pro Met Met Gly Tyr Val Val Ile Asp
       370                 375                 380
   Ile Asp Ala Glu His Glu Leu Ala Glu Lys Ala Leu Gln Leu Met Lys
   385                 390                 395                 400
   Ala Ile Pro Gly Thr Ile Arg Ala Arg Leu Leu Tyr
                   405                 410
<210> 51
   <211> 33
   <212> DNA
   <213> Artificial Sequence
<220>
   <223> primer P17
<400> 51
   agctgagtcg acatggcaaa ggtatcactg gaa 33
<210> 52
   <211> 34
   <212> DNA
   <213> Artificial Sequence
<220>
   <223> primer P18
<400> 52
   gagaacgccc gggcgggctt cgtgaatatg cagc 34
<210> 53
   <211> 32
   <212> DNA
   <213> Artificial Sequence
<220>
   <223> primer P19
<400> 53
   agctgatcta gacgtgggat cagtaaagca gg 32
<210> 54
   <211> 22
   <212> DNA
   <213> Artificial Sequence
<220>
   <223> primer P20
<400> 54
   aaaaccgccc gggcgttctc ac 22
<210> 55
   <211> 39
   <212> DNA
   <213> Artificial Sequence
<220>
   <223> primer P21
<220>
   <221> misc_feature
   <222> (13)..(15)
   <223> n is a, c, g, or t
<400> 55
   agctgagtcg acnnngtggt ggcaaccttt aaaaaactg 39
<210> 56
   <211> 192
   <212> DNA
   <213> Pantoea ananatis
<400> 56
   gcatgcacgc gtggcgatct ggcctgactg ccttgttagc atttcttcat aactgtttca      60
   tggaatcagg tagttgatat tgctactatc cagttcattc aacgaaaatc cagcgtttaa     120
   cgtgccgcac agtgtattgt gctggtgaga cgagtaagtc agttttttaa aggttgccac     180
   cacggagtcg ac                                                         192
<210> 57
   <211> 35
   <212> DNA
   <213> Artificial Sequence
<220>
   <223> primer P22
<400> 57
   agctgagtcg acgtgagtac attagaacaa acaat 35
<210> 58
   <211> 33
   <212> DNA
   <213> Artificial Sequence
<220>
   <223> primer P23
<400> 58
   agctgatcta gaagtctccg atgctattaa tcc 33
<210> 59
   <211> 80
   <212> DNA
   <213> Artificial Sequence
<220>
   <223> Dd0191-FW
<400> 59
   ccgtgtctga agcctatttt gcccgcctgc tgggcttgcc ttttattgcc tgaagcctgc      60
   ttttttatac taagttggca                                                  80
<210> 60
   <211> 80
   <212> DNA
   <213> Artificial Sequence
<220>
   <223> Dd0191-RV
<400> 60
   ctagcccagt tcgcgctgcc agggcgtaat atcgccaatg tgctcggcaa cgctcaagtt      60
   agtataaaaa agctgaacga                                                  80
<210> 61
   <211> 1800
   <212> DNA
   <213> Escherichia coli
<220>
   <221> CDS
   <222> (1)..(1800)
<400> 61
   atg acg aca cag gtc cca cct tcc gcg ttg ctt ccg ttg aac ccg gag       48
   Met Thr Thr Gln Val Pro Pro Ser Ala Leu Leu Pro Leu Asn Pro Glu
   1               5                   10                  15
   caa ctg gca cgc ctt cag gcg gcc acg acc gat tta act ccc acc cag       96
   Gln Leu Ala Arg Leu Gln Ala Ala Thr Thr Asp Leu Thr Pro Thr Gln
               20                  25                  30
   ctt gcc tgg gtt tct ggc tat ttc tgg ggc gta ctc aat cag cag cct      144
   Leu Ala Trp Val Ser Gly Tyr Phe Trp Gly Val Leu Asn Gln Gln Pro
           35                  40                  45
   gct gcg ctt gca gcg acg cca gcg cca gcc gca gaa atg ccg ggt ata      192
   Ala Ala Leu Ala Ala Thr Pro Ala Pro Ala Ala Glu Met Pro Gly Ile
       50                  55                  60
   act att atc tcc gcc tcg caa acc ggc aat gcg cgc cgg gtt gct gaa      240
   Thr Ile Ile Ser Ala Ser Gln Thr Gly Asn Ala Arg Arg Val Ala Glu
   65                  70                  75                  80
   gca tta cgt gat gat tta tta gca gca aaa ctg aac gtt aag ctg gtg      288
   Ala Leu Arg Asp Asp Leu Leu Ala Ala Lys Leu Asn Val Lys Leu Val
                   85                  90                  95
   aac gcg ggc gac tat aaa ttc aaa caa atc gcc agc gaa aaa ctg ctc      336
   Asn Ala Gly Asp Tyr Lys Phe Lys Gln Ile Ala Ser Glu Lys Leu Leu
               100                 105                 110
   atc gta gtg acg tca acg caa ggg gaa ggg gaa ccg ccg gaa gaa gcc      384
   Ile Val Val Thr Ser Thr Gln Gly Glu Gly Glu Pro Pro Glu Glu Ala
           115                 120                 125
   gtc gcg ctg cat aag ttc ctg ttc tcc aaa aaa gcg cca aag ctg gaa      432
   Val Ala Leu His Lys Phe Leu Phe Ser Lys Lys Ala Pro Lys Leu Glu
       130                 135                 140
   aac acc gcg ttt gcc gtg ttt agc ctc ggc gat agc tct tat gaa ttt      480
   Asn Thr Ala Phe Ala Val Phe Ser Leu Gly Asp Ser Ser Tyr Glu Phe
   145                 150                 155                 160
   ttc tgc cag tcc ggg aaa gat ttc gac agc aag ctg gcg gaa ctg ggt      528
   Phe Cys Gln Ser Gly Lys Asp Phe Asp Ser Lys Leu Ala Glu Leu Gly
                   165                 170                 175
   ggt gaa cgc ctg ctc gac cgt gtc gat gcc gat gtt gaa tac cag gct      576
   Gly Glu Arg Leu Leu Asp Arg Val Asp Ala Asp Val Glu Tyr Gln Ala
               180                 185                 190
   gct gcc agc gag tgg cgc gcc cgc gtg gtt gat gcg ctt aaa tcg cgt      624
   Ala Ala Ser Glu Trp Arg Ala Arg Val Val Asp Ala Leu Lys Ser Arg
           195                 200                 205
   gcg cct gtc gcg gca cct tcg caa tcc gtc gct act ggc gcg gta aat      672
   Ala Pro Val Ala Ala Pro Ser Gln Ser Val Ala Thr Gly Ala Val Asn
       210                 215                 220
   gaa atc cac acc agc ccg tac agc aaa gac gcg ccg ctg gtg gct agc      720
   Glu Ile His Thr Ser Pro Tyr Ser Lys Asp Ala Pro Leu Val Ala Ser
   225                 230                 235                 240
   ctc tct gtt aac cag aaa att acc ggg cgt aac tct gaa aaa gac gtt      768
   Leu Ser Val Asn Gln Lys Ile Thr Gly Arg Asn Ser Glu Lys Asp Val
                   245                 250                 255
   cgc cat atc gaa att gac tta ggt gac tcg ggc atg cgt tac cag ccg      816
   Arg His Ile Glu Ile Asp Leu Gly Asp Ser Gly Met Arg Tyr Gln Pro
               260                 265                 270
   ggt gac gcg ctg ggc gtc tgg tat cag aac gat ccg gca ctg gtg aaa      864
   Gly Asp Ala Leu Gly Val Trp Tyr Gln Asn Asp Pro Ala Leu Val Lys
           275                 280                 285
   gaa ctt gtc gaa ctg ctg tgg ctg aaa ggc gat gaa cct gtc acc gtc      912
   Glu Leu Val Glu Leu Leu Trp Leu Lys Gly Asp Glu Pro Val Thr Val
       290                 295                 300
   gag ggc aaa acg ttg cct ctg aac gaa gcg cta cag tgg cac ttc gaa      960
   Glu Gly Lys Thr Leu Pro Leu Asn Glu Ala Leu Gln Trp His Phe Glu
   305                 310                 315                 320
   ctg acc gtc aac acc gcc aac att gtt gag aat tac gcc acg ctt acc     1008
   Leu Thr Val Asn Thr Ala Asn Ile Val Glu Asn Tyr Ala Thr Leu Thr
                   325                 330                 335
   cgc agt gaa aca ctg ctg ccg ctg gtg ggc gat aaa gcg aag tta cag     1056
   Arg Ser Glu Thr Leu Leu Pro Leu Val Gly Asp Lys Ala Lys Leu Gln
               340                 345                 350
   cat tac gcc gcg acg acg ccg att gtt gac atg gtg cgt ttc tcc ccg     1104
   His Tyr Ala Ala Thr Thr Pro Ile Val Asp Met Val Arg Phe Ser Pro
           355                 360                 365
   gca cag ctt gat gcc gaa gcg cta att aat ctg ctg cgc ccg ctg acg     1152
   Ala Gln Leu Asp Ala Glu Ala Leu Ile Asn Leu Leu Arg Pro Leu Thr
       370                 375                 380
   ccg cgt ctg tat tcc atc gcc tcc tcg cag gcg gaa gtc gag aac gaa     1200
   Pro Arg Leu Tyr Ser Ile Ala Ser Ser Gln Ala Glu Val Glu Asn Glu
   385                 390                 395                 400
   gta cac gtc acc gtt ggt gtg gtg cgt tac gac gtg gaa ggc cgc gcc     1248
   Val His Val Thr Val Gly Val Val Arg Tyr Asp Val Glu Gly Arg Ala
                   405                 410                 415
   cgt gcc ggt ggt gcc tcc agc ttc ctc gct gac cgc gtg gaa gaa gag     1296
   Arg Ala Gly Gly Ala Ser Ser Phe Leu Ala Asp Arg Val Glu Glu Glu
               420                 425                 430
   ggc gaa gtc cgc gta ttt atc gaa cat aac gat aac ttc cgc ctg cca     1344
   Gly Glu Val Arg Val Phe Ile Glu His Asn Asp Asn Phe Arg Leu Pro
           435                 440                 445
   gcc aat cca gaa acc ccg gtg att atg att ggc cca ggc acc ggt att      1392
   Ala Asn Pro Glu Thr Pro Val Ile Met Ile Gly Pro Gly Thr Gly Ile
       450                 455                 460
   gcg ccg ttc cgc gcc ttt atg cag caa cgc gcc gcc gac gaa gcg cca      1440
   Ala Pro Phe Arg Ala Phe Met Gln Gln Arg Ala Ala Asp Glu Ala Pro
   465                 470                 475                 480
   ggt aaa aac tgg ctg ttc ttt ggt aat ccg cac ttt acg gaa gac ttc      1488
   Gly Lys Asn Trp Leu Phe Phe Gly Asn Pro His Phe Thr Glu Asp Phe
                   485                 490                 495
   ctg tac cag gtg gag tgg cag cgc tac gtc aaa gat ggc gtg ctg aca      1536
   Leu Tyr Gln Val Glu Trp Gln Arg Tyr Val Lys Asp Gly Val Leu Thr
               500                 505                 510
   cgt atc gat ctt gcc tgg tcg cgc gat caa aaa gaa aaa gtt tac gta      1584
   Arg Ile Asp Leu Ala Trp Ser Arg Asp Gln Lys Glu Lys Val Tyr Val
           515                 520                 525
   caa gac aaa ctg cgc gaa cag ggc gcg gag ctg tgg cgc tgg atc aat      1632
   Gln Asp Lys Leu Arg Glu Gln Gly Ala Glu Leu Trp Arg Trp Ile Asn
       530                 535                 540
   gat ggt gcc cac att tat gtc tgc ggc gac gct aat cgc atg gcg aaa      1680
   Asp Gly Ala His Ile Tyr Val Cys Gly Asp Ala Asn Arg Met Ala Lys
   545                 550                 555                 560
   gac gtt gag cag gca ctt ctg gaa gtg att gcc gaa ttt ggt ggc atg      1728
   Asp Val Glu Gln Ala Leu Leu Glu Val Ile Ala Glu Phe Gly Gly Met
                   565                 570                 575
   gac acc gaa gcg gcg gat gaa ttt tta agt gag ctg cgc gta gag cgc      1776
   Asp Thr Glu Ala Ala Asp Glu Phe Leu Ser Glu Leu Arg Val Glu Arg
               580                 585                 590
   cgt tat cag cga gat gtc tac taa                                      1800
   Arg Tyr Gln Arg Asp Val Tyr
           595
<210> 62
   <211> 599
   <212> PRT
   <213> Escherichia coli
<400> 62
   Met Thr Thr Gln Val Pro Pro Ser Ala Leu Leu Pro Leu Asn Pro Glu
   1               5                   10                  15
   Gln Leu Ala Arg Leu Gln Ala Ala Thr Thr Asp Leu Thr Pro Thr Gln
               20                  25                  30
   Leu Ala Trp Val Ser Gly Tyr Phe Trp Gly Val Leu Asn Gln Gln Pro
           35                  40                  45
   Ala Ala Leu Ala Ala Thr Pro Ala Pro Ala Ala Glu Met Pro Gly Ile
       50                  55                  60
   Thr Ile Ile Ser Ala Ser Gln Thr Gly Asn Ala Arg Arg Val Ala Glu
   65                  70                  75                  80
   Ala Leu Arg Asp Asp Leu Leu Ala Ala Lys Leu Asn Val Lys Leu Val
                   85                  90                  95
   Asn Ala Gly Asp Tyr Lys Phe Lys Gln Ile Ala Ser Glu Lys Leu Leu
               100                 105                 110
   Ile Val Val Thr Ser Thr Gln Gly Glu Gly Glu Pro Pro Glu Glu Ala
           115                 120                 125
   Val Ala Leu His Lys Phe Leu Phe Ser Lys Lys Ala Pro Lys Leu Glu
       130                 135                 140
   Asn Thr Ala Phe Ala Val Phe Ser Leu Gly Asp Ser Ser Tyr Glu Phe
   145                 150                 155                 160
   Phe Cys Gln Ser Gly Lys Asp Phe Asp Ser Lys Leu Ala Glu Leu Gly
                   165                 170                 175
   Gly Glu Arg Leu Leu Asp Arg Val Asp Ala Asp Val Glu Tyr Gln Ala
               180                 185                 190
   Ala Ala Ser Glu Trp Arg Ala Arg Val Val Asp Ala Leu Lys Ser Arg
           195                 200                 205
   Ala Pro Val Ala Ala Pro Ser Gln Ser Val Ala Thr Gly Ala Val Asn
       210                 215                 220
   Glu Ile His Thr Ser Pro Tyr Ser Lys Asp Ala Pro Leu Val Ala Ser
   225                 230                 235                 240
   Leu Ser Val Asn Gln Lys Ile Thr Gly Arg Asn Ser Glu Lys Asp Val
                   245                 250                 255
   Arg His Ile Glu Ile Asp Leu Gly Asp Ser Gly Met Arg Tyr Gln Pro
               260                 265                 270
   Gly Asp Ala Leu Gly Val Trp Tyr Gln Asn Asp Pro Ala Leu Val Lys
           275                 280                 285
   Glu Leu Val Glu Leu Leu Trp Leu Lys Gly Asp Glu Pro Val Thr Val
       290                 295                 300
   Glu Gly Lys Thr Leu Pro Leu Asn Glu Ala Leu Gln Trp His Phe Glu
   305                 310                 315                 320
   Leu Thr Val Asn Thr Ala Asn Ile Val Glu Asn Tyr Ala Thr Leu Thr
                   325                 330                 335
   Arg Ser Glu Thr Leu Leu Pro Leu Val Gly Asp Lys Ala Lys Leu Gln
               340                 345                 350
   His Tyr Ala Ala Thr Thr Pro Ile Val Asp Met Val Arg Phe Ser Pro
           355                 360                 365
   Ala Gln Leu Asp Ala Glu Ala Leu Ile Asn Leu Leu Arg Pro Leu Thr
       370                 375                 380
   Pro Arg Leu Tyr Ser Ile Ala Ser Ser Gln Ala Glu Val Glu Asn Glu
   385                 390                 395                 400
   Val His Val Thr Val Gly Val Val Arg Tyr Asp Val Glu Gly Arg Ala
                   405                 410                 415
   Arg Ala Gly Gly Ala Ser Ser Phe Leu Ala Asp Arg Val Glu Glu Glu
               420                 425                 430
   Gly Glu Val Arg Val Phe Ile Glu His Asn Asp Asn Phe Arg Leu Pro
           435                 440                 445
   Ala Asn Pro Glu Thr Pro Val Ile Met Ile Gly Pro Gly Thr Gly Ile
       450                 455                 460
   Ala Pro Phe Arg Ala Phe Met Gln Gln Arg Ala Ala Asp Glu Ala Pro
   465                 470                 475                 480
   Gly Lys Asn Trp Leu Phe Phe Gly Asn Pro His Phe Thr Glu Asp Phe
                   485                 490                 495
   Leu Tyr Gln Val Glu Trp Gln Arg Tyr Val Lys Asp Gly Val Leu Thr
               500                 505                 510
   Arg Ile Asp Leu Ala Trp Ser Arg Asp Gln Lys Glu Lys Val Tyr Val
           515                 520                 525
   Gln Asp Lys Leu Arg Glu Gln Gly Ala Glu Leu Trp Arg Trp Ile Asn
       530                 535                 540
   Asp Gly Ala His Ile Tyr Val Cys Gly Asp Ala Asn Arg Met Ala Lys
   545                 550                 555                 560
   Asp Val Glu Gln Ala Leu Leu Glu Val Ile Ala Glu Phe Gly Gly Met
                   565                 570                 575
   Asp Thr Glu Ala Ala Asp Glu Phe Leu Ser Glu Leu Arg Val Glu Arg
               580                 585                 590
   Arg Tyr Gln Arg Asp Val Tyr
           595
<210> 63
   <211> 1803
   <212> DNA
   <213> Pantoea ananatis
<220>
   <221> CDS
   <222> (1)..(1803)
<400> 63
   atg acg act cag gca cca ggc tca atg ctt ccg ctc tct ccg gag caa       48
   Met Thr Thr Gln Ala Pro Gly Ser Met Leu Pro Leu Ser Pro Glu Gln
   1               5                   10                  15
   ctt gct cgt tta cag gca gca acg aca gat ttt tcc acc act cag atg       96
   Leu Ala Arg Leu Gln Ala Ala Thr Thr Asp Phe Ser Thr Thr Gln Met
               20                  25                  30
   gcc tgg ctc tct ggc tat ttc tgg ggc atg gtc aat caa atg cca ggc      144
   Ala Trp Leu Ser Gly Tyr Phe Trp Gly Met Val Asn Gln Met Pro Gly
           35                  40                  45
   cag gcg gtt gcc agc gcg cct gcg cag gct gaa acc ccg gcg att acg      192
   Gln Ala Val Ala Ser Ala Pro Ala Gln Ala Glu Thr Pro Ala Ile Thr
       50                  55                  60
   ctg ctc tct gcc tca caa acc ggc aat gcc cgc cgg gta gcc gag cag      240
   Leu Leu Ser Ala Ser Gln Thr Gly Asn Ala Arg Arg Val Ala Glu Gln
   65                  70                  75                  80
   tta cgc gat gac ctg cag gcg gca aaa ctg acc gtg aat ctg gtc aac      288
   Leu Arg Asp Asp Leu Gln Ala Ala Lys Leu Thr Val Asn Leu Val Asn
                   85                  90                  95
   gcg ggc gac ttt aaa ttt aag cag att ggc cag gaa aaa att ctg att      336
   Ala Gly Asp Phe Lys Phe Lys Gln Ile Gly Gln Glu Lys Ile Leu Ile
               100                 105                 110
   gtc gtg acc tcg acg cag ggc gaa ggc gaa cca ccg gaa gag gcc gtt      384
   Val Val Thr Ser Thr Gln Gly Glu Gly Glu Pro Pro Glu Glu Ala Val
           115                 120                 125
   gcg ctg cat aaa ttc ctg atg tct aag aaa gcg ccc aaa atg gac ggc      432
   Ala Leu His Lys Phe Leu Met Ser Lys Lys Ala Pro Lys Met Asp Gly
       130                 135                 140
   gcg gcc ttt gcc gtg ttt ggt ctg ggc gat acc tcc tat gaa ttc ttc      480
   Ala Ala Phe Ala Val Phe Gly Leu Gly Asp Thr Ser Tyr Glu Phe Phe
   145                 150                 155                 160
   agc aaa gcg ggc aaa gat ttt gac agc cgc ctg gcc gag ctg ggt gca      528
   Ser Lys Ala Gly Lys Asp Phe Asp Ser Arg Leu Ala Glu Leu Gly Ala
                   165                 170                 175
   gaa cga ctg ctg gat cgg gtc gat gcg gat gtt gaa tat gcc gag cag      576
   Glu Arg Leu Leu Asp Arg Val Asp Ala Asp Val Glu Tyr Ala Glu Gln
               180                 185                 190
   gcc agc gcc tgg cgc gcg gca att act gcc gca ctg aaa gaa cgc gta      624
   Ala Ser Ala Trp Arg Ala Ala Ile Thr Ala Ala Leu Lys Glu Arg Val
           195                 200                 205
   ccg gca gcg tca ccg gcc cag agt gcg gca acc gtc gcc ggc agc gtg      672
   Pro Ala Ala Ser Pro Ala Gln Ser Ala Ala Thr Val Ala Gly Ser Val
       210                 215                 220
   aac gaa gtc ttc acc agc cct tac act aaa gag cag ccg ctt agt gcc      720
   Asn Glu Val Phe Thr Ser Pro Tyr Thr Lys Glu Gln Pro Leu Ser Ala
   225                 230                 235                 240
   agc ctg gcg gtt aac cag aaa att acc ggg cgc gat tcg gac aaa gat      768
   Ser Leu Ala Val Asn Gln Lys Ile Thr Gly Arg Asp Ser Asp Lys Asp
                   245                 250                 255
   gtg cgt cac atc gaa atc gat ctg ggt gat tca gga tta cgc tac cag      816
   Val Arg His Ile Glu Ile Asp Leu Gly Asp Ser Gly Leu Arg Tyr Gln
               260                 265                 270
   cct ggt gat gcg ctg ggc gtc tgg tat gag aat gac gcg gcc ctg gtt      864
   Pro Gly Asp Ala Leu Gly Val Trp Tyr Glu Asn Asp Ala Ala Leu Val
           275                 280                 285
   aac gag ctg ctt gaa ctg gtg tgg cta aaa ggc gat gag ccg gtt gag      912
   Asn Glu Leu Leu Glu Leu Val Trp Leu Lys Gly Asp Glu Pro Val Glu
       290                 295                 300
   gtg cag ggc cag acg ctg cca ttg gca gag gcg ttg cag aaa cat ttt      960
   Val Gln Gly Gln Thr Leu Pro Leu Ala Glu Ala Leu Gln Lys His Phe
   305                 310                 315                 320
   gag cta acg gtt aac acc gcg caa atc gtc gcg cag tac gca gcc tta     1008
   Glu Leu Thr Val Asn Thr Ala Gln Ile Val Ala Gln Tyr Ala Ala Leu
                   325                 330                 335
   gca cgt aac gac gaa ctg ctg tcg ctg gtg gat gac aaa gcc aaa ctg      1056
   Ala Arg Asn Asp Glu Leu Leu Ser Leu Val Asp Asp Lys Ala Lys Leu
               340                 345                 350
   cag cag tat gcg caa cgc tat ccg att gtt gat atg gtt cgt ctg gca      1104
   Gln Gln Tyr Ala Gln Arg Tyr Pro Ile Val Asp Met Val Arg Leu Ala
           355                 360                 365
   cca gcc caa ctg agc gca gaa cag tta tcc ggc ttg ctg cgt ccg tta      1152
   Pro Ala Gln Leu Ser Ala Glu Gln Leu Ser Gly Leu Leu Arg Pro Leu
       370                 375                 380
   acg ccg cgt ctt tac tct atc gct tcg tct cag gcc gaa acc gat acc      1200
   Thr Pro Arg Leu Tyr Ser Ile Ala Ser Ser Gln Ala Glu Thr Asp Thr
   385                 390                 395                 400
   gaa gta cat atc acc gtg ggg gcc gtg cgc ttt gat att gaa ggg cgc      1248
   Glu Val His Ile Thr Val Gly Ala Val Arg Phe Asp Ile Glu Gly Arg
                   405                 410                 415
   ccg cgc ggc ggc ggc gct tcc acc tgg ctg gcg gat cgc att gaa gaa      1296
   Pro Arg Gly Gly Gly Ala Ser Thr Trp Leu Ala Asp Arg Ile Glu Glu
               420                 425                 430
   gac ggt gag gtg cgc gtg ttt atc gaa cac aac gat aat ttt cgc ctg      1344
   Asp Gly Glu Val Arg Val Phe Ile Glu His Asn Asp Asn Phe Arg Leu
           435                 440                 445
   ccc gcc aac ccg gat gca ccg gtc att atg att ggg cct ggc acc ggt      1392
   Pro Ala Asn Pro Asp Ala Pro Val Ile Met Ile Gly Pro Gly Thr Gly
       450                 455                 460
   att gcg ccg ttc cgc gcc ttt atg cag cag cgc gag aac gac ggc gcc      1440
   Ile Ala Pro Phe Arg Ala Phe Met Gln Gln Arg Glu Asn Asp Gly Ala
   465                 470                 475                 480
   agc ggt aaa aac tgg cta ttc ttt ggc aat ccc cac ttt act gac gat      1488
   Ser Gly Lys Asn Trp Leu Phe Phe Gly Asn Pro His Phe Thr Asp Asp
                   485                 490                 495
   ttc ctg tat cag gtg gaa tgg cag aaa tac gtg aaa gat ggc ctg tta      1536
   Phe Leu Tyr Gln Val Glu Trp Gln Lys Tyr Val Lys Asp Gly Leu Leu
               500                 505                 510
   acg aat atc gat ctg gcc tgg tcg cgc gat cag gcc gag aaa att tac      1584
   Thr Asn Ile Asp Leu Ala Trp Ser Arg Asp Gln Ala Glu Lys Ile Tyr
           515                 520                 525
   gta caa gat aaa atc cgc gcc aag ggt gcg gag gtg tgg agc tgg tta      1632
   Val Gln Asp Lys Ile Arg Ala Lys Gly Ala Glu Val Trp Ser Trp Leu
       530                 535                 540
   cag gaa ggc gcg cac ctt tac gtc tgc ggc gac gcc aat cgt atg gca      1680
   Gln Glu Gly Ala His Leu Tyr Val Cys Gly Asp Ala Asn Arg Met Ala
   545                 550                 555                 560
   aaa gac gtt gag cag gca tta ctg gat gtg gtg gtc gaa cac ggc gct      1728
   Lys Asp Val Glu Gln Ala Leu Leu Asp Val Val Val Glu His Gly Ala
                   565                 570                 575
   atg gat cgt gaa acg gct gac gaa ttt tta agt gag ctg cgc att gag      1776
   Met Asp Arg Glu Thr Ala Asp Glu Phe Leu Ser Glu Leu Arg Ile Glu
               580                 585                 590
   cgc cgt tat cag cga gac gtt tac taa                                  1803
   Arg Arg Tyr Gln Arg Asp Val Tyr
           595                 600
<210> 64
   <211> 600
   <212> PRT
   <213> Pantoea ananatis
<400> 64
   Met Thr Thr Gln Ala Pro Gly Ser Met Leu Pro Leu Ser Pro Glu Gln
   1               5                   10                  15
   Leu Ala Arg Leu Gln Ala Ala Thr Thr Asp Phe Ser Thr Thr Gln Met
               20                  25                  30
   Ala Trp Leu Ser Gly Tyr Phe Trp Gly Met Val Asn Gln Met Pro Gly
           35                  40                  45
   Gln Ala Val Ala Ser Ala Pro Ala Gln Ala Glu Thr Pro Ala Ile Thr
       50                  55                  60
   Leu Leu Ser Ala Ser Gln Thr Gly Asn Ala Arg Arg Val Ala Glu Gln
   65                  70                  75                  80
   Leu Arg Asp Asp Leu Gln Ala Ala Lys Leu Thr Val Asn Leu Val Asn
                   85                  90                  95
   Ala Gly Asp Phe Lys Phe Lys Gln Ile Gly Gln Glu Lys Ile Leu Ile
               100                 105                 110
   Val Val Thr Ser Thr Gln Gly Glu Gly Glu Pro Pro Glu Glu Ala Val
           115                 120                 125
   Ala Leu His Lys Phe Leu Met Ser Lys Lys Ala Pro Lys Met Asp Gly
       130                 135                 140
   Ala Ala Phe Ala Val Phe Gly Leu Gly Asp Thr Ser Tyr Glu Phe Phe
   145                 150                 155                 160
   Ser Lys Ala Gly Lys Asp Phe Asp Ser Arg Leu Ala Glu Leu Gly Ala
                   165                 170                 175
   Glu Arg Leu Leu Asp Arg Val Asp Ala Asp Val Glu Tyr Ala Glu Gln
               180                 185                 190
   Ala Ser Ala Trp Arg Ala Ala Ile Thr Ala Ala Leu Lys Glu Arg Val
           195                 200                 205
   Pro Ala Ala Ser Pro Ala Gln Ser Ala Ala Thr Val Ala Gly Ser Val
       210                 215                 220
   Asn Glu Val Phe Thr Ser Pro Tyr Thr Lys Glu Gln Pro Leu Ser Ala
   225                 230                 235                 240
   Ser Leu Ala Val Asn Gln Lys Ile Thr Gly Arg Asp Ser Asp Lys Asp
                   245                 250                 255
   Val Arg His Ile Glu Ile Asp Leu Gly Asp Ser Gly Leu Arg Tyr Gln
               260                 265                 270
   Pro Gly Asp Ala Leu Gly Val Trp Tyr Glu Asn Asp Ala Ala Leu Val
           275                 280                 285
   Asn Glu Leu Leu Glu Leu Val Trp Leu Lys Gly Asp Glu Pro Val Glu
       290                 295                 300
   Val Gln Gly Gln Thr Leu Pro Leu Ala Glu Ala Leu Gln Lys His Phe
   305                 310                 315                 320
   Glu Leu Thr Val Asn Thr Ala Gln Ile Val Ala Gln Tyr Ala Ala Leu
                   325                 330                 335
   Ala Arg Asn Asp Glu Leu Leu Ser Leu Val Asp Asp Lys Ala Lys Leu
               340                 345                 350
   Gln Gln Tyr Ala Gln Arg Tyr Pro Ile Val Asp Met Val Arg Leu Ala
           355                 360                 365
   Pro Ala Gln Leu Ser Ala Glu Gln Leu Ser Gly Leu Leu Arg Pro Leu
       370                 375                 380
   Thr Pro Arg Leu Tyr Ser Ile Ala Ser Ser Gln Ala Glu Thr Asp Thr
   385                 390                 395                 400
   Glu Val His Ile Thr Val Gly Ala Val Arg Phe Asp Ile Glu Gly Arg
                   405                 410                 415
   Pro Arg Gly Gly Gly Ala Ser Thr Trp Leu Ala Asp Arg Ile Glu Glu
               420                 425                 430
   Asp Gly Glu Val Arg Val Phe Ile Glu His Asn Asp Asn Phe Arg Leu
           435                 440                 445
   Pro Ala Asn Pro Asp Ala Pro Val Ile Met Ile Gly Pro Gly Thr Gly
       450                 455                 460
   Ile Ala Pro Phe Arg Ala Phe Met Gln Gln Arg Glu Asn Asp Gly Ala
   465                 470                 475                 480
   Ser Gly Lys Asn Trp Leu Phe Phe Gly Asn Pro His Phe Thr Asp Asp
                   485                 490                 495
   Phe Leu Tyr Gln Val Glu Trp Gln Lys Tyr Val Lys Asp Gly Leu Leu
               500                 505                 510
   Thr Asn Ile Asp Leu Ala Trp Ser Arg Asp Gln Ala Glu Lys Ile Tyr
           515                 520                 525
   Val Gln Asp Lys Ile Arg Ala Lys Gly Ala Glu Val Trp Ser Trp Leu
       530                 535                 540
   Gln Glu Gly Ala His Leu Tyr Val Cys Gly Asp Ala Asn Arg Met Ala
   545                 550                 555                 560
   Lys Asp Val Glu Gln Ala Leu Leu Asp Val Val Val Glu His Gly Ala
                   565                 570                 575
   Met Asp Arg Glu Thr Ala Asp Glu Phe Leu Ser Glu Leu Arg Ile Glu
               580                 585                 590
   Arg Arg Tyr Gln Arg Asp Val Tyr
           595                 600
<210> 65
   <211> 1713
   <212> DNA
   <213> Escherichia coli
<220>
   <221> CDS
   <222> (1)..(1713)
<400> 65
   atg agc gaa aaa cat cca ggg cct tta gtg gtc gaa gga aaa ctg aca      48
   Met Ser Glu Lys His Pro Gly Pro Leu Val Val Glu Gly Lys Leu Thr
   1               5                   10                  15
   gac gcc gag cgc atg aag cat gaa agc aac tac ctg cgc ggc acc att      96
   Asp Ala Glu Arg Met Lys His Glu Ser Asn Tyr Leu Arg Gly Thr Ile
               20                  25                  30
   gcg gaa gat tta aac gac ggt ctg acc ggc ggc ttt aag ggc gac aac     144
   Ala Glu Asp Leu Asn Asp Gly Leu Thr Gly Gly Phe Lys Gly Asp Asn
           35                  40                  45
   ttc ctg ctg att cgc ttc cac ggc atg tat cag cag gat gac cgc gac     192
   Phe Leu Leu Ile Arg Phe His Gly Met Tyr Gln Gln Asp Asp Arg Asp
       50                  55                  60
   atc cgc gcc gaa cgt gct gaa cag aag ctg gag ccg cgc cac gcg atg     240
   Ile Arg Ala Glu Arg Ala Glu Gln Lys Leu Glu Pro Arg His Ala Met
   65                  70                  75                  80
   ctg ctt cgc tgt cgt ctg ccg ggt ggg gtg att acc act aaa cag tgg     288
   Leu Leu Arg Cys Arg Leu Pro Gly Gly Val Ile Thr Thr Lys Gln Trp
                   85                  90                  95
   cag gcg atc gac aaa ttt gcc ggt gaa aac acc atc tat ggc agc att     336
   Gln Ala Ile Asp Lys Phe Ala Gly Glu Asn Thr Ile Tyr Gly Ser Ile
               100                 105                 110
   cgc ctg acc aac cgc cag acg ttt cag ttc cac ggc att ctg aaa aag     384
   Arg Leu Thr Asn Arg Gln Thr Phe Gln Phe His Gly Ile Leu Lys Lys
           115                 120                 125
   aac gtc aaa ccg gtg cac cag atg ctg cac tcg gtc ggt ctt gat gcg     432
   Asn Val Lys Pro Val His Gln Met Leu His Ser Val Gly Leu Asp Ala
       130                 135                 140
   ctg gcg aca gct aac gac atg aac cgt aac gta ctc tgc acc tcg aac     480
   Leu Ala Thr Ala Asn Asp Met Asn Arg Asn Val Leu Cys Thr Ser Asn
   145                 150                 155                 160
   cct tac gag tcg cag ctg cac gcg gaa gcg tac gag tgg gcg aag aag     528
   Pro Tyr Glu Ser Gln Leu His Ala Glu Ala Tyr Glu Trp Ala Lys Lys
                   165                 170                 175
   att tct gag cat ctg ttg cct cgt acc cgc gcg tat gcg gag atc tgg     576
   Ile Ser Glu His Leu Leu Pro Arg Thr Arg Ala Tyr Ala Glu Ile Trp
               180                 185                 190
   ctc gac cag gaa aaa gtc gcc act act gat gaa gaa ccg atc ctc ggc     624
   Leu Asp Gln Glu Lys Val Ala Thr Thr Asp Glu Glu Pro Ile Leu Gly
           195                 200                 205
   cag acc tac ctg ccg cgt aaa ttc aaa acc acg gta gtg atc ccg cca     672
   Gln Thr Tyr Leu Pro Arg Lys Phe Lys Thr Thr Val Val Ile Pro Pro
       210                 215                 220
   cag aac gat atc gat ctg cac gcc aac gac atg aac ttc gtg gcg atc      720
   Gln Asn Asp Ile Asp Leu His Ala Asn Asp Met Asn Phe Val Ala Ile
   225                 230                 235                 240
   gcc gaa aac ggc aag ctg gtg ggc ttt aac ctg ttg gtg ggc ggt ggg      768
   Ala Glu Asn Gly Lys Leu Val Gly Phe Asn Leu Leu Val Gly Gly Gly
                   245                 250                 255
   ctt tcc atc gaa cac ggc aac aag aaa acc tac gcc cgc acc gcg agt      816
   Leu Ser Ile Glu His Gly Asn Lys Lys Thr Tyr Ala Arg Thr Ala Ser
               260                 265                 270
   gag ttt ggc tat ctg ccg ctg gag cat acg ctg gcg gtg gcc gaa gcc      864
   Glu Phe Gly Tyr Leu Pro Leu Glu His Thr Leu Ala Val Ala Glu Ala
           275                 280                 285
   gtc gtg aca act cag cgt gac tgg ggt aac cga acc gat cgt aaa aat      912
   Val Val Thr Thr Gln Arg Asp Trp Gly Asn Arg Thr Asp Arg Lys Asn
       290                 295                 300
   gcc aaa acc aaa tac acg ctg gag cgc gtg ggg gtt gag acg ttt aaa      960
   Ala Lys Thr Lys Tyr Thr Leu Glu Arg Val Gly Val Glu Thr Phe Lys
   305                 310                 315                 320
   gcg gaa gtg gag cgt cgc gcg ggg atc aaa ttt gaa ccg atc cgt cca     1008
   Ala Glu Val Glu Arg Arg Ala Gly Ile Lys Phe Glu Pro Ile Arg Pro
                   325                 330                 335
   tat gag ttc acc gga cga ggc gat cgt att ggc tgg gtt aag ggc att     1056
   Tyr Glu Phe Thr Gly Arg Gly Asp Arg Ile Gly Trp Val Lys Gly Ile
               340                 345                 350
   gat gat aac tgg cac ctg acg ctg ttt atc gaa aat ggt cgc atc ctt     1104
   Asp Asp Asn Trp His Leu Thr Leu Phe Ile Glu Asn Gly Arg Ile Leu
           355                 360                 365
   gat tat ccg gcg cgt ccg ctg aaa acc ggc ctg ctg gag atc gcg aag     1152
   Asp Tyr Pro Ala Arg Pro Leu Lys Thr Gly Leu Leu Glu Ile Ala Lys
       370                 375                 380
   atc cac aaa ggc gat ttc cgc att acg gcg aac cag aat ctg atc atc     1200
   Ile His Lys Gly Asp Phe Arg Ile Thr Ala Asn Gln Asn Leu Ile Ile
   385                 390                 395                 400
   gcc ggt gta ccg gaa agc gag aaa gcg aag atc gag aag atc gcc aaa     1248
   Ala Gly Val Pro Glu Ser Glu Lys Ala Lys Ile Glu Lys Ile Ala Lys
                   405                 410                 415
   gag agc ggg tta atg aat gcc gtc acg ccg cag cgt gaa aac tcg atg     1296
   Glu Ser Gly Leu Met Asn Ala Val Thr Pro Gln Arg Glu Asn Ser Met
               420                 425                 430
   gct tgc gtg tca ttc ccg act tgc ccg ctg gcg atg gcg gaa gca gag     1344
   Ala Cys Val Ser Phe Pro Thr Cys Pro Leu Ala Met Ala Glu Ala Glu
           435                 440                 445
   cgt ttc ctg ccg tct ttt atc gac aac atc gat aat tta atg gcg aaa     1392
   Arg Phe Leu Pro Ser Phe Ile Asp Asn Ile Asp Asn Leu Met Ala Lys
       450                 455                 460
   cat ggt gtc agc gat gag cat atc gtg atg cgt gta aca ggc tgc ccg     1440
   His Gly Val Ser Asp Glu His Ile Val Met Arg Val Thr Gly Cys Pro
   465                 470                 475                 480
   aac ggt tgt ggt cgc gcg atg ctg gcg gaa gtg ggc ctg gtg ggt aaa     1488
   Asn Gly Cys Gly Arg Ala Met Leu Ala Glu Val Gly Leu Val Gly Lys
                   485                 490                 495
   gcg ccg ggt cgc tac aac ctg cat ctt ggc ggc aac cgc att ggg aca     1536
   Ala Pro Gly Arg Tyr Asn Leu His Leu Gly Gly Asn Arg Ile Gly Thr
               500                 505                 510
   cgt atc cca cgg atg tat aaa gaa aac atc acc gag ccg gaa atc ctg     1584
   Arg Ile Pro Arg Met Tyr Lys Glu Asn Ile Thr Glu Pro Glu Ile Leu
           515                 520                 525
   gcg tcg ctt gat gaa ctg ata ggg cgc tgg gcg aaa gag cgc gaa gcg     1632
   Ala Ser Leu Asp Glu Leu Ile Gly Arg Trp Ala Lys Glu Arg Glu Ala
       530                 535                 540
   ggt gaa ggc ttc ggc gac ttt acg gtg cgt gcg ggc atc att cgc ccg     1680
   Gly Glu Gly Phe Gly Asp Phe Thr Val Arg Ala Gly Ile Ile Arg Pro
   545                 550                 555                 560
   gtg ctc gat ccg gcg cgt gat ttg tgg gat taa                    1713
   Val Leu Asp Pro Ala Arg Asp Leu Trp Asp
                   565                 570
<210> 66
   <211> 570
   <212> PRT
   <213> Escherichia coli
<400> 66
   Met Ser Glu Lys His Pro Gly Pro Leu Val Val Glu Gly Lys Leu Thr
   1               5                   10                  15
   Asp Ala Glu Arg Met Lys His Glu Ser Asn Tyr Leu Arg Gly Thr Ile
               20                  25                  30
   Ala Glu Asp Leu Asn Asp Gly Leu Thr Gly Gly Phe Lys Gly Asp Asn
           35                  40                  45
   Phe Leu Leu Ile Arg Phe His Gly Met Tyr Gln Gln Asp Asp Arg Asp
       50                  55                  60
   Ile Arg Ala Glu Arg Ala Glu Gln Lys Leu Glu Pro Arg His Ala Met
   65                  70                  75                  80
   Leu Leu Arg Cys Arg Leu Pro Gly Gly Val Ile Thr Thr Lys Gln Trp
                   85                  90                  95
   Gln Ala Ile Asp Lys Phe Ala Gly Glu Asn Thr Ile Tyr Gly Ser Ile
               100                 105                 110
   Arg Leu Thr Asn Arg Gln Thr Phe Gln Phe His Gly Ile Leu Lys Lys
           115                 120                 125
   Asn Val Lys Pro Val His Gln Met Leu His Ser Val Gly Leu Asp Ala
       130                 135                 140
   Leu Ala Thr Ala Asn Asp Met Asn Arg Asn Val Leu Cys Thr Ser Asn
   145                 150                 155                 160
   Pro Tyr Glu Ser Gln Leu His Ala Glu Ala Tyr Glu Trp Ala Lys Lys
                   165                 170                 175
   Ile Ser Glu His Leu Leu Pro Arg Thr Arg Ala Tyr Ala Glu Ile Trp
               180                 185                 190
   Leu Asp Gln Glu Lys Val Ala Thr Thr Asp Glu Glu Pro Ile Leu Gly
           195                 200                 205
   Gln Thr Tyr Leu Pro Arg Lys Phe Lys Thr Thr Val Val Ile Pro Pro
       210                 215                 220
   Gln Asn Asp Ile Asp Leu His Ala Asn Asp Met Asn Phe Val Ala Ile
   225                 230                 235                 240
   Ala Glu Asn Gly Lys Leu Val Gly Phe Asn Leu Leu Val Gly Gly Gly
                   245                 250                 255
   Leu Ser Ile Glu His Gly Asn Lys Lys Thr Tyr Ala Arg Thr Ala Ser
               260                 265                 270
   Glu Phe Gly Tyr Leu Pro Leu Glu His Thr Leu Ala Val Ala Glu Ala
           275                 280                 285
   Val Val Thr Thr Gln Arg Asp Trp Gly Asn Arg Thr Asp Arg Lys Asn
       290                 295                 300
   Ala Lys Thr Lys Tyr Thr Leu Glu Arg Val Gly Val Glu Thr Phe Lys
   305                 310                 315                 320
   Ala Glu Val Glu Arg Arg Ala Gly Ile Lys Phe Glu Pro Ile Arg Pro
                   325                 330                 335
   Tyr Glu Phe Thr Gly Arg Gly Asp Arg Ile Gly Trp Val Lys Gly Ile
               340                 345                 350
   Asp Asp Asn Trp His Leu Thr Leu Phe Ile Glu Asn Gly Arg Ile Leu
           355                 360                 365
   Asp Tyr Pro Ala Arg Pro Leu Lys Thr Gly Leu Leu Glu Ile Ala Lys
       370                 375                 380
   Ile His Lys Gly Asp Phe Arg Ile Thr Ala Asn Gln Asn Leu Ile Ile
   385                 390                 395                 400
   Ala Gly Val Pro Glu Ser Glu Lys Ala Lys Ile Glu Lys Ile Ala Lys
                   405                 410                 415
   Glu Ser Gly Leu Met Asn Ala Val Thr Pro Gln Arg Glu Asn Ser Met
               420                 425                 430
   Ala Cys Val Ser Phe Pro Thr Cys Pro Leu Ala Met Ala Glu Ala Glu
           435                 440                 445
   Arg Phe Leu Pro Ser Phe Ile Asp Asn Ile Asp Asn Leu Met Ala Lys
       450                 455                 460
   His Gly Val Ser Asp Glu His Ile Val Met Arg Val Thr Gly Cys Pro
   465                 470                 475                 480
   Asn Gly Cys Gly Arg Ala Met Leu Ala Glu Val Gly Leu Val Gly Lys
                   485                 490                 495
   Ala Pro Gly Arg Tyr Asn Leu His Leu Gly Gly Asn Arg Ile Gly Thr
               500                 505                 510
   Arg Ile Pro Arg Met Tyr Lys Glu Asn Ile Thr Glu Pro Glu Ile Leu
           515                 520                 525
   Ala Ser Leu Asp Glu Leu Ile Gly Arg Trp Ala Lys Glu Arg Glu Ala
       530                 535                 540
   Gly Glu Gly Phe Gly Asp Phe Thr Val Arg Ala Gly Ile Ile Arg Pro
   545                 550                 555                 560
   Val Leu Asp Pro Ala Arg Asp Leu Trp Asp
                   565                 570
<210> 67
   <211> 1722
   <212> DNA
   <213> Pantoea ananatis
<220>
   <221> CDS
   <222> (1)..(1722)
<400> 67
   atg act gaa aaa tac cct ggc ccg ctg gtg gtt gaa ggc aaa ctc acc      48
   Met Thr Glu Lys Tyr Pro Gly Pro Leu Val Val Glu Gly Lys Leu Thr
   1               5                   10                  15
   gac gct gag cgt tta aaa aaa gag agc aac tac ctg cgc ggt acc atc      96
   Asp Ala Glu Arg Leu Lys Lys Glu Ser Asn Tyr Leu Arg Gly Thr Ile
               20                  25                  30
   ctt gag gac ctc gac gag ggc ctg acc ggt ggt ttc agc ggc gat aac     144
   Leu Glu Asp Leu Asp Glu Gly Leu Thr Gly Gly Phe Ser Gly Asp Asn
           35                  40                  45
   ttc ctg ctg atc cgt ttt cac ggc atg tac cag cag gat gac cgc gat     192
   Phe Leu Leu Ile Arg Phe His Gly Met Tyr Gln Gln Asp Asp Arg Asp
       50                  55                  60
   att cgt gct gaa cgt gcg gag caa aaa ctt gag cct cgc cat gcg atg     240
   Ile Arg Ala Glu Arg Ala Glu Gln Lys Leu Glu Pro Arg His Ala Met
   65                  70                  75                  80
   atg ctg cgc tgt cgt ctg ccg ggc ggg atc att acg ccg acc cag tgg     288
   Met Leu Arg Cys Arg Leu Pro Gly Gly Ile Ile Thr Pro Thr Gln Trp
                   85                  90                  95
   ctg gcg att gat aaa ttt gct acc gat aaa acc ctt tac ggc agc atc     336
   Leu Ala Ile Asp Lys Phe Ala Thr Asp Lys Thr Leu Tyr Gly Ser Ile
               100                 105                 110
   cgt ttg acc aac cgt cag act ttc cag ttt cac ggc att ctg aag aag     384
   Arg Leu Thr Asn Arg Gln Thr Phe Gln Phe His Gly Ile Leu Lys Lys
           115                 120                 125
   aac gtg aag cct acg cac cag atg ctg cac gaa gtg ggg ctg gat gcg     432
   Asn Val Lys Pro Thr His Gln Met Leu His Glu Val Gly Leu Asp Ala
       130                 135                 140
   ctg gcg acc gcg aat gac gtt aac cgt aac gtc ctc tgc acc tca aac     480
   Leu Ala Thr Ala Asn Asp Val Asn Arg Asn Val Leu Cys Thr Ser Asn
   145                 150                 155                 160
   cca gtg gag tct gag ttg cat cag gaa gcc tat gag tgg gcc aaa aag     528
   Pro Val Glu Ser Glu Leu His Gln Glu Ala Tyr Glu Trp Ala Lys Lys
                   165                 170                 175
   ctg tcg gag cat ctg ctg ccg caa acc cgc gct tat gcc gag atc tgg       576
   Leu Ser Glu His Leu Leu Pro Gln Thr Arg Ala Tyr Ala Glu Ile Trp
               180                 185                 190
   tgg gat cag gaa aaa gtg gcg acc acc gac gaa gag ccg atc ctg ggc       624
   Trp Asp Gln Glu Lys Val Ala Thr Thr Asp Glu Glu Pro Ile Leu Gly
           195                 200                 205
   cag acc tac ctg ccg cgt aag ttt aaa acc acg gtg gtg att ccg ccg       672
   Gln Thr Tyr Leu Pro Arg Lys Phe Lys Thr Thr Val Val Ile Pro Pro
       210                 215                 220
   cat aac gat gtt gat ctg cac gct aac gat ctg aac ttt atc gcg att       720
   His Asn Asp Val Asp Leu His Ala Asn Asp Leu Asn Phe Ile Ala Ile
   225                 230                 235                 240
   gcg gaa aac ggc aag ctg gtg ggc ttt aac ctg ctg gtg ggc ggc ggc       768
   Ala Glu Asn Gly Lys Leu Val Gly Phe Asn Leu Leu Val Gly Gly Gly
                   245                 250                 255
   ttg tct atc gag cac ggt aat aaa gcc acc tat gcg cgc acc gcc agc       816
   Leu Ser Ile Glu His Gly Asn Lys Ala Thr Tyr Ala Arg Thr Ala Ser
               260                 265                 270
   gaa ttt ggc tac ttc ccg gtc gat aag atc ctg gat gtg gca gcg gcg       864
   Glu Phe Gly Tyr Phe Pro Val Asp Lys Ile Leu Asp Val Ala Ala Ala
           275                 280                 285
   gta gtg acg acg cag cgc gac tgg ggc aac cgt acc gat cgt aaa aac       912
   Val Val Thr Thr Gln Arg Asp Trp Gly Asn Arg Thr Asp Arg Lys Asn
       290                 295                 300
   gcc aaa acc aaa tac acg ctg gaa cgc gta ggc gta gag acg ttt aag       960
   Ala Lys Thr Lys Tyr Thr Leu Glu Arg Val Gly Val Glu Thr Phe Lys
   305                 310                 315                 320
   gcg gaa gtc gag aag cgc gcc ggt gtc acg ttt gca ccg aca cgt cct      1008
   Ala Glu Val Glu Lys Arg Ala Gly Val Thr Phe Ala Pro Thr Arg Pro
                   325                 330                 335
   tac gaa ttc acc acc cgt ggc gat cgc att ggc tgg atc aag ggc att      1056
   Tyr Glu Phe Thr Thr Arg Gly Asp Arg Ile Gly Trp Ile Lys Gly Ile
               340                 345                 350
   gat aac aag tgg cat ctg act ctg ttc atc gaa aac ggt cgt ctg ctg      1104
   Asp Asn Lys Trp His Leu Thr Leu Phe Ile Glu Asn Gly Arg Leu Leu
           355                 360                 365
   gat tat ccg gga cgt ccg ctg aaa agc ggc gtg gcg gaa atc gcc aaa      1152
   Asp Tyr Pro Gly Arg Pro Leu Lys Ser Gly Val Ala Glu Ile Ala Lys
       370                 375                 380
   atc cat cag ggc gat ttc cgt ctg acc gcc aac cag aac ctg att gtg      1200
   Ile His Gln Gly Asp Phe Arg Leu Thr Ala Asn Gln Asn Leu Ile Val
   385                 390                 395                 400
   gca ggc gtg ccg gaa agc caa aaa gcg cag att gaa gcg att gcc cgc      1248
   Ala Gly Val Pro Glu Ser Gln Lys Ala Gln Ile Glu Ala Ile Ala Arg
                   405                 410                 415
   gaa cac ggt ctg atg gaa aac gtg acg gcc cag cgg gaa aac tcg atg      1296
   Glu His Gly Leu Met Glu Asn Val Thr Ala Gln Arg Glu Asn Ser Met
               420                 425                 430
   gcc tgc gtg gcg ttt cca acc tgt ccg ctg gcg atg gcg gag gct gaa      1344
   Ala Cys Val Ala Phe Pro Thr Cys Pro Leu Ala Met Ala Glu Ala Glu
           435                 440                 445
   cgt ttt ctg ccg tct ttc gtg acg aaa gtc gaa gag atc atg cac agc      1392
   Arg Phe Leu Pro Ser Phe Val Thr Lys Val Glu Glu Ile Met His Ser
       450                 455                 460
   cat ggc gta ggc gaa gag cac ata gtg tta cgc gtt acc ggt tgt ccg      1440
   His Gly Val Gly Glu Glu His Ile Val Leu Arg Val Thr Gly Cys Pro
   465                 470                 475                 480
   aac ggc tgt ggc cgt gcc atg ctg gca gaa gtg ggc ctg gtg ggg aaa      1488
   Asn Gly Cys Gly Arg Ala Met Leu Ala Glu Val Gly Leu Val Gly Lys
                   485                 490                 495
   gcg ccg ggc cgt tac aac ctg cat atc ggg ggt aac cgc atc gga acg      1536
   Ala Pro Gly Arg Tyr Asn Leu His Ile Gly Gly Asn Arg Ile Gly Thr
               500                 505                 510
   cgc att cca cgt atg tat cgc gaa aat att act gaa agt gaa att ctc      1584
   Arg Ile Pro Arg Met Tyr Arg Glu Asn Ile Thr Glu Ser Glu Ile Leu
           515                 520                 525
   gcc aca ctt gat gag ctg gta ggt cgc tgg gcg aaa gag cgc gag gcc      1632
   Ala Thr Leu Asp Glu Leu Val Gly Arg Trp Ala Lys Glu Arg Glu Ala
       530                 535                 540
   gca gaa ggc ttt ggt gac ttc gtg att cgg gct ggc att gtt acg ccg      1680
   Ala Glu Gly Phe Gly Asp Phe Val Ile Arg Ala Gly Ile Val Thr Pro
   545                 550                 555                 560
   gtg ttg gat cct gcg cgc gac ttc tgg cag gag gca aaa tga              1722
   Val Leu Asp Pro Ala Arg Asp Phe Trp Gln Glu Ala Lys
                   565                 570
<210> 68
   <211> 573
   <212> PRT
   <213> Pantoea ananatis
<400> 68
   Met Thr Glu Lys Tyr Pro Gly Pro Leu Val Val Glu Gly Lys Leu Thr
   1               5                   10                  15
   Asp Ala Glu Arg Leu Lys Lys Glu Ser Asn Tyr Leu Arg Gly Thr Ile
               20                  25                  30
   Leu Glu Asp Leu Asp Glu Gly Leu Thr Gly Gly Phe Ser Gly Asp Asn
           35                  40                  45
   Phe Leu Leu Ile Arg Phe His Gly Met Tyr Gln Gln Asp Asp Arg Asp
       50                  55                  60
   Ile Arg Ala Glu Arg Ala Glu Gln Lys Leu Glu Pro Arg His Ala Met
   65                  70                  75                  80
   Met Leu Arg Cys Arg Leu Pro Gly Gly Ile Ile Thr Pro Thr Gln Trp
                   85                  90                  95
   Leu Ala Ile Asp Lys Phe Ala Thr Asp Lys Thr Leu Tyr Gly Ser Ile
               100                 105                 110
   Arg Leu Thr Asn Arg Gln Thr Phe Gln Phe His Gly Ile Leu Lys Lys
           115                 120                 125
   Asn Val Lys Pro Thr His Gln Met Leu His Glu Val Gly Leu Asp Ala
       130                 135                 140
   Leu Ala Thr Ala Asn Asp Val Asn Arg Asn Val Leu Cys Thr Ser Asn
   145                 150                 155                 160
   Pro Val Glu Ser Glu Leu His Gln Glu Ala Tyr Glu Trp Ala Lys Lys
                   165                 170                 175
   Leu Ser Glu His Leu Leu Pro Gln Thr Arg Ala Tyr Ala Glu Ile Trp
               180                 185                 190
   Trp Asp Gln Glu Lys Val Ala Thr Thr Asp Glu Glu Pro Ile Leu Gly
           195                 200                 205
   Gln Thr Tyr Leu Pro Arg Lys Phe Lys Thr Thr Val Val Ile Pro Pro
       210                 215                 220
   His Asn Asp Val Asp Leu His Ala Asn Asp Leu Asn Phe Ile Ala Ile
   225                 230                 235                 240
   Ala Glu Asn Gly Lys Leu Val Gly Phe Asn Leu Leu Val Gly Gly Gly
                   245                 250                 255
   Leu Ser Ile Glu His Gly Asn Lys Ala Thr Tyr Ala Arg Thr Ala Ser
               260                 265                 270
   Glu Phe Gly Tyr Phe Pro Val Asp Lys Ile Leu Asp Val Ala Ala Ala
           275                 280                 285
   Val Val Thr Thr Gln Arg Asp Trp Gly Asn Arg Thr Asp Arg Lys Asn
       290                 295                 300
   Ala Lys Thr Lys Tyr Thr Leu Glu Arg Val Gly Val Glu Thr Phe Lys
   305                 310                 315                 320
   Ala Glu Val Glu Lys Arg Ala Gly Val Thr Phe Ala Pro Thr Arg Pro
                   325                 330                 335
   Tyr Glu Phe Thr Thr Arg Gly Asp Arg Ile Gly Trp Ile Lys Gly Ile
               340                 345                 350
   Asp Asn Lys Trp His Leu Thr Leu Phe Ile Glu Asn Gly Arg Leu Leu
           355                 360                 365
   Asp Tyr Pro Gly Arg Pro Leu Lys Ser Gly Val Ala Glu Ile Ala Lys
       370                 375                 380
   Ile His Gln Gly Asp Phe Arg Leu Thr Ala Asn Gln Asn Leu Ile Val
   385                 390                 395                 400
   Ala Gly Val Pro Glu Ser Gln Lys Ala Gln Ile Glu Ala Ile Ala Arg
                   405                 410                 415
   Glu His Gly Leu Met Glu Asn Val Thr Ala Gln Arg Glu Asn Ser Met
               420                 425                 430
   Ala Cys Val Ala Phe Pro Thr Cys Pro Leu Ala Met Ala Glu Ala Glu
           435                 440                 445
   Arg Phe Leu Pro Ser Phe Val Thr Lys Val Glu Glu Ile Met His Ser
       450                 455                 460
   His Gly Val Gly Glu Glu His Ile Val Leu Arg Val Thr Gly Cys Pro
   465                 470                 475                 480
   Asn Gly Cys Gly Arg Ala Met Leu Ala Glu Val Gly Leu Val Gly Lys
                   485                 490                 495
   Ala Pro Gly Arg Tyr Asn Leu His Ile Gly Gly Asn Arg Ile Gly Thr
               500                 505                 510
   Arg Ile Pro Arg Met Tyr Arg Glu Asn Ile Thr Glu Ser Glu Ile Leu
           515                 520                 525
   Ala Thr Leu Asp Glu Leu Val Gly Arg Trp Ala Lys Glu Arg Glu Ala
       530                 535                 540
   Ala Glu Gly Phe Gly Asp Phe Val Ile Arg Ala Gly Ile Val Thr Pro
   545                 550                 555                 560
   Val Leu Asp Pro Ala Arg Asp Phe Trp Gln Glu Ala Lys
                   565                 570
<210> 69
   <211> 1374
   <212> DNA
   <213> Escherichia coli
<220>
   <221> CDS
   <222> (1)..(1374)
<400> 69
   gtg gat cat ttg cct ata ttt tgc caa tta cgc gat cgc gac tgt ctg        48
   Met Asp His Leu Pro Ile Phe Cys Gln Leu Arg Asp Arg Asp Cys Leu
   1               5                   10                  15
   att gtc ggc ggt ggt gat gtc gcg gaa cgc aaa gca agg ttg ctg tta        96
   Ile Val Gly Gly Gly Asp Val Ala Glu Arg Lys Ala Arg Leu Leu Leu
               20                  25                  30
   gac gca ggc gct cgc tta acg gtg aat gca tta gcg ttt att cca cag       144
   Asp Ala Gly Ala Arg Leu Thr Val Asn Ala Leu Ala Phe Ile Pro Gln
           35                  40                  45
   ttc acc gca tgg gca gat gca ggc atg tta acc ctc gtc gaa ggg cca       192
   Phe Thr Ala Trp Ala Asp Ala Gly Met Leu Thr Leu Val Glu Gly Pro
       50                  55                  60
   ttt gat gaa agc ctt ctc gac acc tgc tgg ctg gcg att gca gcg acg       240
   Phe Asp Glu Ser Leu Leu Asp Thr Cys Trp Leu Ala Ile Ala Ala Thr
   65                  70                  75                  80
   gat gat gac gcg ctt aac cag cgc gtc agc gaa gcc gct gaa gct cgt       288
   Asp Asp Asp Ala Leu Asn Gln Arg Val Ser Glu Ala Ala Glu Ala Arg
                   85                  90                  95
   cgc atc ttc tgt aac gtg gtc gat gcg ccg aaa gcc gcc agc ttt att       336
   Arg Ile Phe Cys Asn Val Val Asp Ala Pro Lys Ala Ala Ser Phe Ile
               100                 105                 110
   atg ccg tcg att att gac cgc tca ccg ctc atg gta gcg gtc tcc tct       384
   Met Pro Ser Ile Ile Asp Arg Ser Pro Leu Met Val Ala Val Ser Ser
           115                 120                 125
   ggc ggc acc tct ccg gtt ctg gca cgc ctg ttg cgc gaa aaa ctt gaa       432
   Gly Gly Thr Ser Pro Val Leu Ala Arg Leu Leu Arg Glu Lys Leu Glu
       130                 135                 140
   tca ctg ctg ccg tta cat ctg ggc cag gta gcg aaa tac gcc ggg caa       480
   Ser Leu Leu Pro Leu His Leu Gly Gln Val Ala Lys Tyr Ala Gly Gln
   145                 150                 155                 160
   tta cgc ggg cga gtg aaa caa cag ttc gcc acg atg ggt gag cgt cgc       528
   Leu Arg Gly Arg Val Lys Gln Gln Phe Ala Thr Met Gly Glu Arg Arg
                   165                 170                 175
   cgt ttc tgg gag aaa ttg ttc gtt aac gac cgc ctg gcg cag tcg ctg       576
   Arg Phe Trp Glu Lys Leu Phe Val Asn Asp Arg Leu Ala Gln Ser Leu
               180                 185                 190
   gca aac aac gat cag aaa gcc att act gaa acg acc gaa cag tta atc       624
   Ala Asn Asn Asp Gln Lys Ala Ile Thr Glu Thr Thr Glu Gln Leu Ile
           195                 200                 205
   aac gaa ccg ctc gac cat cgc ggt gaa gtg gtg ctg gtt ggt gca ggt       672
   Asn Glu Pro Leu Asp His Arg Gly Glu Val Val Leu Val Gly Ala Gly
       210                 215                 220
   ccg ggc gat gcc ggg ctg ctg aca ctg aaa gga ctg caa caa att cag       720
   Pro Gly Asp Ala Gly Leu Leu Thr Leu Lys Gly Leu Gln Gln Ile Gln
   225                 230                 235                 240
   cag gca gat gtg gtg gtc tac gac cgt ctg gtt tct gac gat att atg       768
   Gln Ala Asp Val Val Val Tyr Asp Arg Leu Val Ser Asp Asp Ile Met
                   245                 250                 255
   aat ctg gta cgc cgc gat gcg gac cgt gtt ttc gtc ggc aaa cgc gcg       816
   Asn Leu Val Arg Arg Asp Ala Asp Arg Val Phe Val Gly Lys Arg Ala
               260                 265                 270
   gga tac cac tgc gta ccc cag gaa gag att aac cag atc ctg ctg cgg       864
   Gly Tyr His Cys Val Pro Gln Glu Glu Ile Asn Gln Ile Leu Leu Arg
           275                 280                 285
   gaa gcg caa aaa ggc aaa cgc gtg gtg cgg ctg aaa ggt ggc gat ccg       912
   Glu Ala Gln Lys Gly Lys Arg Val Val Arg Leu Lys Gly Gly Asp Pro
       290                 295                 300
   ttt att ttt ggc cgt ggt ggc gaa gag ctg gaa aca ctg tgc aac gcg       960
   Phe Ile Phe Gly Arg Gly Gly Glu Glu Leu Glu Thr Leu Cys Asn Ala
   305                 310                 315                 320
   ggt att ccg ttc tcg gtg gtt ccg ggt att acc gca gct tct ggt tgc      1008
   Gly Ile Pro Phe Ser Val Val Pro Gly Ile Thr Ala Ala Ser Gly Cys
                   325                 330                 335
   tct gcc tat tcg ggt att cca ctc acg cat cgc gat tat gcc cag agc      1056
   Ser Ala Tyr Ser Gly Ile Pro Leu Thr His Arg Asp Tyr Ala Gln Ser
               340                 345                 350
   gta cgc tta att acc gga cac tta aaa acc ggt ggc gag ctg gac tgg      1104
   Val Arg Leu Ile Thr Gly His Leu Lys Thr Gly Gly Glu Leu Asp Trp
           355                 360                 365
   gaa aac ctg gcg gca gaa aaa cag acg ctg gtg ttc tat atg ggg ttg      1152
   Glu Asn Leu Ala Ala Glu Lys Gln Thr Leu Val Phe Tyr Met Gly Leu
       370                 375                 380
   aat cag gcc gcg act att cag caa aag ctg att gaa cac gga atg cca      1200
   Asn Gln Ala Ala Thr Ile Gln Gln Lys Leu Ile Glu His Gly Met Pro
   385                 390                 395                 400
   ggc gaa atg ccg gtg gca att gtc gaa aac ggt acg gca gtc acg cag      1248
   Gly Glu Met Pro Val Ala Ile Val Glu Asn Gly Thr Ala Val Thr Gln
                   405                 410                 415
   cgc gtg att gac ggt acg ctc aca cag ctg gga gaa ctg gcg cag caa      1296
   Arg Val Ile Asp Gly Thr Leu Thr Gln Leu Gly Glu Leu Ala Gln Gln
               420                 425                 430
   atg aac agt cca tcg cta att att att ggt cgg gtt gtt ggc ctg cgc      1344
   Met Asn Ser Pro Ser Leu Ile Ile Ile Gly Arg Val Val Gly Leu Arg
           435                     440                     445
   gat aaa ctg aac tgg ttc tcc aac cat taa                              1374
   Asp Lys Leu Asn Trp Phe Ser Asn His
       450                 455
<210> 70
   <211> 457
   <212> PRT
   <213> Escherichia coli
<400> 70
   Met Asp His Leu Pro Ile Phe Cys Gln Leu Arg Asp Arg Asp Cys Leu
   1               5                   10                  15
   Ile Val Gly Gly Gly Asp Val Ala Glu Arg Lys Ala Arg Leu Leu Leu
               20                  25                  30
   Asp Ala Gly Ala Arg Leu Thr Val Asn Ala Leu Ala Phe Ile Pro Gln
           35                  40                  45
   Phe Thr Ala Trp Ala Asp Ala Gly Met Leu Thr Leu Val Glu Gly Pro
       50                  55                  60
   Phe Asp Glu Ser Leu Leu Asp Thr Cys Trp Leu Ala Ile Ala Ala Thr
   65                  70                  75                  80
   Asp Asp Asp Ala Leu Asn Gln Arg Val Ser Glu Ala Ala Glu Ala Arg
                   85                  90                  95
   Arg Ile Phe Cys Asn Val Val Asp Ala Pro Lys Ala Ala Ser Phe Ile
               100                 105                 110
   Met Pro Ser Ile Ile Asp Arg Ser Pro Leu Met Val Ala Val Ser Ser
           115                 120                 125
   Gly Gly Thr Ser Pro Val Leu Ala Arg Leu Leu Arg Glu Lys Leu Glu
       130                 135                 140
   Ser Leu Leu Pro Leu His Leu Gly Gln Val Ala Lys Tyr Ala Gly Gln
   145                 150                 155                 160
   Leu Arg Gly Arg Val Lys Gln Gln Phe Ala Thr Met Gly Glu Arg Arg
                   165                 170                 175
   Arg Phe Trp Glu Lys Leu Phe Val Asn Asp Arg Leu Ala Gln Ser Leu
               180                 185                 190
   Ala Asn Asn Asp Gln Lys Ala Ile Thr Glu Thr Thr Glu Gln Leu Ile
           195                 200                 205
   Asn Glu Pro Leu Asp His Arg Gly Glu Val Val Leu Val Gly Ala Gly
       210                 215                 220
   Pro Gly Asp Ala Gly Leu Leu Thr Leu Lys Gly Leu Gln Gln Ile Gln
   225                 230                 235                 240
   Gln Ala Asp Val Val Val Tyr Asp Arg Leu Val Ser Asp Asp Ile Met
                   245                 250                 255
   Asn Leu Val Arg Arg Asp Ala Asp Arg Val Phe Val Gly Lys Arg Ala
               260                 265                 270
   Gly Tyr His Cys Val Pro Gln Glu Glu Ile Asn Gln Ile Leu Leu Arg
           275                 280                 285
   Glu Ala Gln Lys Gly Lys Arg Val Val Arg Leu Lys Gly Gly Asp Pro
       290                 295                 300
   Phe Ile Phe Gly Arg Gly Gly Glu Glu Leu Glu Thr Leu Cys Asn Ala
   305                 310                 315                 320
   Gly Ile Pro Phe Ser Val Val Pro Gly Ile Thr Ala Ala Ser Gly Cys
                   325                 330                 335
   Ser Ala Tyr Ser Gly Ile Pro Leu Thr His Arg Asp Tyr Ala Gln Ser
               340                 345                 350
   Val Arg Leu Ile Thr Gly His Leu Lys Thr Gly Gly Glu Leu Asp Trp
           355                 360                 365
   Glu Asn Leu Ala Ala Glu Lys Gln Thr Leu Val Phe Tyr Met Gly Leu
       370                 375                 380
   Asn Gln Ala Ala Thr Ile Gln Gln Lys Leu Ile Glu His Gly Met Pro
   385                 390                 395                 400
   Gly Glu Met Pro Val Ala Ile Val Glu Asn Gly Thr Ala Val Thr Gln
                   405                 410                 415
   Arg Val Ile Asp Gly Thr Leu Thr Gln Leu Gly Glu Leu Ala Gln Gln
               420                 425                 430
   Met Asn Ser Pro Ser Leu Ile Ile Ile Gly Arg Val Val Gly Leu Arg
           435                 440                 445
   Asp Lys Leu Asn Trp Phe Ser Asn His
       450                 455
<210> 71
   <211> 1416
   <212> DNA
   <213> Pantoea ananatis
<220>
   <221> CDS
   <222> (1)..(1416)
<400> 71
   gtg gat tat ttg cct ctt ttt gcc gat ctc gca ggt cga ccc gta ctg      48
   Met Asp Tyr Leu Pro Leu Phe Ala Asp Leu Ala Gly Arg Pro Val Leu
   1               5                   10                  15
   gtc gtc ggc ggc gga gat atc gcg gcg cgc aag att gag ctg ctg cgt      96
   Val Val Gly Gly Gly Asp Ile Ala Ala Arg Lys Ile Glu Leu Leu Arg
               20                  25                  30
   cgg gcc ggg gcg cgc att caa atc gcc tca cgc gaa ctc tgc ccc gag      144
   Arg Ala Gly Ala Arg Ile Gln Ile Ala Ser Arg Glu Leu Cys Pro Glu
           35                  40                  45
   tta cag gct ttg ctg gat gaa cag cag ctt gaa tgg ctg gcc acg tcc      192
   Leu Gln Ala Leu Leu Asp Glu Gln Gln Leu Glu Trp Leu Ala Thr Ser
       50                  55                  60
   ttt gaa ccc gct cag ctc gac aag gtc ttt ctg gtc att gcc gct acc      240
   Phe Glu Pro Ala Gln Leu Asp Lys Val Phe Leu Val Ile Ala Ala Thr
   65                  70                  75                  80
   gat gac aat gcg ctg aat gcg cag gtc tat gac gaa gcg aat gcc cgc      288
   Asp Asp Asn Ala Leu Asn Ala Gln Val Tyr Asp Glu Ala Asn Ala Arg
                   85                  90                  95
   cac aag ctg gta aac gtg gta gac gat cag cct aaa tgc agc ttt att      336
   His Lys Leu Val Asn Val Val Asp Asp Gln Pro Lys Cys Ser Phe Ile
               100                 105                 110
   ttc ccc tct att gtt gat cga tcg cca ctg gtc gtg gcg atc tcc tcc      384
   Phe Pro Ser Ile Val Asp Arg Ser Pro Leu Val Val Ala Ile Ser Ser
           115                 120                 125
   agt ggc acc gcg ccg gtg ctg gcc cgc atg ctg cgc gag aaa ctc gaa      432
   Ser Gly Thr Ala Pro Val Leu Ala Arg Met Leu Arg Glu Lys Leu Glu
       130                 135                 140
   acg ctg ctg cca tcc cat ctg ggc caa atg gcc gag ctg gcg ggt cag      480
   Thr Leu Leu Pro Ser His Leu Gly Gln Met Ala Glu Leu Ala Gly Gln
   145                 150                 155                 160
   tgg cgt gac aaa gtc aaa gct cgc ttc agc cgt atg tcc gat cgc cgt      528
   Trp Arg Asp Lys Val Lys Ala Arg Phe Ser Arg Met Ser Asp Arg Arg
                   165                 170                 175
   cgt tac tgg gaa aga ata ttt aat ggc cgt ttt gcc agt cag atg gcg      576
   Arg Tyr Trp Glu Arg Ile Phe Asn Gly Arg Phe Ala Ser Gln Met Ala
               180                 185                 190
   acg ggc gac gtt acg gcc gct aaa cag acg ctg gat agc gaa ctg ggc      624
   Thr Gly Asp Val Thr Ala Ala Lys Gln Thr Leu Asp Ser Glu Leu Gly
           195                 200                 205
   gat cag ccg ccc cga cag ggc gaa att att ctg gtt ggc gcg ggg cct      672
   Asp Gln Pro Pro Arg Gln Gly Glu Ile Ile Leu Val Gly Ala Gly Pro
       210                 215                 220
   ggc gac agc ggc ctg tta acc ctg cgc gga ctg cag gtg atg cag ctg      720
   Gly Asp Ser Gly Leu Leu Thr Leu Arg Gly Leu Gln Val Met Gln Leu
   225                 230                 235                 240
   gcg gac gtg gtg ctc tac gat cat ctc gtc agc gat gag gtg ctc gat      768
   Ala Asp Val Val Leu Tyr Asp His Leu Val Ser Asp Glu Val Leu Asp
                   245                 250                 255
   ctg gtc cgg cgc gat gcc gat cgt atc tgc gta ggc aag cgt gcc agc      816
   Leu Val Arg Arg Asp Ala Asp Arg Ile Cys Val Gly Lys Arg Ala Ser
               260                 265                 270
   gcc cat ctc ctg ccg cag gac gaa att aac cag ttg atg gtg caa ctg      864
   Ala His Leu Leu Pro Gln Asp Glu Ile Asn Gln Leu Met Val Gln Leu
           275                 280                 285
   gcg cag aaa ggc aaa cgt gtg gtg cgc ctt aaa ggc ggc gat ccc ttt      912
   Ala Gln Lys Gly Lys Arg Val Val Arg Leu Lys Gly Gly Asp Pro Phe
       290                 295                 300
   att ttt ggc cgc ggc ggc gaa gag tta cag gcg gcg gcg caa gcg ggc      960
   Ile Phe Gly Arg Gly Gly Glu Glu Leu Gln Ala Ala Ala Gln Ala Gly
   305                 310                 315                 320
   att cca ttc cag gtc gtg cct ggc gta acg gcc gcc gca ggg gct acc     1008
   Ile Pro Phe Gln Val Val Pro Gly Val Thr Ala Ala Ala Gly Ala Thr
                   325                 330                 335
   gcc tat gct ggc att ccg ctg acg cac cgt gat tac gca caa agc gtg     1056
   Ala Tyr Ala Gly Ile Pro Leu Thr His Arg Asp Tyr Ala Gln Ser Val
               340                 345                 350
   ctg ttt atc acc gga cac tgc cgt ccg gat ggc gat gat att gac tgg     1104
   Leu Phe Ile Thr Gly His Cys Arg Pro Asp Gly Asp Asp Ile Asp Trp
           355                 360                 365
   cca tcc ctc gcg cgt gcc cgt cag acg ctg gcg att tac atg ggc gct     1152
   Pro Ser Leu Ala Arg Ala Arg Gln Thr Leu Ala Ile Tyr Met Gly Ala
       370                 375                 380
   gtc aag gcg gct cac atc agc cag cag ctt att ctt cat ggg cgc gcc     1200
   Val Lys Ala Ala His Ile Ser Gln Gln Leu Ile Leu His Gly Arg Ala
   385                 390                 395                 400
   gcc tca aca ccg gtt gcg gtg att ggg cgc ggt acc cgg ccg gat caa     1248
   Ala Ser Thr Pro Val Ala Val Ile Gly Arg Gly Thr Arg Pro Asp Gln
                   405                 410                 415
   cag gta ttg acc ggc aca ctc gaa cat ctg gag acg ctg gca gcg tca     1296
   Gln Val Leu Thr Gly Thr Leu Glu His Leu Glu Thr Leu Ala Ala Ser
               420                 425                 430
   gcg cct tcc ccg gcc ctg ctg gtg att ggg gaa gtg gtt aat tta cac     1344
   Ala Pro Ser Pro Ala Leu Leu Val Ile Gly Glu Val Val Asn Leu His
           435                 440                 445
   ggg caa ctg gcc tgg ttt cag cat tcg gca cag cag ggg gct cgc gag     1392
   Gly Gln Leu Ala Trp Phe Gln His Ser Ala Gln Gln Gly Ala Arg Glu
       450                 455                 460
   tcc gcc gtt gtc aat ctg gct tga                                     1416
   Ser Ala Val Val Asn Leu Ala
   465                 470
<210> 72
   <211> 471
   <212> PRT
   <213> Pantoea ananatis
<400> 72
   Met Asp Tyr Leu Pro Leu Phe Ala Asp Leu Ala Gly Arg Pro Val Leu
   1               5                   10                  15
   Val Val Gly Gly Gly Asp Ile Ala Ala Arg Lys Ile Glu Leu Leu Arg
               20                  25                  30
   Arg Ala Gly Ala Arg Ile Gln Ile Ala Ser Arg Glu Leu Cys Pro Glu
           35                  40                  45
   Leu Gln Ala Leu Leu Asp Glu Gln Gln Leu Glu Trp Leu Ala Thr Ser
       50                  55                  60
   Phe Glu Pro Ala Gln Leu Asp Lys Val Phe Leu Val Ile Ala Ala Thr
   65                  70                  75                  80
   Asp Asp Asn Ala Leu Asn Ala Gln Val Tyr Asp Glu Ala Asn Ala Arg
                   85                  90                  95
   His Lys Leu Val Asn Val Val Asp Asp Gln Pro Lys Cys Ser Phe Ile
               100                 105                 110
   Phe Pro Ser Ile Val Asp Arg Ser Pro Leu Val Val Ala Ile Ser Ser
           115                 120                 125
   Ser Gly Thr Ala Pro Val Leu Ala Arg Met Leu Arg Glu Lys Leu Glu
       130                 135                 140
   Thr Leu Leu Pro Ser His Leu Gly Gln Met Ala Glu Leu Ala Gly Gln
   145                 150                 155                 160
   Trp Arg Asp Lys Val Lys Ala Arg Phe Ser Arg Met Ser Asp Arg Arg
                   165                 170                 175
   Arg Tyr Trp Glu Arg Ile Phe Asn Gly Arg Phe Ala Ser Gln Met Ala
               180                 185                 190
   Thr Gly Asp Val Thr Ala Ala Lys Gln Thr Leu Asp Ser Glu Leu Gly
           195                 200                 205
   Asp Gln Pro Pro Arg Gln Gly Glu Ile Ile Leu Val Gly Ala Gly Pro
       210                 215                 220
   Gly Asp Ser Gly Leu Leu Thr Leu Arg Gly Leu Gln Val Met Gln Leu
   225                 230                 235                 240
   Ala Asp Val Val Leu Tyr Asp His Leu Val Ser Asp Glu Val Leu Asp
                   245                 250                 255
   Leu Val Arg Arg Asp Ala Asp Arg Ile Cys Val Gly Lys Arg Ala Ser
               260                 265                 270
   Ala His Leu Leu Pro Gln Asp Glu Ile Asn Gln Leu Met Val Gln Leu
           275                 280                 285
   Ala Gln Lys Gly Lys Arg Val Val Arg Leu Lys Gly Gly Asp Pro Phe
       290                 295                 300
   Ile Phe Gly Arg Gly Gly Glu Glu Leu Gln Ala Ala Ala Gln Ala Gly
   305                 310                 315                 320
   Ile Pro Phe Gln Val Val Pro Gly Val Thr Ala Ala Ala Gly Ala Thr
                   325                 330                 335
   Ala Tyr Ala Gly Ile Pro Leu Thr His Arg Asp Tyr Ala Gln Ser Val
               340                 345                 350
   Leu Phe Ile Thr Gly His Cys Arg Pro Asp Gly Asp Asp Ile Asp Trp
           355                 360                 365
   Pro Ser Leu Ala Arg Ala Arg Gln Thr Leu Ala Ile Tyr Met Gly Ala
       370                 375                 380
   Val Lys Ala Ala His Ile Ser Gln Gln Leu Ile Leu His Gly Arg Ala
   385                 390                 395                 400
   Ala Ser Thr Pro Val Ala Val Ile Gly Arg Gly Thr Arg Pro Asp Gln
                   405                 410                 415
   Gln Val Leu Thr Gly Thr Leu Glu His Leu Glu Thr Leu Ala Ala Ser
               420                 425                 430
   Ala Pro Ser Pro Ala Leu Leu Val Ile Gly Glu Val Val Asn Leu His
           435                 440                 445
   Gly Gln Leu Ala Trp Phe Gln His Ser Ala Gln Gln Gly Ala Arg Glu
       450                 455                 460
   Ser Ala Val Val Asn Leu Ala
   465                 470
<210> 73
   <211> 146
   <212> DNA
   <213> Escherichia coli
<400> 73
   aaaacgtgag gaaatacctg gatttttcct ggttattttg ccgcaggtca gcgtatcgtg     60
   aacatctttt ccagtgttca gtagggtgcc ttgcacggta attatgtcac tggttattaa    120
   ccaatttttc ctggggataa atgagc                                         146
<210> 74
   <211> 146
   <212> DNA
   <213> Escherichia coli
<400> 74
   aaaacgtgag gaaatacctg gatttttcct ggttattttg ccgcaggtca gcgtataatg     60
   aagatctttt ccagtgttga caagggtgcc ttgcacggtt ataatgtcac tggttattaa    120
   ccaatttttc ctggggataa atgagc                                         146

## Description

### Field of the Invention

The present invention relates to a method for producing L-cysteine or a related substance thereof. L-cysteine and related substances thereof are used in the fields of drugs, cosmetics, and foods.

### Brief Description of the Related Art

L-cysteine is conventionally obtained by extraction from keratin-containing substances such as hairs, horns, and feathers, or conversion of DL-2-aminothiazoline-4-carboxylic acid as a precursor using a microbial enzyme. It is also planned to produce L-cysteine in a large scale by an immobilized enzyme method utilizing a novel enzyme. Furthermore, it is also attempted to produce L-cysteine by fermentation utilizing a microorganism.

L-cysteine-producing ability of a microorganism can be improved by enhancing activity of an enzyme of the L-cysteine biosynthesis pathway or activity of an enzyme involved in generation of a compound serving as a substrate of that pathway, such as L-serine. As microorganisms having an ability to produce L-cysteine, there is known, for example, a coryneform bacterium of which intracellular serine acetyltransferase activity is increased (Patent document 1). There is also known a technique of increasing L-cysteine-producing ability by incorporating a mutant serine acetyltransferase of which feedback inhibition by L-cysteine is attenuated (Patent documents 2 to 4). A mutant *serA* gene coding for a mutant 3-phosphoglycerate dehydrogenase of which feedback inhibition by serine is attenuated is also known, and use thereof in production of L-cysteine by *Escherichia coli* has been suggested (Patent documents 5 and 6).

Further, L-cysteine-producing ability of a microorganism can also be improved by suppressing the L-cysteine decomposition system. As microorganisms of which L-cysteine-producing ability is enhanced by suppressing the L-cysteine decomposition system, there are known coryneform bacteria or *Escherichia* bacteria in which activity of cystathionine-β-lyase (Patent document 2), tryptophanase (Patent document 7), or O-acetylserine sulfhydrylase B (Patent document 8) is attenuated or deleted.

Moreover, L-cysteine-producing ability of a microorganism can also be improved by enhancing L-cysteine-secreting ability. For example, there are known techniques of enhancing L-cysteine-producing ability by enhancing expression of the *ydeD* gene (Non-patent document 1), *yfiK* gene (Non-patent document 9), or *yeaS* gene (Non-patent document 10), which codes for a protein that participates in secretion of L-cysteine. Furthermore, there are also known techniques of enhancing L-cysteine-producing ability by enhancing expression of the *mar* locus, *emr* locus, *acr* locus, *cmr* locus, *mex* gene, *bmr* gene or *qacA* gene (Patent document 11), or *emrAB, emrKY, yojIH, acrEF, bcr* or *cusA* gene (Patent document 12), which encode proteins suitable for secreting a substance showing cytotoxicity from cells.

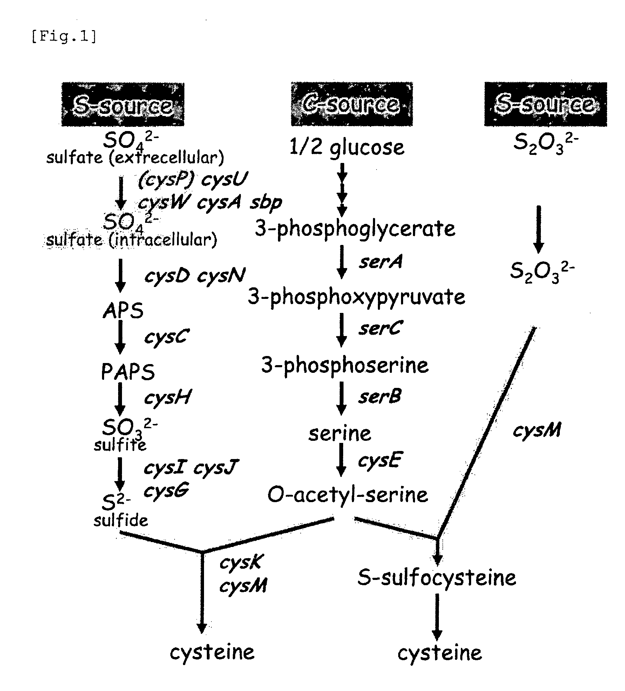

L-cysteine is a sulfur-containing amino acid, and metabolism of a sulfur source is involved in the production of L-cysteine. Outline of the biosynthesis pathway of L-cysteine for the case of using glucose as the carbon source and sulfate ion or thiosulfate ion as the sulfur source, and examples of genes involved in that pathway are shown in Fig. 1.

Sulfite reductase is an enzyme that catalyzes, in the sulfate reduction pathway (Non-patent document 2) in which sulfate ion (SO₄²⁻) is converted into sulfide ion (S²⁻), the reaction of reducing sulfite ion (SO₃²⁻) into sulfide ion (S²⁻) as the final step. The sulfide ion generated from the sulfate ion via the sulfate reduction pathway reacts with O-acetylserine (OAS) to generate L-cysteine. That is, when L-cysteine is produced by using sulfate ions as the sulfur source, the sulfite reductase is considered to be one of the enzymes that participate in the L-cysteine biosynthesis. It is known that the sulfite reductase of *Escherichia coli* has an α₈β₄ complex structure comprising α subunits encoded by the *cysJ* gene and β subunits encoded by the *cysI* gene. Further, the *cysG* gene codes for the biosynthesis enzyme of siroheme, which is a cofactor of the sulfite reductase β subunit. The *cysJ* gene and the *cysI* gene exist in the same operon, whereas the *cysG* gene exists on a different site of the genome. The details of the *cysJ* gene, the *cysI* gene and the proteins encoded by these genes are described in Non-patent documents 3 to 7. Further, the details of the *cysG* gene and the protein encoded by this gene are described in Non-patent documents 8 to 11.

Although there is no finding directly indicating usefulness of enhancing expression of the *cysG* gene, *cysJ* gene or *cysI* gene in L-cysteine production, *Escherichia coli* in which activity of a protein encoded by the *cysB* gene is enhanced is known as an L-cysteine-producing bacterium (Patent document 13). Because the protein encoded by the *cysB* gene positively regulates expression of the *cysJIH* operon containing the *cysJ* gene and *cysI* gene coding for sulfite reductase, expression of the *cysJ* gene and *cysI* gene can be increased in that *Escherichia coli.* On the other hand, expression of the *cysG* gene is not regulated by the protein encoded by the *cysB* gene, and enhancement of expression of the *cysG* gene for L-cysteine production is not known.

Further, effectiveness of enhancement of the *cysG* gene, *cysJ* gene or *cysI* gene expression in the production of amino acids other than L-cysteine has been suggested. For example, enhancement of expression of the *cys* genes including the *cysG* gene, *cysJ* gene and *cysI* gene is referred to concerning production of L-threonine or L-lysine (Patent document 14), production of L-threonine (Patent document 15), and production of L-methionine (Patent documents 16 and 17). Furthermore, enhancement of expression of the *cys* genes including the *cysJ* gene and the *cysI* gene is also referred to concerning production of L-methionine (Patent documents 18 to 21). Moreover, enhancement of expression of the *cysJIH* operon including the *cysJ* gene and the *cysI* gene is also referred to concerning production of L-methionine (Patent document 22). Furthermore, there is known a technique of increasing L-arginine production by enhancing expression of the *cysG* gene (Patent document 23).

On the other hand, thiosulfate is metabolized in a pathway other than the sulfate reduction pathway (Fig. 1), and it is not known that the sulfite reductase participates in the metabolism of thiosulfate. Further, all the aforementioned findings concerning production of amino acids do not suggest that enhancement of expression of the *cysG* gene, *cysJ* gene or *cysI* gene is effective for amino acid production using thiosulfate as the sulfur source. As described above, relation between production of an amino acid such as L-cysteine using thiosulfate as the sulfur source and the *cysG* gene, *cysJ* gene or *cysI* gene has not been known.

### Prior art documents

### Patent documents

Patent document 1: Japanese Patent Laid-open (Kokai) No. 2002-233384
Patent document 2: Japanese Patent Laid-open (Kokai) No. 11-155571
Patent document 3: U.S. Patent Published Application No. 20050112731
Patent document 4: U.S. Patent No. 6,218,168
Patent document 5: U.S. Patent No. 5,856,148
Patent document 6: U.S. Patent Published Application No. 20050009162
Patent document 7: Japanese Patent Laid-open (Kokai) No. 2003-169668
Patent document 8: Japanese Patent Laid-open (Kokai) No. 2005-245311
Patent document 9: Japanese Patent Laid-open (Kokai) No. 2004-49237
Patent document 10: European Patent Laid-open No. 1016710
Patent document 11: U.S. Patent No. 5,972,663
Patent document 12: Japanese Patent Laid-open (Kokai) No. 2005-287333
Patent document 13: International Patent Publication WO01/27307
Patent document 14: U.S. Patent No. 7,759,094
Patent document 15: International Patent Publication WO03/00666
Patent document 16: U.S. Patent Published Application No. No. 2009298136
Patent document 17: International Patent Publication WO2008/12724
Patent document 18: International Patent Publication WO2009/04337
Patent document 19: International Patent Publication WO2005/10856
Patent document 20: International Patent Publication WO2007/07704
Patent document 21: International Patent Publication WO2006/082254
Patent document 22: U.S. Patent Published Application No. No. 20100047879
Patent document 23: U.S. Patent Published Application No. No. 20050069994

### Non-patent documents

Non-patent document 1: Dassler et al., Mol. Microbiol., 36, 1101-1112 (2000)
Non-patent Document 2: Frederich C., Neidhardt et al., Escherichia Coli and Salmonella Cellular and Molecular Biology, 2nd Edition, Vol. 1, ASM Press, 514-527
Non-patent document 3: Ostrowski et al., Journal of Biological Chemistry, 264(27):15796-15808 (1989)
Non-patent document 4: Li et al., Gene, 53(2-3):227-234 (1987)
Non-patent document 5: Gaudu and Fontecave, European Journal of Biochemistry, 226 (2):459-463 (1994)
Non-patent document 6: Eschenbrenner et al., Journal of Biological Chemistry, 270(35):20550-20555 (1995)
Non-patent document 7: Ostrowski et al., Journal of Biological Chemistry, 264(26):15726-15737 (1989)
Non-patent document 8: Peakman et al., European Journal of Biochemistry, 191(2):315-323 (1990)
Non-patent document 9: Macdonald and Cole, Molecular and General Genetics, 200(2):328-334 (1985)
Non-patent document 10: Warren et al., Biochemical Journal, 265(3):725-729 (1990)
Non-patent document 11: Spencer et al., FEBS Letters 335(1):57-60 (1993)

The patent document US2007/0026505 discloses bacterial strains that are engineered to increase the production of amino acids, such as methionine and cysteine. Among others, increased expression of sulfite reductase is proposed to increase sulfate assimilation and production of sulfide.

### Summary of the Invention

An object of the present invention is to provide a method for producing L-cysteine, a related substance thereof, or a mixture thereof by developing a novel technique for improving L-cysteine-producing ability of a bacterium.

The inventors of the present invention conducted various researches in order to achieve the aforementioned object, as a result, found that, when thiosulfate is used as the sulfur source, L-cysteine-producing ability of a bacterium could be improved by modifying the bacterium so that expression of a gene involved in the sulfite reduction is increased, and thus accomplished the present invention.

That is, the present invention can be embodied as follows.
[1] A method for producing L-cysteine, a' related substance thereof, or a mixture thereof, which comprises:
   culturing a bacterium belonging to the family *Enterobacteriaceae* in a medium containing thiosulfate, which bacterium has L-cysteine-producing ability and is modified so that expression of the *cysG* gene is increased by increasing copy number of the gene, or by modifying an expression control sequence of the gene; and
   collecting L-cysteine, a related substance thereof, or a mixture thereof accumulated in the medium,
   wherein the related substance is selected from the group consisting of L-cystine, S-sulfocysteine, a thiazolidine derivative, a hemithioketal, γ-glutamylcysteine, glutathione, cystathionine, homocysteine, L-methionine, and S-adenosylmethionine.
[2] The method according to [1] above, wherein the bacterium is further modified so that expression of a gene selected from the group consisting of the *cysJ* gene and the *cysI* gene by increasing copy number of the gene, or by modifying an expression control sequence of the gene.
[3] The method according to [2] above, wherein expression of the *cysG* gene, the *cysJ* gene, and the *cysI* gene is increased.
[4] The method according to any one of [1] to [3] above, wherein the bacterium is a bacterium belonging to the genus *Pantoea.*
[5] The method according to [4] above, wherein the bacterium is *Pantoea ananatis.*
[6] The method according to any one of [1] to [3] above, wherein the bacterium is a bacterium belonging to the genus *Escherichia.*
[7] The method according to [6] above, wherein the bacterium is *Escherichia coli.*
[8] The method according to any one of [1] to [7] above, wherein the bacterium further has a characteristic selected from the group consisting of the following characteristics (1) and (2):
   (1) activity of an enzyme involved in the biosynthesis of L-cysteine is enhanced,
   (2) expression of a gene coding for a protein involved in secretion of L-cysteine is enhanced.
[9] The method according to any one of [1] to [8] above, wherein the related substance is L-cystine or a thiazolidine derivative.

### Brief Description of the Drawings

Fig. 1 shows outline of the biosynthesis pathway of L-cysteine for the case of using glucose as a carbon source and sulfate ion or thiosulfate ion as a sulfur source, and examples of involved genes.
Fig. 2 shows the nucleotide sequence (SEQ ID NO: 73) of ligation site of a wild-type promoter Pnlp0 of the *nlpD* gene and a gene ligated downstream thereof, and the nucleotide sequence (SEQ ID NO: 74) of ligation site of a mutant type promoter Pnlp8 of the *nlpD* gene and a gene ligated downstream thereof.
Fig. 3 shows disposition of primers in the production of a *cysEX* gene fragment.
Fig. 4 shows the structure of the plasmid pACYC-DES.
Fig. 5 shows outline of disruption of d0191 gene in P. *ananatis* SC17(0).

### Detailed Description of the Preferred Embodiments

### <1> Bacterium used for the method of the present invention

The bacterium used for the method of the present invention (henceforth also referred to as the bacterium of the present invention) is a bacterium belonging to the family *Enterobacteriaceae,* having L-cysteine-producing ability and modified so that expression of a gene involved in sulfite reduction , i.e. the cysG gene, is increased.

In the present invention, L-cysteine can be L-cysteine in the free form, a salt thereof, or a mixture thereof. Examples of the salt include, for example, sulfate, hydrochloride, carbonate, ammonium salt, sodium salt, and potassium salt.

In the present invention, the L-cysteine-producing ability refers to an ability of a bacterium to produce L-cysteine, a related substance thereof, or a mixture thereof and accumulate it in a medium or the cells of the bacterium in such an amount that L-cysteine, the related substance thereof, or the mixture thereof can be collected from the medium or cells, when the bacterium is cultured in the medium. A bacterium having L-cysteine-producing ability means a bacterium that can produce and accumulate L-cysteine, a related substance thereof, or a mixture thereof in a medium in a larger amount compared with a wild-type strain or a parent strain. A bacterium having L-cysteine-producing ability preferably means a bacterium that can produce and accumulate L-cysteine, a related substance thereof, or a mixture thereof in a medium in an amount of 0.05 g/L or more, more preferably 0.1 g/L or more, still more preferably 0.2 g/L or more, particularly preferably 0.4 g/L or more.

A portion of L-cysteine produced by the bacterium can be converted into L-cystine in the medium by formation of a disulfide bond. Further, S-sulfocysteine can be generated by the reaction of L-cysteine and thiosulfate contained in the medium (Szczepkowski T.W., Nature, vol. 182 (1958)). Furthermore, L-cysteine generated in bacterial cells can be condensed with a ketone or aldehyde, for example, pyruvic acid, which exists in the cells, to produce a thiazolidine derivative via a hemithioketal as an intermediate (refer to Japanese Patent No. 2992010). These thiazolidine derivative and hemithioketal can exist as an equilibrated mixture.

Further, L-cysteine is used as a starting material of the biosyntheses of γ-glutamylcysteine, glutathione, cystathionine, homocysteine, L-methionine, S-adenosylmethionine, and so forth. Therefore, by using a bacterium having an ability to produce any of these compounds to be produced via L-cysteine in addition to the ability to produce L-cysteine, these compounds can be produced.

Therefore, in the present invention, the L-cysteine-producing ability is not limited to an ability to accumulate only L-cysteine in a medium or cells, but also includes an ability to accumulate L-cysteine, L-cystine, a derivative of L-cysteine, i.e. S-sulfocysteine, a thiazolidine derivative, and a hemithioketal, a compound to be produced via L-cysteine, i.e. γ-glutamylcysteine, glutathione, cystathionine, homocysteine, L-methionine, and S-adenosylmethionine, or a mixture thereof in the medium or cells. In the present invention, L-cystine, said derivative of L-cysteine and said compound to be produced via L-cysteine are collectively referred to as a related substance of L-cysteine.

The bacterium having L-cysteine-producing ability can be a bacterium inherently having L-cysteine-producing ability, or it can be obtained by modifying a microorganism such as those described below by mutagenesis or a recombinant DNA technique so that it acquires L-cysteine-producing ability.

The bacterium used for the present invention is not particularly limited, so long as a bacterium belonging to the family *Enterobacteriaceae* such as those of the genera *Escherichia, Enterobacter, Pantoea, Klebsiella, Serratia, Erwinia, Salmonella,* and *Morganella* and having L-cysteine-producing ability is chosen. Specifically, those classified into the family *Enterobacteriaceae* according to the taxonomy used in the NCBI (National Center for Biotechnology Information) database (http://www.ncbi.nlm.nih.gov/Taxonomy/Browser/wwwtax.cgi?id 91347) can be used. As a parent strain of the family *Enterobacteriaceae* used for the modification, it is desirable to use, especially, a bacterium of the genus *Escherichia, Enterobacter, Pantoea, Erwinia, Enterobacter,* or *Klebsiella.*

Although the *Escherichia* bacteria are not particularly limited, specifically, those described in the work of Neidhardt et al. (Backmann B.J., 1996, Derivations and Genotypes of some mutant derivatives of Escherichia coli K-12, p.2460-2488, Table 1, In F.D. Neidhardt (ed.), Escherichia coli and Salmonella Cellular and Molecular Biology/Second Edition, American Society for Microbiology Press, Washington, D.C.) can be used. Examples of *Escherichia* bacteria include, for example, *Escherichia coli.* Specific examples of *Escherichia coli* include the *Escherichia coli* W3110 strain (ATCC 27325), *Escherichia coli* MG1655 strain (ATCC 47076) and so forth derived from the prototype wild-type strain, K12 strain.

These strains are available from, for example, the American Type Culture Collection (Address: 12301 Parklawn Drive, Rockville, Maryland 20852, P.O. Box 1549, Manassas, VA 20108, United States of America). That is, registration numbers are given to each of the strains, and the strains can be ordered by using these registration numbers (refer to http://www.atcc.org/). The registration numbers of the strains are listed in the catalogue of the American Type Culture Collection.

Examples of the *Enterobacter* bacteria include, for example, *Enterobacter agglomerans* and *Enterobacter aerogenes.* An example of typical strains of the genus *Enterobacter* includes *Enterobacter agglomerans* ATCC 12287 strain. Also, specifically, the strains exemplified in European Patent Publication No. 952221 can be used.

Examples of the Pantoea bacteria include, for example, *Pantoea ananatis, Pantoea stewartii, Pantoea agglomerans,* and *Pantoea citrea.*

Specific examples of Pantoea *ananatis* include the *Pantoea ananatis* AJ13355 strain (FERM BP-6614) and SC17 strain (FERM BP-11091). The AJ13355 strain is a strain isolated from soil in Iwata-shi, Shizuoka-ken, Japan as a strain that can proliferate in a low pH medium containing L-glutamic acid and a carbon source. The SC17 strain is a strain selected as a low phlegm-producing mutant strain from the AJ13355 strain (U.S. Patent No. 6,596,517). The *Pantoea ananatis* AJ13355 strain was deposited at the National Institute of Bioscience and Human-Technology, Agency of Industrial Science and Technology, Ministry of International Trade and Industry (currently, the National Institute of Advanced Industrial Science and Technology, International Patent Organism Depositary, Address: Tsukuba Central 6, 1-1, Higashi 1-Chome, Tsukuba-shi, Ibaraki-ken, 305-8566, Japan) on February 19, 1998 and assigned an accession number of FERM P-16644. It was then converted to an international deposit under the provisions of Budapest Treaty on January 11, 1999 and assigned an accession number of FERM BP-6614. The *Pantoea ananatis* SC17 strain was deposited at the National Institute of Advanced Industrial Science and Technology, International Patent Organism Depositary (Address: Tsukuba Central 6, 1-1, Higashi 1-Chome, Tsukuba-shi, Ibaraki-ken, 305-8566, Japan) on February 4, 2009 and assigned an accession number of FERM BP-11091. The *Pantoea ananatis* AJ13355 strain was identified as *Enterobacter agglomerans* when it was isolated, and deposited as the *Enterobacter agglomerans* AJ13355 strain. However, it was recently reclassified into *Pantoea ananatis* on the basis of nucleotide sequence analysis of 16S rRNA and so forth.

Examples of the *Erwinia* bacteria include *Erwinia amylovora* and *Erwinia carotovora,* and examples of the *Klebsiella* bacteria include *Klebsiella planticola.*

In particular, *Pantoea* bacteria, *Erwinia* bacteria, and *Enterobacter* bacteria are classified as γ-proteobacteria, and they are taxonomically very close to one another (J. Gen. Appl. Microbiol., 1997, 43, 355-361; International Journal of Systematic Bacteriology, Oct. 1997, pp.1061-1067). Therefore, some bacteria belonging to the genus *Enterobacter* were reclassified into *Pantoea agglomerans, Pantoea dispersa,* or the like, on the basis of DNA-DNA hybridization experiments etc. (International Journal of Systematic Bacteriology, July 1989, 39(3), pp.337-345). For example, some strains of *Enterobacter agglomerans* were reclassified into *Pantoea agglomerans, Pantoea ananatis,* or *Pantoea stewartii* on the basis of nucleotide sequence analysis of 16S rRNA etc. Furthermore, some bacteria belonging to the genus *Erwinia* were reclassified into *Pantoea ananas* or *Pantoea stewartii* (refer to International Journal of Systematic Bacteriology, Jan 1993;43(1), pp.162-173). In the present invention, a bacterium belonging to any of the genus *Enterobacter, Pantoea, Erwinia,* and the like can be used so long as it is a bacterium classified into the family *Enterobacteriaceae.*

Methods for imparting L-cysteine-producing ability to bacteria belonging to the family *Enterobacteriaceae* and methods for enhancing L-cysteine-producing ability of such bacteria are described below.

To impart L-cysteine-producing ability to a bacterium, methods conventionally employed in the breeding of coryneform bacteria, *Escherichia* bacteria, and so forth can be used. Such methods include acquiring an auxotrophic mutant strain, an analogue resistant strain, or a metabolic regulation mutant strain, or constructing a recombinant strain in which an L-cysteine biosynthesis enzyme is overexpressed, and so forth (see "Amino Acid Fermentation", Gakkai Shuppan Center (Ltd.), 1st Edition, published May 30, 1986, pp.77-100). In the breeding of L-cysteine-producing bacteria, one or two or more kinds of the above-described properties such as auxotrophy, analogue resistance, and metabolic regulation mutation can be imparted. Also, expression of one or two or more kinds of the L-cysteine biosynthesis enzymes can be enhanced. Furthermore, imparting such properties as auxotrophy, analogue resistance, and metabolic regulation mutation can be combined with enhancing a biosynthesis enzyme.

An auxotrophic mutant strain, L-cysteine analogue resistant strain, or metabolic regulation mutant strain having L-cysteine-producing ability can be obtained by subjecting a parent strain or wild-type strain to conventional mutagenesis, such as exposure to X-rays or UV irradiation or a treatment with a mutagen such as N-methyl-N'-nitro-N-nitrosoguanidine (MNNG) or ethyl methanesulfonate (EMS), and then selecting a strain exhibiting autotrophy, analogue resistance or a metabolic regulation mutation and having L-cysteine-producing ability from the obtained mutant strains.

Methods for imparting L-cysteine-producing ability to bacteria belonging to the family *Enterobacteriaceae* or enhancing L-cysteine-producing ability of such bacteria, and bacteria having L-cysteine-producing ability are specifically exemplified below.

### <Impartation or enhancement of L-cysteine-producing ability, and L-cysteine-producing bacteria>

L-cysteine-producing ability of a bacterium can be imparted or enhanced by enhancing the biosynthesis system of L-cysteine. "To enhance the biosynthesis system of L-cysteine" means, for example, to enhance activity of an enzyme involved in the biosynthesis of L-cysteine. Examples of the enzyme involved in the biosynthesis of L-cysteine include enzymes of the L-cysteine biosynthesis pathway and enzymes involved in synthesis of a compound serving as a substrate in the L-cysteine biosynthesis pathway, such as L-serine, and specific examples include serine acetyltransferase (SAT) and 3-phosphoglycerate dehydrogenase (PGD). Enhancement of an enzymatic activity can be attained by enhancing expression of a gene coding for the objective enzyme, or by enhancing specific activity of the objective enzyme, as described later. Because serine acetyltransferase is inhibited by feedback inhibition by L-cysteine, the enzymatic activity of this enzyme can be enhanced by, in particular, incorporating a mutant type *cysE* gene coding for serine acetyltransferase of which feedback inhibition is attenuated or eliminated into a bacterium. Further, because 3-phosphoglycerate dehydrogenase is inhibited by feedback inhibition by serine, the enzymatic activity of this enzyme can be enhanced by, in particular, incorporating a mutant type serA gene coding for a mutant 3-phosphoglycerate dehydrogenase of which feedback inhibition is attenuated or eliminated into a bacterium.

As mutant SAT which is derived from *Escherichia coli* and resistant to feedback inhibition, there are specifically known the mutant SAT in which the methionine residue at position 256 is replaced with glutamate residue (Japanese Patent Laid-open No. 11-155571), the mutant SAT in which the methionine residue at position 256 is replaced with isoleucine residue (Denk, D. and Boeck, A., J. General Microbiol., 133, 515-525 (1987)), the mutant SAT having a mutation in the region from the amino acid residue at position 97 to the amino acid residue at position 273 or deletion of the C-terminus region from the amino acid residue at position 227 (International Patent Publication WO97/15673, U.S. Patent No. 6,218,168), the mutant SAT in which the amino acid sequence corresponding to positions 89 to 96 of wild-type SAT contains one or more mutations, and which is desensitized to feedback inhibition by L-cysteine (U.S. Patent Published Application No. 20050112731(A1)), the mutant SAT in which the Val residue and the Asp residue at positions 95 and 96 of wild-type SAT are replaced with Arg residue and Pro residue, respectively (name of the mutant gene: *cysE5*, U.S. Patent Published Application No. 20050112731(A1)), the mutant SAT in which the threonine residue at position 167 is replaced with an alanine residue (U.S. Patent No. 6,218,168, U.S. Patent Published Application No. 20050112731(A1)), and so forth.

The gene coding for SAT is not limited to the gene of *Escherichia coli,* and any gene coding for a protein having the SAT activity can be used. For example, an SAT isozyme of *Arabidopsis thaliana* desensitized to feedback inhibition by L-cysteine is known, and the gene encoding this SAT can also be used (FEMS Microbiol. Lett., 179, 453-459 (1999)).

Further, as a gene coding for a mutant PGD resistant to feedback inhibition by serine, the *serA5* gene (U.S. Patent No. 6,180,373) is known.

Further, L-cysteine-producing ability of a bacterium can also be improved by enhancing the L-cysteine secretion system. The L-cysteine secretion system can be enhanced by enhancing expression of a gene coding for a protein involved in secretion of L-cysteine. Examples of the protein involved in secretion of L-cysteine include the YdeD protein encoded by the *ydeD* gene, the YfiK protein encoded by the *yfiK* gene, and the YeaS protein encoded by the *yeaS* gene. Therefore, by enhancing expression of the *ydeD* gene (Dassler et al., Mol. Microbiol., 36, 1101-1112 (2000)), the *yfiK* gene (Japanese Patent Laid-open (Kokai) No. 2004-49237) or the *yeaS* gene (European Patent Laid-open No. 1016710), L-cysteine-producing ability can be enhanced. Further, by introducing a mutation into the threonine residue of position 28, the phenylalanine residue of position 137, and/or the leucine residue of position 188 of the YeaS protein, the L-cysteine secretion system is also enhanced, and L-cysteine-producing ability can be enhanced (European Patent Laid-open No. 2218729). Specifically, a mutation for replacing the threonine residue at position 28 with asparagine residue, a mutation for replacing the phenylalanine residue at position 137 with serine, glutamine, alanine, histidine, cysteine or glycine residue, and/or a mutation for replacing the leucine residue at position 188 with glutamine residue in the YeaS protein is preferred (European Patent Laid-open No. 2218729).

Further, proteins suitable for secreting a substance showing cytotoxicity include those involved in secretion of L-cysteine. Therefore, by enhancing expression of a gene coding for any one of them, the L-cysteine secretion system can be enhanced. For example, by increasing expression of the *mar* locus, *emr* locus, *acr* locus, *cmr* locus, mex gene, *bmr* gene or *qacA* gene (U.S. Patent No. 5,972,663), or *emrAB, emrKY, yojIH*, *acrEF, bcr* or *cusA* gene (Japanese Patent Laid-open (Kokai) No. 2005-287333), which encode proteins suitable for secreting a substance showing cytotoxicity from cells, L-cysteine-producing ability can be enhanced.

Furthermore, the L-cysteine-producing ability can also be improved by enhancing the sulfate/thiosulfate transport system. The sulfate/thiosulfate transport system proteins are encoded by the *cysPTWAM* cluster genes (Japanese Patent Laid-open (Kokai) No. 2005-137369, European Patent No. 1528108).

Further, L-cysteine-producing ability of a bacterium can be improved by reducing the activity of cysteine desulfhydrase, which contributes to decomposition of L-cysteine. As proteins having the cysteine desulfhydrase activity of *Escherichia coli,* cystathionine-β-lyase encoded by the *metC* gene (Japanese Patent Laid-open (Kokai) No. 11-155571, Chandra et al., Biochemistry, 21, 3064-3069 (1982)), tryptophanase encoded by the *tnaA* gene (Japanese Patent Laid-open (Kokai) No. 2003-169668, Austin Newton et al., J. Biol. Chem., 240, 1211-1218 (1965)), O-acetylserine sulfhydrylase B encoded by the *cysM* gene (Japanese Patent Laid-open (Kokai) No. 2005-245311) and MalY encoded by the *malY* gene (Japanese Patent Laid-open (Kokai) No. 2005-245311) are known. Furthermore, the *d0191* gene of *Pantoea ananatis* is known as a gene encoding a protein having cysteine desulfhydrase activity (Japanese Patent Laid-open (Kokai) No. 2009-232844). The enzymatic activity can be reduced by the methods described later.

The L-cysteine-producing bacterium can have one of the aforementioned properties for improving L-cysteine-producing ability, or two or more of them in any combination. For example, in the L-cysteine-producing bacterium, it is preferred that either the biosynthesis system of L-cysteine or the secretion system of L-cysteine is enhanced, and it is more preferred that the both are enhanced.

Specific examples of L-cysteine-producing bacteria include, but not limited to, *Escherichia* bacteria such as the *E. coli* JM15 strain transformed with multiple kinds of *cysE* gene alleles encoding serine acetyltransferase (SAT) resistant to feedback inhibition (U.S. Patent 6,218,168), *E*. *coli* W3110 strain in which a gene encoding a protein suitable for secretion of a cytotoxic substance is overexpressed (U.S. Patent No. 5,972,663), *E. coli* in which cysteine desulfhydrase activity is decreased (Japanese Patent Laid-open (Kokai) No. 11-155571), and *E. coli* W3110 strain in which activity of the positive transcriptional control factor of the cysteine regulon encoded by the *cysB* gene is increased (WO01/27307), *E.coli* having the plasmid pACYC-DES (Japanese Patent Laid-open (Kokai) No. 2005-137369 (U.S. Patent Published Application No. 20050124049(A1), European Patent Laid-open No. 1528108(A1))) containing the *ydeD* gene, a mutant *cysE* gene, and a mutant *serA5* gene, and so forth. pACYC-DES is a plasmid obtained by inserting the above three kinds of genes into pACYC184, and expression of each of the genes is controlled by the PompA promoter.

The ability to produce compound(s) biosynthesized from L-cysteine as a starting material, such as γ-glutamylcysteine, glutathione, cystathionine, homocysteine, L-methionine, and S-adenosylmethionine, can also be imparted or enhanced by enhancing activity of an enzyme of the biosynthesis system of an objective compound, or reducing activity of an enzyme of a pathway branching from biosynthesis system of the objective compound or an enzyme that decomposes the objective compound.

For example, γ-glutamylcysteine-producing ability can be enhanced by enhancing the γ-glutamylcysteine synthetase activity, and/or reducing the glutathione synthetase activity. Further, glutathione-producing ability can be imparted or enhanced by enhancing the γ-glutamylcysteine synthetase activity and/or the glutathione synthetase activity. Further, by using a mutant γ-glutamylcysteine synthetase resistant to feedback inhibition by glutathione, the ability to produce γ-glutamylcysteine or glutathione can be enhanced. Production of glutathione is described in detail in the overview of Li et al. (Yin Li, Gongyuan Wei, Jian Chen, Appl. Microbiol. Biotechnol., 66:233-242 (2004)).

L-methionine-producing ability can be imparted or enhanced by imparting L-threonine auxotrophy or norleucine resistance (Japanese Patent Laid-open (Kokai) No. 2000-139471). In *E. coli,* the genes of the enzymes involved in the biosynthesis of L-threonine exist as the threonine operon *(thrABC),* and an L-threonine auxotrophic strain that has lost the biosynthesis ability for L-homoserine and the following compounds can be obtained by, for example, deleting the *thrBC* moiety. In a norleucine resistant strain, the S-adenosylmethionine synthetase activity is attenuated, and L-methionine-producing ability is imparted or enhanced. In *E. coli,* S-adenosylmethionine synthetase is encoded by the *metK* gene. L-methionine-producing ability can also be imparted or enhanced by deleting the methionine repressor or enhancing activity of an enzyme involved in the L-methionine biosynthesis, such as homoserine transsuccinylase, cystathionine γ-synthase and aspartokinase-homoserine dehydrogenase II (Japanese Patent Laid-open (Kokai) No. 2000-139471). In *E. coli,* the methionine repressor is encoded by the *metJ* gene, homoserine transsuccinylase is encoded by the metA gene, cystathionine γ-synthase is encoded by the *metB* gene, and aspartokinase-homoserine dehydrogenase II is encoded by the *metL* gene. Further, by using a mutant homoserine transsuccinylase resistant to feedback inhibition by L-methionine, L-methionine-producing ability can also be imparted or enhanced (Japanese Patent Laid-open (Kokai) No. 2000-139471, U.S. Patent Published Application No. 20090029424). Because L-methionine is biosynthesized via L-cysteine as an intermediate, L-methionine-producing ability can also be improved by improving L-cysteine-producing ability (Japanese Patent Laid-open (Kokai) No. 2000-139471, U.S. Patent Published Application No. 20080311632). Therefore, for imparting or enhancing L-methionine-producing ability, it is also effective to impart or enhance L-cysteine-producing ability.

Specific examples of L-methionine-producing bacteria include *E. coli* strains such as AJ11539 (NRRL B-12399), AJ11540 (NRRL B-12400), AJ11541 (NRRL B-12401), AJ11542 (NRRL B-12402) (British Patent No. 2075055), 218 strain resistant to norleucine, which is an analogue of L-methionine (VKPM B-8125, Russian Patent No. 2209248), and 73 strain (VKPM B-8126, Russian Patent No. 2215782).

Further, as an L-methionine-producing bacterium, AJ13425 (FERM P-16808, Japanese Patent Laid-open (Kokai) No. 2000-139471) derived from the *E. coli* W3110 can also be used. AJ13425 is an L-threonine auxotrophic strain in which the methionine repressor is deleted, intracellular S-adenosylmethionine synthetase activity is attenuated, and intracellular homoserine transsuccinylase activity, cystathionine γ-synthase activity, and aspartokinase-homoserine dehydrogenase II activity are enhanced. AJ13425 was deposited on May 14, 1998 at the National Institute of Bioscience and Human Technology, Agency of Industrial Science and Technology, Ministry of International Trade and Industry (currently National Institute of Advanced Industrial Science and Technology, International Patent Organism Depository, Address: Tsukuba Central 6, 1-1, Higashi 1-Chome, Tsukuba-shi, Ibaraki-ken, 305-8566, Japan), and assigned an accession number of FERM P-16808.

Because cystathionine and homocysteine are intermediates of the L-methionine biosynthesis pathway, it is effective to partially use the aforementioned methods for enhancing L-methionine-producing ability for enhancing abilities to produce these substances. As specific methods for enhancing cystathionine-producing ability, there are known a method using a methionine-auxotrophic mutant strain (Japanese Patent Application No. 2003-010654) and a method adding cysteine (or raw material for biosynthesis thereof) and/or homoserine (or raw material for biosynthesis thereof) to a fermentation medium (Japanese Patent Laid-open (Kokai) No. 2005-168422). Since homocysteine is produced by using cystathionine as a precursor, the aforementioned methods for enhancing cystathionine-producing ability are also effective for enhancing homocysteine-producing ability.

Further, the ability to produce compound(s) biosynthesized by using L-methionine as a starting material, such as S-adenosylmethionine, can also be imparted or enhanced by enhancing activity of an enzyme of the biosynthesis system of an objective compound, or reducing activity of an enzyme of a pathway branching from the biosynthesis pathway of the objective compound or an enzyme that decomposes the objective compound. For example, S-adenosylmethionine-producing ability can be imparted or enhanced by enhancing the methionine adenosyltransferase activity (European Patent Laid-open Nos. 0647712 and 1457569) or enhancing the secretion factor MdfA encoded by the *mdfA* gene (U.S. Patent No. 7,410,789).

Methods for increasing enzymatic activities of SAT and so forth and reducing enzymatic activities of cysteine desulfhydrase and so forth are exemplified below.

### <Method for increasing enzymatic activity>

In the present invention, the expression "enzymatic activity is increased" means that an objective enzymatic activity is increased compared with that of a non-modified strain such as a wild-type strain or a parent strain. Although the degree of increase of the enzymatic activity is not particularly limited so long as the enzymatic activity is increased compared with a non-modified strain, the enzymatic activity is increased preferably 1.5 times or more, more preferably 2 times or more, still more preferably 3 times or more, compared with that of a non-modified strain. Further, the case where "enzymatic activity is increased" includes not only a case where an objective enzymatic activity is increased in a strain natively having the objective enzymatic activity, but also a case where an objective enzymatic activity is imparted to a strain not natively having the objective enzymatic activity. Moreover, so long as an objective enzymatic activity is increased as a result, an enzyme that is natively possessed by a bacterium can be attenuated or deleted, and then an appropriate enzyme can be introduced.

A modification for increasing the enzymatic activity can be attained by, for example, enhancing expression of a gene coding for an objective enzyme. In the present invention, the expression "enhancement of expression of a gene" is used for the same meaning as "increase of expression of a gene".

Enhancement of expression of a gene can be attained by, for example, increasing the copy number of the gene.

The copy number of an objective gene can be increased by introducing the gene into a chromosome. A gene can be introduced into a chromosome by, for example, using homologous recombination. For example, a large number of copies of a gene can be introduced into a chromosome by performing homologous recombination for a sequence that exists on a chromosome in a large copy number as a target. Examples of sequence existing on a chromosome in a large copy number include, but not limited to, repetitive DNA and inverted repeats existing at the both ends of a transposon. An objective gene can be ligated on the side of a gene coding for an objective enzyme on a chromosome in tandem or can also be introduced into an unnecessary gene on a chromosome so that the objective gene overlaps with the unnecessary gene. Such gene introduction can be attained by using a temperature sensitive vector or by using an integration vector. Alternatively, as disclosed in U.S. Patent No. 5,595,889, it is also possible to incorporate an objective gene into a transposon, and allow it to be transferred to a chromosomal DNA to introduce a large number of copies of the gene. As the transposon, for example, Mu, Tn10 and Tn5 can be used. Whether an objective gene has been introduced into a chromosome can be confirmed by Southern hybridization using a part of the objective gene as a probe.

Further, the copy number of an objective gene can also be increased by introducing a vector containing the gene into a host bacterium. For example, the copy number of an objective gene can be increased by ligating a DNA fragment containing the objective gene with a vector that functions in the host bacterium to construct an expression vector of the objective gene, and transforming the host bacterium with the expression vector. As the vector, a vector autonomously replicable in the cell of the host bacterium can be used. The vector is preferably a multicopy vector. Further, the vector preferably has a marker such as an antibiotic resistance gene for selection of the transformants. Examples of vector autonomously replicable in *Escherichia coli* cells include pUC19, pUC18, pHSG299, pHSG399, pHSG398, pACYC184, pBR322, pSTV29 (all of these are available from Takara Bio), pMW219 (NIPPON GENE), pTrc99A (Pharmacia), pPROK series vectors (Clontech), pKK233-2 (Clontech), pET series vectors (Novagen), pQE series vectors (QIAGEN), broad host spectrum vector RSF1010, and so forth.

Further, expression of a gene can be enhanced by improving the transcription efficiency of the gene. The transcription efficiency of a gene can be improved by, for example, substituting a stronger promoter for the promoter of the gene on the chromosome. The "stronger promoter" means a promoter providing improved transcription of a gene compared with the natively existing wild-type promoter. As a stronger promoter, for example, known high expression promoters, such as T7 promoter, trp promoter, lac promoter, tac promoter, and PL promoter, can be used. Further, as the stronger promoter, a highly-active type of a usual promoter can be obtained by using various reporter genes. For example, by making the -35 and -10 regions in a promoter region closer to the consensus sequence, activity of the promoter can be enhanced (International Patent Publication WO00/18935). Methods for evaluating the strength of promoters and examples of strong promoter are described in the paper of Goldstein et al. (Prokaryotic Promoters in Biotechnology, Biotechnol. Annu. Rev., 1, 105-128 (1995)), and so forth.

Further, expression of a gene can also be enhanced by improving the translation efficiency of the gene. The translation efficiency of a gene can be improved by, for example, replacing the SD sequence (also referred to as ribosome binding site (RBS)) on a chromosome with a stronger SD sequence. The "stronger SD sequence" means a SD sequence that provides improved translation of mRNA compared with the natively existing wild-type SD sequence. Examples of the stronger SD sequence include, for example, RBS of the gene 10 derived from phage T7 (Olins P.O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and translation efficiency of a gene can also be improved by modifying them.

In the present invention, sites affecting gene expression, such as promoter, SD sequence and spacer region between RBS and the start codon, are also generically called "expression control regions" or "expression control sequences". An expression control region can be determined by using a promoter-search vector or gene analysis software such as GENETYX. Such expression control regions can be modified by, for example, a method using a temperature sensitive vector or the Red driven integration method (WO2005/010175).

Further, expression of an objective gene can also be enhanced by amplifying a regulator that increases expression of the gene, or deleting or attenuating a regulator that reduces expression of the gene. Examples of the regulator include, for example, those belonging to the LysR family, and so forth, and they can be found by using a database, such as EcoCyc (http://ecocyc.org/), or the like. A regulator can be modified with monitoring the increase of the transcription amount of the objective gene or the increase of the amount of the objective protein as an index.

Such methods for enhancing gene expression as mentioned above can be used independently or in an arbitrary combination.

Further, a modification that increases the enzymatic activity can also be attained by, for example, enhancing specific activity of the objective enzyme. An enzyme showing enhanced specific activity can be obtained by, for example, searching various bacteria. Further, a highly-active type of a usual enzyme can also be obtained by introducing a mutation into the usual enzyme. Enhancement of specific activity can be independently used, or can be used in an arbitrary combination with such methods for enhancing gene expression as mentioned above.

The method of the transformation is not particularly limited, and conventionally known methods can be used. There can be used, for example, methods of treating recipient cells with calcium chloride so as to increase permeability thereof for DNA, which has been reported for *Escherichia coli* K-12 strain (Mandel, M. and Higa, A., J. Mol. Biol., 53:159-162 (1970)), and preparing competent cells from cells which are at the growth phase, followed by transformation with DNA, which has been reported for *Bacillus subtilis* (Duncan, C.H., Wilson, G.A. and Young, F.E., Gene, 1, 153 (1977)). Alternatively, there can also be used a method of making DNA-recipient cells into protoplasts or spheroplasts, which can easily take up recombinant DNA, followed by introducing a recombinant DNA into the cells, which is known to be applicable to *Bacillus subtilis,* actinomycetes and yeasts (Chang, S. and Choen, S.N., 1979, Molec. Gen. Genet., 168:111 (1979); Bibb, M.J., Ward, J.M. and Hopwood, O.A., Nature, 274, 398 (1978); Hinnen, A., Hicks, J.B. and Fink, G.R., Proc. Natl. Acad. Sci. USA, 75, 1929 (1978)).

Increase of an objective enzymatic activity can be confirmed by measuring the enzymatic activity.

Increase of the transcription amount of an objective gene can be confirmed by comparing amount of mRNA transcribed from the gene with that of a wild-type strain or a non-modified strain. Examples of method for evaluating amount of mRNA include Northern hybridization, RT-PCR, and so forth (Molecular Cloning, Cold spring Harbor Laboratory Press, Cold Spring Harbor (USA), 2001).

Increase of the amount of an objective protein can be confirmed by Western blotting using an antibody (Molecular Cloning, Cold Spring Harbor Laboratory Press, Cold Spring Harbor (USA), 2001).

### <Method for reducing enzymatic activity>

In the present invention, the expression "enzymatic activity is reduced" means that the enzymatic activity is decreased compared with that of a non-modified strain such as a wild-type strain or a parent strain, and includes a case where the activity has completely disappeared. Although degree of the reduction of the enzymatic activity is not particularly limited so long as the activity is reduced compared with that of a non-modified strain, it is preferably reduced to, for example, 75% or less, 50% or less, 25% or less, or 10% or less compared with that of a non-modified strain, and the complete disappearance of the activity is particularly preferred. Depending on the type of enzyme, it can also be rather preferable not to completely eliminate the enzymatic activity.

A modification for reducing the enzymatic activity can be attained by, for example, reducing expression of a gene coding for the objective enzyme. Expression of a gene can be reduced by, for example, modifying an expression control sequence, such as promoter and SD sequence, of the gene. When an expression control sequence is modified, one or more nucleotides, more preferably two or more nucleotides, particularly preferably three or more nucleotides, of the expression control sequence are modified. Further, a part or all of the expression control sequence can be deleted. Further, expression of a gene can also be reduced by expressing an antisense RNA.

Further, a modification for reducing the enzymatic activity can also be attained by, for example, deleting a part or all of a coding region of a gene coding for an objective enzyme on a chromosome. Further, the whole gene including the sequences upstream and downstream of the gene on a chromosome can be deleted. The region to be deleted can be an N-terminus region, an internal region or a C-terminus region, so long as the enzymatic activity can be reduced. Deletion of a longer region can usually more surely inactivate a gene. Furthermore, it is preferred that reading frames upstream and downstream of the region to be deleted are not the same.

Further, a modification for reducing the enzymatic activity can also be attained by introducing a mutation for amino acid substitution (missense mutation), a stop codon (nonsense mutation), or a frame shift mutation which adds or deletes one or two nucleotides into a coding region of a gene coding for the objective enzyme on a chromosome (Journal of Biological Chemistry, 272:8611-8617 (1997); Proceedings of the National Academy of Sciences, USA, 95 5511-5515 (1998); Journal of Biological Chemistry, 266, 20833-20839 (1991)).

Further, a modification for reducing the enzymatic activity can also be attained by inserting another sequence into a coding region of a gene coding for an objective enzyme on a chromosome. Such a sequence can be inserted into any region of the gene, and insertion of a longer sequence can more surely inactivate the objective gene. It is preferred that reading frames upstream and downstream of the insertion site are not the same. The other sequence is not particularly limited so long as a sequence which decreases or deletes function of the encoded protein is chosen, and examples include a marker gene such as antibiotic resistance genes, a gene useful for L-cysteine production, and so forth.

A gene on a chromosome can be modified as described above by, for example, preparing a deletion-type gene of the gene, in which a partial sequence of the gene is deleted so that the deletion-type gene does not produce a protein that can normally function, and then transforming a bacterium with a recombinant DNA containing the deletion-type gene to cause homologous recombination between the deletion-type gene and the native gene on the chromosome, which results in substitution of the deletion-type gene for the native gene on the chromosome. The above operation can be made easier by introducing a marker gene suitable for a characteristic of the host such as auxotrophy into the recombinant DNA. The protein encoded by the deletion-type gene has a conformation different from that of the wild-type protein, even if it is even produced, and thus the function is reduced or deleted. Such gene disruption based on gene substitution utilizing homologous recombination has already been established, and examples include the method called Red-driven integration (Datsenko, K.A., and Wanner, B.L., Proc. Natl. Acad. Sci. USA, 97:6640-6645 (2000)), methods using a linear DNA such as the method of utilizing Red driven integration in combination with an excision system derived from λ phage (Cho, E.H., Gumport, R.I., Gardner, J.F., J. Bacteriol., 184:5200-5203 (2002)) (refer to WO2005/010175), methods using a plasmid containing a temperature sensitive replication origin, methods using a plasmid capable of conjugative transfer, methods utilizing a suicide vector without a replication origin in a host (U.S. Patent No. 6,303,383, Japanese Patent Laid-open (Kokai) No. 05-007491), and so forth.

Further, a modification for reducing the enzymatic activity can also be attained by, for example, mutagenesis. Examples of mutagenesis include exposure to UV irradiation or a treatment with a mutagen used for usual mutagenesis such as N-methyl-N'-nitro-N-nitrosoguanidine (MNNG), ethyl methanesulfonate (EMS) and methyl methanesulfonate (MMS).

Decrease of the enzymatic activity can be confirmed by measuring the enzymatic activity. The cysteine desulfhydrase activity can be measured by the method described in Japanese Patent Laid-open (Kokai) No. 2002-233384.

Decrease of the transcription amount of an objective gene can be confirmed by comparing the amount of mRNA transcribed from the gene with that of a non-modified strain. Examples of method for evaluating amount of mRNA include Northern hybridization, RT-PCR, and so forth (Molecular Cloning, Cold Spring Harbor Laboratory Press, Cold Spring Harbor, USA, 2001).

Decrease of the amount of an objective protein can be confirmed by Western blotting using an antibody (Molecular Cloning, Cold Spring Harbor Laboratory Press, Cold Spring Harbor, USA, 2001).

### <Enhancement of expression of gene involved in sulfite reduction>

The bacterium of the present invention is modified so that expression of a gene involved in sulfite reduction, i.e. the cysG gene, is increased. The bacterium of the present invention can be obtained by modifying a bacterium belonging to the family *Enterobacteriaceae* and having L-cysteine-producing ability such as those mentioned above so that expression of the gene involved in sulfite reduction is increased. The bacterium of the present invention can also be obtained by modifying a bacterium belonging to the family *Enterobacteriaceae* so that expression of the gene involved in sulfite reduction is increased, and then imparting or enhancing L-cysteine-producing ability.

Examples of a gene involved in sulfite reduction include a gene coding for a sulfite reductase. Sulfite reductase refers to a protein having sulfite-reducing activity, which means an activity of catalyzing the reaction of reducing sulfite ion (SO3²⁻) into sulfide ion (S²⁻). Examples of a gene coding for sulfite reductase include the *cysJ* gene and the *cysI* gene. The sulfite reductase of *Escherichia coli* is an NADPH-dependent sulfite reductase (EC 1.8.1.2), and it has an α₈β₄ complex structure comprising α subunits encoded by the *cysJ* gene and β subunits encoded by the *cysI* gene. Therefore, the expression that a protein shows the sulfite-reducing activity includes not only a case where the protein encoded by the gene shows sulfite-reducing activity by itself, but also a case where a complex including the protein as a subunit shows the sulfite-reducing activity.

Further, examples of the gene involved in sulfite reduction include a gene involved in the biosynthesis of a cofactor of sulfite reductase. Examples of the cofactor of sulfite reductase include siroheme used as a prosthetic group, and NADPH and flavin used as a coenzyme. Among these, siroheme is preferred. Examples of the gene involved in the biosynthesis of siroheme include the *cysG* gene coding for siroheme synthetase. The protein encoded by the *cysG* gene has an activity of catalyzing conversion of uroporphyrinogen III (Uro'gen III) as an intermediate into siroheme.

The proteins encoded by the *cysJ* gene, *cysI* gene and *cysG* gene are also referred to as CysJ protein, CysI protein and CysG protein, respectively.

The *cysJ* gene of the *Escherichia coli* K12 MG1655 strain corresponds to the complementary sequence of the sequence of positions 2888121 to 2889920 in the genome sequence registered at the NCBI database as GenBank accession NC_000913 (VERSION NC_000913.2 GI: 49175990). The *cysJ* gene of the *Escherichia coli* K12 MG1655 strain is synonymous with ECK2759 and JW2734. Further, the CysJ protein of the *Escherichia coli* K12 MG1655 strain is registered as GenBank accession NP_417244 (version NP_417244.1 GI: 16130671, locus_tag="b2764").

Further, the *cysI* gene of the *Escherichia coli* K12 MG1655 strain corresponds to the complementary sequence of the sequence of positions 2886409 to 2888121 of the genome sequence. The *cysI* gene of the *Escherichia coli* K12 MG1655 strain is synonymous with ECK2758 and JW2733. Further, the CysI protein of the *Escherichia coli* K12 MG1655 strain is registered as GenBank accession NP_417243 (version NP_417243.1 GI: 16130670, locus_tag="b2763").

Further, the *cysG* gene of the *Escherichia coli* K12 MG1655 strain corresponds to the sequence of positions 3495850 to 3497223 of the genome sequence. The *cysG* gene of the *Escherichia coli* K12 MG1655 strain is synonymous with ECK3356 and JW3331. Further, the CysG protein of the *Escherichia coli* K12 MG1655 strain is registered as GenBank accession NP_417827 (version NP_417827.1 GI: 16131246, locus_tag="b3368").

The nucleotide sequences of the *cysJ* gene, *cysI* gene, and *cysG* gene of the MG1655 strain are shown as SEQ ID NOS: 61, 65, and 69, respectively. Further, the amino acid sequences of the proteins encoded by these genes are shown as SEQ ID NOS: 62, 66, and 70, respectively. Further, the nucleotide sequences of the *cysJ* gene, *cysI* gene and *cysG* gene of the *Pantoea ananatis* SC17 strain are shown as SEQ ID NOS: 63, 67, and 71, respectively. Further, the amino acid sequences of the proteins encoded by these genes are shown as SEQ ID NOS: 64, 68, and 72, respectively.

Since the nucleotide sequence of the cysJ gene can differ depending on the genus, species or strain to which the bacterium belongs, the *cysJ* gene of which expression is increased can be a variant of the nucleotide sequence shown as SEQ ID NO: 61 or 63, so long as it codes for a protein having the sulfite-reducing activity. A variant of the *cysJ* gene can be searched for by using BLAST (http://blast.genome.jp/) or the like with referring to, for example, the nucleotide sequence of SEQ ID NO: 61 or 63. Further, the variant of the *cysJ* gene includes homologues of the gene, such as genes that can be amplified by PCR using a chromosome of such a microorganism as bacteria belonging to the family *Enterobacteriaceae* and coryneform bacteria as a template and synthetic oligonucleotides prepared on the basis of the nucleotide sequence of SEQ ID NO: 61 or 63.

The *cysJ* gene can be a gene coding for a protein having the amino acid sequence of the CysJ protein as mentioned above, such as the amino acid sequence shown as SEQ ID NO: 62 or 64, but including substitution, deletion, insertion, addition or the like of one or several amino acid residues at one or several positions, so long as it codes for a protein having the sulfite-reducing activity. Although the number meant by the term "one or several" can differ depending on positions in the three-dimensional structure of the protein or types of amino acid residues, specifically, it is preferably 1 to 20, more preferably 1 to 10, still more preferably 1 to 5. The above substitution, deletion, insertion, or addition of one or several amino acid residues is a conservative mutation that maintains normal function of the protein. The conservative mutation is typically a conservative substitution. The conservative substitution is a mutation wherein substitution takes place mutually among Phe, Trp and Tyr, if the substitution site is an aromatic amino acid; among Leu, Ile and Val, if it is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of conservative substitutions include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val. The above-mentioned amino acid substitution, deletion, insertion, addition, inversion etc. can be a result of a naturally-occurring mutation (mutant or variant) due to an individual difference, a difference of species, or the like of a bacterium from which the gene is derived.

Furthermore, the gene having such a conservative mutation as mentioned above can be a gene coding for a protein showing a homology of 80% or more, preferably 90% or more, more preferably 95% or more, still more preferably 97% or more, particularly preferably 99% or more, to the total amino acid sequence of the CysJ protein, such as the total sequence of the amino acid sequence of SEQ ID NO: 62 or 64, and having the sulfite-reducing activity. In this specification, "homology" can mean "identity".

Further, the *cysJ* gene can be a DNA that is able to hybridize with a probe that can be prepared from a known gene sequence, for example, a sequence complementary to a part or all of the nucleotide sequence of SEQ ID NO: 61 or 63, under stringent conditions, and coding for a protein having the sulfite-reducing activity. The "stringent conditions" refer to conditions under which a so-called specific hybrid is formed, and a non-specific hybrid is not formed. Examples of the stringent conditions include those under which highly homologous DNAs hybridize to each other, for example, DNAs not less than 80% homologous, preferably not less than 90% homologous, more preferably not less than 95% homologous, still more preferably not less than 97% homologous, particularly preferably not less than 99% homologous, hybridize to each other, and DNAs less homologous than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

The probe used for the aforementioned hybridization can be a sequence that is complementary to a part of the gene as described above. Such a probe can be prepared by PCR using oligonucleotides prepared on the basis of a known gene sequence as primers and a DNA fragment containing the nucleotide sequence as a template. For example, when a DNA fragment having a length of about 300 bp is used as the probe, the washing conditions of the hybridization can be, for example, 50°C, 2 x SSC and 0.1% SDS.

Similarly, since the nucleotide sequence of the *cysI* gene can differ depending on the genus, species or strain to which the bacterium belongs, the *cysI* gene of which expression is increased can be a variant of the nucleotide sequence of SEQ ID NO: 65 or 67, so long as it codes for a protein having the sulfite-reducing activity. The above explanations for variants of the *cysJ* gene and CysJ protein are similarly applied to the above variant of the *cysI* gene.

Similarly, since the nucleotide sequence of the *cysG* gene can differ depending on the genus, species or strain to which the bacterium belongs, the *cysG* gene of which expression is increased can be a variant of the nucleotide sequence of SEQ ID NO: 69 or 71, so long as it codes for a protein having the activity of catalyzing conversion of uroporphyrinogen III (Uro'gen III) into siroheme. The above explanation for variants of the *cysJ* gene and CysJ protein is similarly applied to the above variant of the *cysG* gene.

The aforementioned explanation of the variants of genes and proteins are also similarly applied to arbitrary proteins including enzymes such as serine acetyltransferase and 3-phosphoglycerate dehydrogenase and transporters such as YdeD protein, and the genes coding for them.

The expression "expression of a gene involved in sulfite reduction is increased" means that expression amount of the gene involved in sulfite reduction is increased compared with that of a non-modified strain such as a wild-type strain or a parent strain. Although degree of increase of expression amount of the gene involved in sulfite reduction is not particularly limited so long as it is increased compared with that of a non-modified strain, it is increased preferably 1.5 times or more, more preferably 2 times or more, still more preferably 3 times or more, compared with that of a non-modified strain. Further, the case where "expression of a gene involved in sulfite reduction is increased" includes not only a case where expression of a gene involved in sulfite reduction is increased in a strain in which the gene involved in sulfite reduction is natively expressed, but also a case where a gene involved in sulfite reduction is expressed in a strain in which the gene involved in sulfite reduction has not been natively expressed. That is, the case meant by the above expression includes, for example, a case where a gene involved in sulfite reduction is introduced into a strain not having the gene involved in sulfite reduction, and the gene involved in sulfite reduction is expressed in the strain. Moreover, so long as expression of a gene involved in sulfite reduction is increased as a result, expression of a gene involved in sulfite reduction and natively possessed by a bacterium can be attenuated or deleted, and then an appropriate sulfite reductase can be introduced.

The bacterium of the present invention is preferably modified so that expression of at least a gene coding for siroheme synthetase is increased. Further, the bacterium of the present invention is preferably modified so that expression of a gene coding for siroheme synthetase is increased, and expression of a gene coding for sulfite reductase is increased.

A modification for increasing expression of a gene involved in sulfite reduction can be attained by the methods for enhancing expression of a gene exemplified in the section of <Methods for increasing enzymatic activity> mentioned above.

In addition, as mentioned above, a modification for increasing expression of a gene can also be attained by amplifying a regulator that increases expression of an objective gene. Examples of the regulator that increases expression of a gene coding for a sulfite reductase include the protein (CysB) encoded by the *cysB* gene. It is known that CysB positively regulates expression of the *cysJIH* operon containing the *cysJ* gene and *cysI* gene coding for a sulfite reductase, and therefore expression of a gene involved in sulfite reduction can be enhanced by enhancing expression of the *cysB* gene.

Increase of expression of a gene can be confirmed by, for example, confirming the increase of the transcription amount of the gene by Northern hybridization or RT-PCR as described above, or by confirming the increase of the protein amount by Western blotting. Further, increase of expression of a gene involved in sulfite reduction can also be confirmed by measuring the activity of the protein encoded by the gene involved in sulfite reduction. The activity of sulfite reductase can be measured by a known method (Covbs J. et al., J. Biol. Chem., 268, 18604-18609 (1993)). Further, the activity of siroheme synthetase can also be measured by a known method (Spencer J.B. et al., FEBS Lett., 335(1):57-60, Nov. 29, 1993).

### <2> Method for producing L-cysteine, related substance thereof, or mixture thereof of the present invention

By culturing the bacterium of the present invention obtained as described above in a medium containing thiosulfate and collecting L-cysteine, a related substance thereof, or a mixture thereof from the medium, these compounds can be produced. Examples of the related substance of L-cysteine include L-cystine, S-sulfocysteine, thiazolidine derivatives, hemithioketals corresponding to the thiazolidine derivatives, L-methionine, S-adenosylmethionine, and so forth mentioned above.

As the medium, so long as thiosulfate is contained, ordinary media containing a carbon source, nitrogen source, sulfur source, inorganic ions, and other organic components as required can be used.

As the carbon source, saccharides such as glucose, fructose, sucrose, molasses and starch hydrolysate, and organic acids such as fumaric acid, citric acid and succinic acid can be used.

As the nitrogen source, inorganic ammonium salts such as ammonium sulfate, ammonium chloride and ammonium phosphate, organic nitrogen such as soybean hydrolysate, ammonia gas, aqueous ammonia and so forth can be used.

As the sulfur source, only thiosulfate can be contained in the medium, or another sulfur compound can be contained in the medium in addition to thiosulfate. Examples of the sulfur compound other than thiosulfate include inorganic sulfur compounds such as sulfates, sulfites, hyposulfites and sulfides. Although weight ratio of thiosulfate and the other sulfur compound contained in the medium is not particularly limited, for example, the weight ratio of thiosulfate:other sulfur source is usually 5:95 to 100:0, preferably 20:80 to 100:0, more preferably 50:50 to 100:0, particularly preferably 80:20 to 100:0.

The content of thiosulfate in the medium is, for example, usually 0.1 g/L or higher, preferably 0.3 g/L or higher. Although the maximum content of thiosulfate is not particularly limited, it can be, for example, 100 g/L or lower, or 10 g/L or lower. Thiosulfate can be a free acid or thiosulfate salt, or can be an arbitrary mixture of them. Type of thiosulfate salt is not particularly limited, and examples include sodium salt, calcium salt, ammonium salt, and so forth.

Thiosulfate can be contained in the medium over the entire period of the culture, or contained in the medium over only a part of the culture period. For example, if the method of the present invention comprises the stage of proliferating the L-cysteine-producing bacterium, and the stage of producing and accumulating L-cysteine, it is sufficient that thiosulfate is contained in the medium during at least the stage of producing and accumulating L-cysteine, and thiosulfate may be or may not be contained in the medium during the stage of proliferating the L-cysteine-producing bacterium. Further, thiosulfate can be contained in the medium over the entire period or a part of the period of the stage of producing and accumulating L-cysteine. For example, the content of thiosulfate does not need to be within the aforementioned range over the entire period of the stage of producing and accumulating L-cysteine, that is, thiosulfate can be contained in the medium at a content within the aforementioned range in an early period of that stage, and the thiosulfate content can be decreased with lapse of culture time. Further, thiosulfate can be supplementarily added continuously or intermittently. In addition, the contents of medium components other than thiosulfate can also vary during the culture period, and they can be supplementarily added during the culture period.

As organic trace amount nutrients, it is desirable to add required substances such as vitamin B₁, yeast extract and so forth in appropriate amounts. Other than these, potassium phosphate, magnesium sulfate, iron ions, manganese ions and so forth are added in small amounts, as required.

The culture is preferably performed under aerobic conditions for 30 to 90 hours. The culture temperature is preferably controlled to be at 25°C to 37°C, and pH is preferably controlled to be 5 to 8 during the culture. For pH adjustment, inorganic or organic acidic or alkaline substances, ammonia gas and so forth can be used.

L-cysteine, a related substance thereof, or a mixture thereof can be collected from culture broth by a combination of ion-exchange resin method (Nagai, H. et al., Separation Science and Technology, 39(16), 3691-3710), membrane separation method (Japanese Patent Laid-open (Kokai) Nos. 9-164323 and 9-173792), crystallization method (WO2008/078448, WO2008/078646) and other conventionally known methods.

L-cysteine, a related substance thereof, or a mixture thereof collected according to the present invention can contain microbial cells, medium components, moisture, metabolic by-products of microbes, and so forth in addition to the objective compounds.

L-cysteine obtained as described above can be used for production of L-cysteine derivatives. The L-cysteine derivatives include methylcysteine, ethylcysteine, carbocisteine, sulfocysteine, acetylcysteine, and so forth.

Further, when a thiazolidine derivative of L-cysteine is accumulated in the medium, L-cysteine can be produced by collecting the thiazolidine derivative from the medium and breaking the reaction equilibrium between the thiazolidine derivative and L-cysteine so that L-cysteine is excessively produced. Further, when S-sulfocysteine is accumulated in the medium, it can be converted into L-cysteine by reduction using a reducing agent such as dithiothreitol.

### Examples

Hereafter, the present invention will be explained more specifically with reference to an example. In the following example, cysteine means L-cysteine.

### Example 1: Production of cysteine by E. coli overexpressing cysG gene or cysGJI genes using thiosulfate as sulfur source

### (1) Construction of expression plasmids for genes involved in sulfite reduction

As genes involved in sulfite reduction, the *cysG* gene of *P. ananatis* SC17 strain, the *cysGJI* genes of *P. ananatis* SC17 strain, the *cysG* gene of *E. coli* MG1655 strain, and the *cysGJI* genes of *E. coli* MG1655 strain were used. These genes were each cloned in an expression vector to construct expression plasmids of the genes. The procedure is described in (1-1) to (1-7).

### (1-1) Construction of expression vectors

As the expression vector, pMIV-Pnlp23-ter constructed from pMIV-5JS (Japanese Patent Laid-open (Kokai) No. 2008-99668) was used. This vector has the nlp23 promoter (Pnlp23), which is a strong promoter, and the rrnB terminator, and has *Sal*I and *XbaI* sites between Pnlp23 and the rrnB terminator. By amplifying an objective gene using primers in which *Sal*I (or *Xho*I*)* and *Xba*I sites are designed, and inserting the amplified gene into the vector at a position between Pnlp23 and the rrnB terminator, an expression plasmid of the objective gene can be constructed. "Pnlp23" is a mutant promoter constructed from "Pnlp0", which is a promoter of the wild-type *nlpD* gene derived from the *E. coli* K12 strain, in order to control expression intensity.

Further, as the expression vector, pMIV-Pnlp8-YeaS7 constructed from pMIV-5JS (Japanese Patent Laid-open (Kokai) No. 2008-99668) was also used. This vector has the nlp8 promoter (Pnlp8), which is a strong promoter, and the rrnB terminator, and further has the *yeaS* gene of the *E*. *coli* K12 strain cloned at a position between Pnlp8 and the rrnB terminator using *Sai*I and *Xba*I sites. By amplifying an objective gene using primers in which *Sal*I (or *Xho*I) and *Xba*I sites are designed, and inserting the amplified gene into the vector, an expression plasmid in which the *yeaS* gene fragment of the vector is replaced with the objective gene fragment can be constructed. "Pnlp8" is a mutant promoter constructed from "Pnlp0", which is a promoter of the wild-type *nlpD* gene of the *E. coli* K12 strain, in order to control expression intensity.

The details of the construction of these expression vectors are described below.

First, by PCR using the chromosomal DNA of the *E*. *coli* MG1655 strain as the template as well as primer P1 (agctgagtcgacccccagga aaaattggttaataac, SEQ ID NO: 13) and primer P2 (agctgagcatgcttccaactgcgctaatgacgc, SEQ ID NO: 14) as primers, a DNA fragment containing a promoter region of the *nlpD* gene (henceforth wild-type *nlpD* gene promoter is referred to as "Pnlp0") of about 300 bp was obtained. At the 5' ends of the aforementioned primers, sites for the restriction enzymes *Sal*I and *Pae*I were designed, respectively. The PCR cycle was as follows: 95°C for 3 minutes, following 2 cycles of 95°C for 60 seconds, 50°C for 30 seconds, and 72°C for 40 seconds, 25 cycles of 94°C for 20 seconds, 55°C for 20 seconds, and 72°C for 15 seconds, and 72°C for 5 minutes as the final cycle. The obtained fragment was treated with *Sal*I and *Pae*I, and inserted into pMIV-5JS (Japanese Patent Laid-open (Kokai) No. 2008-99668) at the *Sal*I*-Pae*I site to obtain a plasmid pMIV-Pnlp0. The nucleotide sequence of the *Pae*I*-Sal*I fragment containing the Pnlp0 promoter inserted into this pMIV-Pnlp0 plasmid was as shown in SEQ ID NO: 1.

Then, by PCR using the chromosomal DNA of the MG1655 strain as the template, as well as primer P3 (agctgatcta gaaaacagaa tttgcctggc ggc, SEQ ID NO: 15) and primer P4 (agctgaggat ccaggaagag tttgtagaaa cgc, SEQ ID NO: 16) as primers, a DNA fragment containing a terminator region of the *rrnB* gene of about 300 bp was obtained. At the 5' ends of the aforementioned primers, sites for the restriction enzymes *Xba*I and *Bam*HI were designed, respectively. The PCR cycle was as follows: 95°C for 3 minutes, following 2 cycles of 95°C for 60 seconds, 50°C for 30 seconds, and 72°C for 40 seconds, 25 cycles of 94°C for 20 seconds, 59°C for 20 seconds, and 72°C for 15 seconds, and 72°C for 5 minutes as the final cycle. The obtained fragment was treated with *XbaI* and *Bam*HI*,* and inserted into pMIV-Pnlp0 at the *Xba*I*-Bam*HI site to obtain a plasmid pMIV-Pnlp0-ter.

Then, by PCR using the chromosomal DNA of the MG1655 strain as the template, as well as primer P5 (agctgagtcg acgtgttcgc tgaatacggg gt, SEQ ID NO: 17) and primer P6 (agctgatcta gagaaagcat caggattgca gc, SEQ ID NO: 18) as primers, a DNA fragment of about 700 bp containing the *yeaS* gene was obtained. At the 5' ends of the aforementioned primers, sites for the restriction enzymes *Sal*I and *Xba*I were designed, respectively. The PCR cycle was as follows: 95°C for 3 minutes, following 2 cycles of 95°C for 60 seconds, 50°C for 30 seconds, and 72°C for 40 seconds, 25 cycles of 94°C for 20 seconds, 55°C for 20 seconds, and 72°C for 15 seconds, and 72°C for 5 minutes as the final cycle. The obtained fragment was treated with *Sal*I and *Xba*I, and inserted into pMIV-PnlpO-ter at the *Sal*I*-Xba*I site to obtain a plasmid pMIV-Pnlp0-YeaS3. As described above, a *yeaS* expression unit comprising the *nlpD* promoter, the *yeaS* gene, and the *rrnB* terminator ligated in this order was constructed on the pMIV-5JS vector.

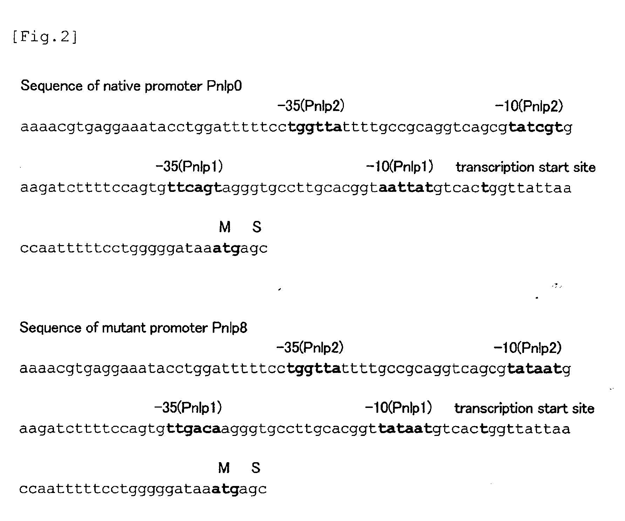

In order to modify the -10 region of the *nlpD* promoter to make it a stronger promoter, the -10 region was randomized by the following method. The *nlpD* promoter region contains two of regions presumed to function as promoters (Fig. 2), and they are indicated as pnlp1 and pnlp2, respectively, in the drawing. By PCR using the plasmid pMIV-Pnlp0 as the template as well as the primer P1 and primer P7 (atcgtgaaga tcttttccag tgttnannag ggtgccttgc acggtnatna ngtcactgg ("n" means that the corresponding residue can be any of a, t, g and c), SEQ ID NO: 19) as primers, a DNA fragment in which the -10 region contained in the 3' end side of the *nlpD* promoter (-10(Pnlp1), Fig.

2) was randomized was obtained. The PCR cycle was as follows: 95°C for 3 minutes, following 2 cycles of 95°C for 60 seconds, 50°C for 30 seconds, and 72°C for 40 seconds, 25 cycles of 94°C for 20 seconds, 60°C for 20 seconds, and 72°C for 15 seconds, and 72°C for 5 minutes as the final cycle.

Similarly, by PCR using the plasmid pMIV-Pnlp0 as the template as well as the primer P2 and primer P8 (tggaaaagat cttcannnnn cgctgacctg cg ("n" means that the corresponding residue can be any of a, t, g and c), SEQ ID NO: 20) as primers, a DNA fragment in which the -10 region contained in the 5' end side of the *nlpD* promoter (-10(Pnlp2), Fig. 2) was randomized was obtained. The PCR cycle was as follows: 95°C for 3 minutes, following 2 cycles of 95°C for 60 seconds, 50°C for 30 seconds, and 72°C for 40 seconds, 25 cycles of 94°C for 20 seconds, 60°C for 20 seconds, and 72°C for 15 seconds, and 72°C for 5 minutes as the final cycle.

The obtained 3' and 5' end side fragments were ligated by using the *Bgl*II sites designed in the primers P7 and P8 to construct a fragment containing the full length of the *nlpD* promoter in which two -10 regions were randomized. By PCR using this fragment as the template as well as the primers P1 and P2 as primers, a DNA fragment containing the full length of a mutant type *nlpD* promoter was obtained. The PCR cycle was as follows: 95°C for 3 minutes, following 2 cycles of 95°C for 60 seconds, 50°C for 30 seconds, and 72°C for 40 seconds, 12 cycles of 94°C for 20 seconds, 60°C for 20 seconds, and 72°C for 15 seconds, and 72°C for 5 minutes as the final cycle.

The obtained DNA fragment containing the mutant Pnlp was treated with the restriction enzymes *Sal*I and *Pae*I, for which sites were designed in the 5' ends of the primers, and inserted into the plasmid pMIV-Pnlp0-YeaS3 similarly treated with *Sal*I and *Pae*I to substitute the mutant Pnlp for the wild-type *nlpD* promoter region (Pnlp0) on the plasmid. From the resultants, one having the promoter sequence (Pnlp8) shown in Fig. 2 was selected, and designated pMIV-Pnlp8-YeaS7. The nucleotide sequence of the *Pae*I-*Sal*I fragment of the Pnlp8 promoter inserted into this plasmid was as shown in SEQ ID NO: 21.

In the same manner, a DNA fragment containing the mutant Pnlp was inserted into the plasmid pMIV-Pnlp0-ter treated with *Sai*I and *Pae*I to substitute the mutant Pnlp for the *nlpD* promoter region (region of Pnlp0) on the plasmid. One of the resultants was designated pMIV-Pnlp23-ter. The nucleotide sequence of the *Pae*I-*Sal*I fragment of the Pnlp23 promoter inserted into this plasmid was as shown in SEQ ID NO: 2.

### (1-2) Construction of overexpression plasmid for cysG gene of E. coli MG1655 strain

By PCR using the chromosomal DNA of the *E*. *coli* MG1655 strain (ATCC No. 47076) as the template, as well as primer SalI-cysG (Ec) Fw (ACGCGTCGACATGGATCATTTGCCTATATT, SEQ ID NO: 8) and primer XbaI-cysG (Ec) Rv (GCTCTAGATTAATGGTTGGAGAACCAGTTC, SEQ ID NO: 9) as primers, the *cysG* gene fragment was amplified. At the 5' ends of these primers, sites for the restriction enzymes *Sal*I and *Xba*I were designed. PCR was performed by using PrimeSTAR Polymerase (TaKaRa) and a standard reaction mixture composition described in the attached protocol with a PCR cycle consisting of 94°C for 5 minutes, following 30 cycles of 98°C for 5 seconds, 55°C for 10 seconds, and 72°C for 2 minutes, and keeping at 4°C as the final cycle. The obtained fragment was treated with *Sal*I and *Xba*I, and inserted into pMIV-Pnlp23-ter treated with the same restriction enzymes to obtain a plasmid pMIV-Pnlp23-cysG (Ec) in which the *cysG* gene of the *E. coli* MG1655 strain was cloned.

### (1-3) Cloning of cysJI genes of E. coli MG1655 strain

By PCR using the chromosomal DNA of the *E. coli* MG1655 strain as the template as well as primer SalI-cysJ(Ec) Fw (ACGCGTCGACATGACGACACAGGTCCCACC, SEQ ID NO: 10) and primer XbaI-cysI (Ec) Rv (GCTCTAGATTAATCCCACAAATCACGCGCC, SEQ ID NO: 11), the *cysJI* gene fragment was amplified. At the 5' ends of these primers, sites for the restriction enzymes *Sal*I and *Xba*I were designed. PCR was performed by using PrimeSTAR Polymerase (TaKaRa) and a standard reaction mixture composition described in the attached protocol with a PCR cycle consisting of 94°C for 5 minutes, following 30 cycles of 98°C for 5 seconds, 55°C for 10 seconds, and 72°C for 2 minutes, and keeping at 4°C as the final cycle. The obtained fragment was treated with *Sal*I and *Xba*I, and inserted into pMIV-Pnlp23-ter treated with the same restriction enzymes to obtain a plasmid pMIV-Pnlp23-cysJI (Ec) in which the *cysJI* genes of the *E. coli* MG1655 strain were cloned.

### (1-4) Construction of coexpression plasmid for cysG gene and cysJI genes of E. coli MG1655 strain

PCR was performed by using the chromosomal DNA of the *E. coli* MG1655 strain as the template as well as the primer SalI-cysG (Ec) Fw (SEQ ID NO: 8) and primer cysG-JI (Ec) Rv (CAACGCGGAAGGTGGGACCTGTGTCGTCATGCGTCGTTATGTTCCAGTTTAATGGTTG GAGAACCAGTTCAGTTTATC, SEQ ID NO: 12) as primers. PCR was performed by using PrimeSTAR Polymerase (TaKaRa) and a standard reaction mixture composition described in the attached protocol with a PCR cycle consisting of 94°C for 5 minutes, following 30 cycles of 98°C for 5 seconds, 55°C for 10 seconds, and 72°C for 2 minutes, and keeping at 4°C as the final cycle. By this PCR, a gene fragment comprising the *cysG* gene and a sequence of 18 bp containing the start codon of the *cysJ* gene added downstream of the *cysG* gene was obtained. The salts, polymerase, and primers were removed from the reaction mixture containing the obtained fragment, and the resultant was used as primer of the following PCR.

PCR was performed by using the plasmid DNA of pMIV-Pnlp23-cysJI (Ec) constructed in (1-3) as the template, as well as the aforementioned gene fragment and the primer XbaI-cysI(Ec) Rv (SEQ ID NO: 11) as primers. PCR was performed by using PrimeSTAR GXL Polymerase (TaKaRa) and a standard reaction mixture composition described in the attached protocol with a PCR cycle consisting of 98°C for 10 seconds, following 30 cycles of 98°C for 10 seconds, 58°C for 15 seconds, and 68°C for 2 minutes, and keeping at 4°C as the final cycle. By this PCR, a gene fragment comprising the *cysG* gene sequence and the cysJI gene sequence ligated downstream of the *cysG* gene sequence was obtained. At the 5' ends of the primers, *Sal*I and *Xba*I sites were designed, respectively. The obtained fragment was treated with *Sal*I and *Xba*I, and inserted into pMIV-Pnlp23-ter treated with the same restriction enzymes to obtain a plasmid pMIV-Pnlp23-cysGJI (Ec) in which the *cysGJI* genes of the *E. coli* MG1655 strain were cloned.

### (1-5) Construction of overexpression plasmid for cysG gene of P. ananatis SC17 strain

By PCR using the chromosomal DNA of the *P*. *ananatis* SC17 strain (FERM BP-11091) as the template, as well as primer CysG Fw (XhoI) (ACGCCTCGAGATGGATTATTTGCCTCTTTT, SEQ ID NO: 3) and primer CysG Rv (XbaI) (GCTCTAGATCAAGCCAGATTGACAACGG, SEQ ID NO: 4) as primers, the *cysG* gene fragment was amplified. PCR was performed by using PrimeSTAR Polymerase (TaKaRa) and a standard reaction mixture composition described in the attached protocol with a PCR cycle consisting of 94°C for 5 minutes, following 30 cycles of 98°C for 5 seconds, 55°C for 10 seconds, and 72°C for 2 minutes, and keeping at 4°C as the final cycle. Although the start codon of the *cysG* gene on the chromosomal DNA of *P. ananatis* was GTG, the start codon of the *cysG* gene contained in the DNA fragment obtained by PCR was modified to ATG by changing GTG in the sequence of the start codon attaching site of the primer to ATG. Further, at the 5' ends of the aforementioned primers, *Xho*I and *Xba*I sites were designed, respectively. Because a restriction enzyme site for *Sal*I existed in the ORF of the *cysG* gene of *P. ananatis, Xho*I was used as a substitute for *Sal*I. The obtained fragment was treated with *Xho*I and *Xba*I, and inserted into pMIV-Pnlp8-YeaS7 treated with *Sal*I and *Xba*I to obtain a plasmid pMIV-Pnlp8-cysG (Pa) in which the *cysG* gene of the *P. ananatis* SC17 strain was cloned. pMIV-Pnlp8-cysG (Pa) has a structure that the *yeaS* gene fragment on pMIV-Pnlp8-YeaS7 was replaced with the *cysG* gene fragment of the *P. ananatis* SC17 strain.

Similarly, The obtained fragment was treated with *Xho*I and *Xba*I, and inserted into pMIV-Pnlp23-ter treated with *Sal*I and *XbaI* to obtain a plasmid pMIV-Pnlp23-cysG (Pa) in which the *cysG* gene of the *P. ananatis* SC17 strain was cloned.

### (1-6) Cloning of cysJI genes of P. ananatis SC17 strain

By PCR using the chromosomal DNA of the *P*. *ananatis* SC17 strain as the template, as well as primer CysJ Fw (SalI) (ACGCGTCGACATGACGACTCAGGCACCAGG, SEQ ID NO: 5) and primer CysI Rv (XbaI) (GCTCTAGATCATTTTGCCTCCTGCCAGA, SEQ ID NO: 6) as primers, the *cysJI* gene fragment was amplified. At the 5' ends of the aforementioned primers, sites for the restriction enzymes *Sal*I and *Xba*I were designed, respectively. PCR was performed by using PrimeSTAR Polymerase (TaKaRa) and a standard reaction mixture composition described in the attached protocol with a PCR cycle consisting of 94°C for 5 minutes, following 30 cycles of 98°C for 5 seconds, 55°C for 10 seconds, and 72°C for 2 minutes, and keeping at 4°C as the final cycle. The obtained fragment was treated with *Sal*I and *Xba*I, and inserted into pMIV-Pnlp23-ter treated with the same restriction enzymes to obtain a plasmid pMIV-Pnlp23-cysJI (Pa) in which the *cysJI* genes of the *P. ananatis* SC17 strain were cloned.

### (1-7) Construction of coexpression plasmid for cysG gene and cysJI genes of P. ananatis SC17 strain

PCR was performed by using the chromosomal DNA of the *P*. *ananatis* SC17 strain as the template as well as the primer CysG Fw (XhoI) (SEQ ID NO: 3) and primer CysG-JI Rv (CTGGTGCCTGAGTCGTCATCGTTTTTCCTCTCAAGCCAGATTGACAACGGCGGACTCG CG, SEQ ID NO: 7) as primers. PCR was performed by using PrimeSTAR Polymerase (TaKaRa) and a standard reaction mixture composition described in the attached protocol with a PCR cycle consisting of 94°C for 5 minutes, following 30 cycles of 98°C for 5 seconds, 55°C for 10 seconds, and 72°C for 2 minutes, and keeping at 4°C as the final cycle. By this PCR, a gene fragment comprising the *cysG* gene and a sequence of 11 bp containing the start codon of the *cysJ* gene added downstream of the *cysG* gene was obtained. The salts, polymerase, and primers were removed from the reaction mixture containing the obtained fragment, and the resultant was used as primer for the following PCR.

PCR was performed by using the plasmid DNA of pMIV-Pnlp23-cysJI (Pa) constructed in (1-6) as the template, as well as the aforementioned gene fragment and the primer CysI Rv (XbaI) (SEQ ID NO: 6) as primers. PCR was performed by using PrimeSTAR GXL Polymerase (TaKaRa) and a standard reaction mixture composition described in the attached protocol with a PCR cycle consisting of 98°C for 10 seconds, following 30 cycles of 98°C for 10 seconds, 58°C for 15 seconds, and 68°C for 2 minutes, and keeping at 4°C as the final cycle. By this PCR, a gene fragment comprising the *cysG* gene sequence and the *cysJI* gene sequence ligated downstream of the *cysG* gene sequence was obtained. Although the start codon of the *cysG* gene on the chromosomal DNA of *P. ananatis* was GTG, the start codon of the *cysG* gene contained in the DNA fragment obtained by PCR was modified to ATG by changing GTG in the sequence of the start codon attaching site of the primer to ATG. Further, at the 5' ends of the primers, *Xho*I and *Xba*I sites were designed, respectively. Since a restriction enzyme site for *Sal*I existed in the ORF of the *cysG* gene of *P. ananatis, Xho*I was used as a substitute for *Sal*I*.* The obtained fragment was treated with *Xho*I and *XbaI,* and inserted into pMIV-Pnlp23-ter treated with *Sal*I and *Xba*I to obtain a plasmid pMIV-Pnlp23-cysGJI (Pa) in which the *cysGJI* genes of the *P. ananatis* SC17 strain were cloned.

### (2) Construction of cysteine-producing E. coli bacterium

As a cysteine-producing *E. coli* bacterium, there was constructed the MG1655int-4M/pACYC-DES strain harboring a plasmid pACYC-DES having the *ydeD* gene, *cysEX* gene and *serA5* gene, in which strain the *cysM* gene was introduced into the chromosome. The construction procedure will be described in (2-1) and (2-2).

### (2-1) Construction of plasmid pACYC-DES having ydeD gene, cysEX gene and serA5 gene

The *ydeD* gene, *cysEX* gene and *serA5* gene were cloned into the plasmid pACYC184 to construct the plasmid pACYC-DES. The *ydeD* gene is a gene coding for a transmembrane protein involved in secretion of metabolic products of the cysteine pathway, and it has been reported that it is useful for the cysteine production (U.S. Patent No. 5,972,663). The *cysEX* gene is a mutant gene of *cysE* and codes for a mutant serine acetyltransferase (SAT) desensitized to feedback inhibition by cysteine (U.S. Patent No. 6,218,168). The *serA*5 gene is a mutant gene of *serA* and codes for a mutant 3-phosphoglycerate dehydrogenase (PGD) deficient in the tyrosine residue at the C-terminus of the wild-type 3-phosphoglycerate dehydrogenase of *Escherichia coli* and desensitized to feedback inhibition by L-serine (U.S. Patent No. 6,180,373). The construction procedure is shown below.

By PCR using the chromosomal DNA of the *E. coli* MG1655 strain as the template, as well as primer ydeD299F (agctgagtcg acatgtcgcg aaaagatggg gtg, SEQ ID NO: 22) and primer ydeD299R (agctgatcta gagtttgttc tggccccgac atc, SEQ ID NO: 23) as primers, the *ydeD* gene fragment of the *Escherichia coli* MG1655 strain was amplified.

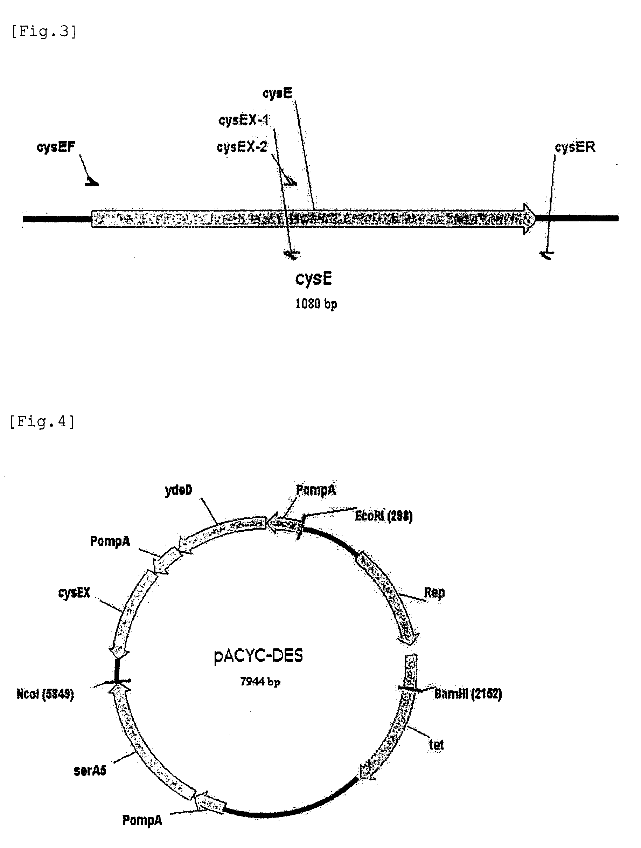

Furthermore, as shown in Fig. 3, the *cysEX* gene fragment was prepared. Specifically, first, by PCR using the chromosomal DNA of the *E. coli* MG1655 strain as the template, as well as primer cysEF (agctgagtcg acatgtcgtg tgaagaactg gaa, SEQ ID NO: 24) and primer cysEX-1 (atcaccgccg cttcaccaac g, SEQ ID NO: 25) as primers, an upstream side fragment of the *cysEX* gene was amplified. Then, by PCR using the chromosomal DNA of the *E. coli* MG1655 strain as the template, as well as primer cysEX-2 (cgttggtgaa gcggcggtga t, SEQ ID NO: 26) and primer cysER (agctgatcta gaatagatga ttacatcgca tcc, SEQ ID NO: 27) as primers, a downstream side fragment of the *cysEX* gene was amplified. These two kinds of PCR products were separated by electrophoresis and eluted from the gel. Subsequently, by performing PCR again using these two kinds of PCR products, these two kinds of PCR products were annealed at the overlapping portions to generate a DNA fragment containing the full length of the *cysEX* gene.

Further, by PCR using the chromosomal DNA of the *E*. *coli* MG1655 strain as the template, as well as primer serA5F (agctgagtcg acatggcaaa ggtatcgctg gag, SEQ ID NO: 28) and primer serA5R (agctgatcta gattacagca gacgggcgcg aatgg, SEQ ID NO: 29) as primers, a *serA5* gene fragment was amplified.

Furthermore, by PCR using the chromosomal DNA of the *E. coli* MG1655 strain as the template, as well as primer PrOMPAF (agctgagtcg accgcctcgt tatcatccaa aatc, SEQ ID NO: 30) and primer PrOMPAR (agctgagcat gcactaattt tccttgcgga ggc, SEQ ID NO: 31) as primers, a promoter PompA fragment was amplified.

At the 5' ends of the primer PrOMPAF, the primers ydeD299F, cysEF and serA5F for gene fragment amplification, *Sal*I sites were designed. The amplified PompA fragment and each amplified gene fragment were treated with *Sal*I and ligated to obtain three kinds of DNA fragments comprising PompA and each gene ligated downstream of PompA.

Further, at the 5' end of the primer PrOMPAR, a *Pae*I site was designed. At the 5' ends of the primers ydeD299R, cysER and serA5R for gene fragment amplification, restriction enzyme sites for incorporation into a vector were designed. By using these restriction enzyme sites, the three kinds of DNA fragments comprising PompA and each gene ligated downstream of PompA were introduced into the plasmid pACYC184. A plasmid obtained as described above was designated pACYC-DES. The structure of pACYC-DES is shown in Fig. 4.

### (2-2) Introduction of O-acetyl-L-serine sulfhydrylase B gene (cysM) into E. coli MG1655 strain

The O-acetyl-L-serine sulfhydrylase B gene (*cysM*) was introduced into the chromosome of the *E. coli* MG1655 strain to construct an MG1655int-4M strain. Furthermore, the plasmid pACYC-DES was introduced into the MG1655int-4M strain to construct an MG1655int-4M/pACYC-DES strain. For expression of the *cysM* gene introduced into the *E*. *coli* MG1655 strain, the promoter of the wild-type *nlpD* gene of the *P*. *ananatis* SC17 strain, "Pnlp4", was used. The construction procedure is explained below.

First, in order to express the *cysM* gene by using a promoter of an appropriate strength, a novel promoter Pnlp4 was obtained by the following procedure.

First, a DNA fragment containing about 180 bp of the promoter region of the *nlpD* gene was obtained by PCR using the genome of the *P*. *ananatis* SC17 strain as the template. The promoter of the wild-type *nlpD* gene of the *P*. *ananatis* SC17 strain amplified as described above is referred to as "Pnlp4". The primers used were primer P9 (agctgaaagc ttgcatgcac gcgtggcgat ctggcctgac tgc, SEQ ID NO: 32) and primer P10 (agctgagtcg accccgtggt ggcaaccttt aaaaaactg, SEQ ID NO: 33), and at the 5' ends of these primers, sites for the restriction enzymes *Sal*I and *Pae*I were designed, respectively. The PCR cycle was as follows: 95°C for 5 minutes, following 27 cycles of 94°C for 20 seconds, 59°C for 20 seconds, and 72°C for 20 seconds, and 72°C for 5 minutes as the final cycle. The obtained DNA fragment was treated with *Sal*I and *Pae*I, and inserted into pMIV-Pnlp0-ter similarly treated with *Sal*I and *Pae*I to obtain a plasmid pMIV-Pnlp4-ter, which corresponded to pMIV-Pnlp0-ter of which Pnlp0 was replaced with Pnlp4. The nucleotide sequence of the *Pae*I*-Sal*I fragment of Pnlp4 inserted into pMIV-Pnlp4-ter was as shown in SEQ ID NO: 34.

The *cysM* gene cloned from the *E. coli* MG1655 strain was incorporated into the plasmid pMIV-Pnlp4-ter obtained in (2-2). Specifically, by PCR using the genome of the *E*. *coli* MG1655 strain as the template, as well as primer P11 (agctgagtcg acgtgagtacattagaacaa acaat, SEQ ID NO: 37) and primer P12 (agctgatcta gaagtctccg atgctattaa tcc, SEQ ID NO: 38) as primers (98°C for 5 minutes, following 30 cycles of 98°C for 5 seconds, 50°C for 10 seconds, and 72°C for 60 seconds, and 72°C for 2 minutes as the final cycle), the *cysM* gene fragment was amplified. The DNA fragment obtained as described above was treated with *Sal*I and *Xba*I, and inserted into pMIV-Pnlp4-ter treated with the same enzymes to prepare a plasmid pMIV-Pnlp4-CysM, in which the *cysM* gene of the *E. coli* MG1655 strain was cloned. The nucleotide sequence of the *cysM* gene of the *E. coli* MG1655 strain is shown as SEQ ID NO: 35, and the amino acid sequence encoded by this gene is shown as SEQ ID NO: 36.

pMIV-Pnlp4-CysM has the attachment sites for Mu phage originated from pMIV-5JS. Therefore, by allowing pMIV-Pnlp4-CysM to coexist with the helper plasmid pMH10 having the Mu transposase gene (Zimenkov D. et al., Biotechnologiya (in Russian), 6, 1-22 (2004)) in a host cell, the cassette of Pnlp4-CysM-rrnB terminator containing the chloramphenicol resistance gene existing on pMIV-Pnlp4-CysM between the attachment sites of Mu phage can be inserted into the chromosome of the host. Furthermore, the chloramphenicol resistance gene carried by the pMIV-Pnlp4-CysM plasmid exists between two attachment sites of λ phage (λattR and λattL), and can be excised and removed by the method described later.

First, the helper plasmid pMH10 was introduced into the MG1655 strain by electroporation, and the strain was cultured overnight at 30°C on the LB agar medium containing 20 mg/L of kanamycin to select a strain into which pMH10 was introduced. The obtained transformant was cultured at 30°C, and pMIV-Pnlp4-CysM was further introduced into this strain by electroporation. This strain transformed with both pMH10 and pMIV-Pnlp4-CysM was subjected to a heat shock with the conditions of 42°C for 20 minutes, and then cultured at 39°C on the LB agar medium containing 20 mg/L of chloramphenicol to select colonies of chloramphenicol resistant strains. About 50 clones of the chloramphenicol resistant strains obtained as described above were each cultured at 39°C for 48 hours on the LB agar medium to eliminate pMH10 and pMIV-Pnlp4-CysM. Then, there was obtained a strain showing chloramphenicol resistance as a result of the insertion of the cassette containing the chloramphenicol resistance gene into the chromosome and showing kanamycin and ampicillin sensitivities as a result of the elimination of the both plasmids. Furthermore, it was confirmed that the objective cassette was inserted into the chromosome of the obtained strain by PCR using the chromosomal DNA of this strain as the template as well as the primers P1 and P6 as primers. The strain prepared as described above was designated MG1655int-4M (CmR).

The chloramphenicol resistance gene introduced into the MG1655int-4M (CmR) strain was removed with an excision system derived from λ phage. Specifically, the MG1655int-4M (CmR) strain was transformed with pMT-Int-Xis2 (WO2005/010175 carrying the *Int-Xis* gene of λ phage, and the MG1655int-4M strain showing chloramphenicol sensitivity was obtained from the obtained transformants.

Then, the plasmid pACYC-DES was introduced into MG1655int-4M to obtain the MG1655int-4M/pACYC-DES strain.

### (3) Cysteine production by E. coli overexpressing cysG gene or cysGJI genes using thiosulfate as sulfur source

There were constructed strains of the above-obtained *E. coli* cysteine-producing bacterium, MG1655int-4M/pACYC-DES, into which the *cysG* overexpression plasmids pMIV-Pnlp23-cysG (Ec) and pMIV-Pnlp8-*cysG* (Pa), the *cysG* and *cysJI* coexpression plasmids pMIV-Pnlp23-cysGJI (Ec) and pMIV-Pnlp23-cysGJI (Pa), and pMIV-5JS as a control were each introduced. Cysteine production culture was performed with these strains, and cysteine production amounts were compared. For the cysteine production culture, a cysteine production medium having the following composition containing glucose as a carbon source and thiosulfate as a sulfur source was used.

### [Cysteine production medium] (concentrations of the components are final concentrations)

**Components 1:**

| | |
|---|---|
| NH₄Cl | 12.1 g/L |
| KH₂PO₄ | 1.5 g/L |
| MgSO₄·7H₂O | 1 g/L |
| Thiamine hydrochloride | 0.1 mg/L |

**Components 2:**

| | |
|---|---|
| FeSO₄·7H₂O | 1.7 mg/L |
| Na₂MoO₄·2H₂O | 0.15 mg/L |
| CoCl₂·6H₂O | 0.7 mg/L |
| MnCl·4H₂O | 1.6 mg/L |
| ZnSO₄·7H₂O | 0.3 mg/L |
| CuSO₄·5H₂O | 0.25 mg/L |

**Components 3:**

| | |
|---|---|
| Tryptone | 0.6 g/L |
| Yeast extract | 0.3 g/L |
| NaCl | 0.6 g/L |

**Component 4:**

| | |
|---|---|
| Calcium carbonate | 20 g/L |

**Component 5:**

| | |
|---|---|
| L-Histidine hydrochloride monohydrate | 135 mg/L |

**Component 6:**

| | |
|---|---|
| Sodium thiosulfate | 0.6 g/L |

**Component 7:**

| | |
|---|---|
| Pyridoxine hydrochloride | 2 mg/L |

**Component 8:**

| | |
|---|---|
| Glucose | 20 g/L |

For these components, there were prepared stock solutions of 10-fold concentration (Components 1), 1000-fold concentration (Components 2), 100/6-fold concentration (Components 3), 100-fold concentration (Component 5), 1750/3-fold concentration (Component 6), 1000-fold concentration (Component 7), and 20-fold concentration (Component 8), they were mixed at the time of use, and the defined volume was obtained with sterilized water to attain the final concentrations. Sterilization was performed by autoclaving at 110°C for 30 minutes (Components 1, 2, 3, 5 and 8), dry heat sterilization at 180°C for 5 hours or longer (Component 4), or filter sterilization (Components 6 and 7). The L-cysteine production culture was performed as follows. Each L-cysteine-producing strain was spread on the LB agar medium to perform pre-culture overnight at 34°C, and then cells corresponding to about 7 cm on the plate were scraped with an inoculation loop of 10-µl size (NUNC Blue Loop), and inoculated into 2 ml of the L-cysteine production medium contained in a large test tube (internal diameter: 23 mm, length: 20 cm) so as to make cell amounts at the time of the start of the culture substantially the same. Culture was performed at 37°C with shaking, and terminated when glucose contained in the medium was completely consumed (21 to 24 hours). L-cysteine produced in the medium was quantified by the method described by Gaitonde, M.K. (Biochem. J., 104(2):627-33, Aug. 1967). In order to allow the strains to harbor the plasmids, chloramphenicol (25 mg/L) was added over the entire culture period. The experiment was performed in quadruplicate for each strain, and averages and standard deviations for the concentrations (g/L) of the accumulated cysteine are shown in Table 1.

It was revealed that, by enhancing expression of the *cysG* gene coding for the synthetase for siroheme, which is a cofactor of sulfite reductase, and further, by enhancing expression of the *cysJI* genes coding for the subunits of sulfite reductase in addition to the *cysG* gene, production of cysteine by fermentation in *E. coli* using thiosulfate as a sulfur source could be improved.

[Table 1]

**Table 1: Effect of enhancement of expression of genes involved in sulfite reduction in E. coli cysteine-producing bacterium, MG1655int-4M/pACYC-DES**

| Strain | L-cysteine (g/L) |
|---|---|
| MG1655int-4M/pACYC-DES/5JS | 0.28 ± 0.04 |
| MG1655int-4M/pACYC-DES/pMIV-Pnlp23-cysG (Ec) | 0.60 ± 0.01 |
| MG1655int-4M/pACYC-DES/pMIV-Pnlp23-cysGJI (Ec) | 0.51 ± 0.05 |
| MG1655int-4M/pACYC-DES/pMIV-PnlpB-cysG (Pa) | 0.57 ± 0.05 |
| MG1655int-4M/pACYC-DES/pMIV-Pnlp23-cysGJI (Pa) | 0.58 ± 0.01 |

### Example 2: Production of cysteine by P. ananatis overexpressing cysG gene or cysGJI genes using thiosulfate as sulfur source

### (1) Construction of P. ananatis cysteine-producing strain

As a *P*. *ananatis* cysteine-producing strain, there was constructed EYPSint-1MΔd0191(S) strain, in which the *cysEF5* gene, the *yeaS* gene, the *serA348* gene, and the *cysM* gene were introduced into the chromosome, of which expression of the *cysPTWA* gene was enhanced, and of which *d0191* gene was deleted. The construction procedures are shown in (1-1) to (1-5).

### (1-1) Introduction of cysE5 gene and yeaS gene into P. ananatis SC17 strain

The *cysE5* gene and the *yeaS* gene were introduced into the chromosome of the *P*. *ananatis* SC17 strain to construct EY19(s) strain. The *cysE5* gene is a mutant type gene of *cysE*, and it codes for a mutant type serine acetyltransferase (SAT) in which the Val residue and the Asp residue at positions 95 and 96 of the wild-type SAT of *E. coli* are replaced with Arg residue and Pro residue, respectively, so that it is desensitized to feedback inhibition by cysteine (U.S. Patent Published Application No. 20050112731 (A1)). The *yeaS* gene is a gene coding for a cysteine excretion system. The construction procedure is shown below.

First, from the plasmid pMW-Pomp-cysE5 (WO2005/007841) the Pomp-cysE5 cassette portion was excised with *Pae*I and *Sac*I, and inserted into pMIV-5JS at the same restriction site to construct a plasmid pMIV-Pomp-CysE5. pMW-Pomp-cysE5 is a plasmid obtained by inserting the *cysEF5* gene coding for the mutant SAT ligated with the *ompC* gene promoter into pMW118. From the plasmid pACYC184 (GenBank/EMBL accession number X06403, available from NIPPON GENE), the tetracycline resistance gene was excised with *Xba*I and *Eco*88I, and this gene fragment was treated with the Klenow fragment, and then inserted into pMIV-Pomp-CysE5 at the *PvuI* site to construct a plasmid pMT-Pomp-CysE5. Then, pMIV-Pnlp8-YeaS7 constructed in Example 1, (1-1) was digested with *Hind*III, blunt-ended with the Klenow fragment, and then digested with *Nco*I to excise a fragment containing the cassette of the Pnlp8-YeaS-rrnB terminator and the chloramphenicol resistance gene. This fragment was ligated with a *Sma*I and *Nco*I digestion fragment of pMT-Pomp-CysE5 similarly derived from pMIV-5JS to construct a plasmid pMT-EY2. pMT-EY2 has the Pnlp8-YeaS-rrnB terminator cassette and the Pomp-CysE5 cassette on one plasmid.

pMT-EY2 has the attachment sites for Mu phage originated from pMIV-5JS. Therefore, by allowing pMT-EY2 to coexist with the helper plasmid pMH10 having the Mu transposase gene (Zimenkov D. et al., Biotechnologiya (in Russian), 6, 1-22 (2004)) in a host cell, the cassette of PompC-cysE5-Pnlp8-YeaS-rrnB terminator containing the chloramphenicol resistance gene existing on pMT-EY2 between the attachment sites of Mu phage can be inserted into the chromosome of the host. Furthermore, the chloramphenicol resistance gene carried by the pMT-EY2 plasmid exists between two attachment sites of A phage (λattR and λattL), and can be excised and removed by the method described later.

First, the helper plasmid pMH10 was introduced into the *P*. *ananatis* SC17 strain by electroporation, and the strain was cultured overnight at 30°C on the LB agar medium containing 20 mg/L of kanamycin to select a transformant strain into which pMH10 was introduced. The obtained transformant was cultured at 30°C, and pMT-E2 was further introduced into this strain by electroporation. This strain transformed with both pMH10 and pMT-EY2 was subjected to a heat shock with the conditions of 42°C for 20 minutes, and then cultured at 39°C on the LB agar medium containing 20 mg/L of chloramphenicol to select colonies of chloramphenicol resistant strains. About 50 clones of the chloramphenicol resistant strains obtained as described above were each cultured at 39°C for 48 hours on the LB agar medium to eliminate pMH10 and pMT-EY2. Then, there was obtained a strain showing chloramphenicol resistance as a result of the insertion of the cassette of PompC-cysE5-Pnlp8-YeaS-rrnB terminator containing the chloramphenicol resistance gene into the chromosome and showing kanamycin and ampicillin sensitivities as a result of the elimination of the both plasmids. Furthermore, it was confirmed that the objective cassette was inserted into the chromosome of the obtained strain by PCR using the chromosomal DNA of this strain as the template as well as the primers P1 and P6 as primers. All the clones obtained as described above each were designated EY01 to EY50. L-cysteine production culture was performed by using the EY01 to EY50 strains, and the EY19 strain was selected, which was a clone that produced L-cysteine in the largest amount.

The chloramphenicol resistance gene introduced into the EY19 strain was removed with an excision system derived from λ phage. Specifically, the EY19 strain was transformed with pMT-Int-Xis2 (WO2005/010175) carrying the *Int-Xis* gene of λ phage, and the EY19(s) strain showing chloramphenicol sensitivity was obtained from the obtained transformants.

The cysteine production culture can be performed by using the medium and culture method exemplified below.

### [Cysteine production medium] (concentrations of the components are final concentrations)

**Components 1:**

| | |
|---|---|
| (NH₄)₂SO₄ | 15 g/L |
| KH₂PO₄ | 1.5 g/L |
| MgSO₄·H₂O | 1 g/L |
| Thiamine hydrochloride | 0.1 mg/L |

**Components 2:**

| | |
|---|---|
| FeSO₄·7H₂O | 1.7 mg/L |
| Na₂MoO₄·2H₂O | 0.15 mg/L |
| CoCl₂·6H₂O | 0.7 mg/L |
| MnCl·4H₂O | 1.6 mg/L |
| ZnSO₄·7H₂O | 0.3 mg/L |
| CuSO₄·5H₂O | 0.25 mg/L |

**Components 3:**

| | |
|---|---|
| Tryptone | 0.6 g/L |
| Yeast extract | 0.3 g/L |
| NaCl | 0.6 g/L |

**Component 4:**

| | |
|---|---|
| Calcium carbonate | 20 g/L |

**Component 5:**

| | |
|---|---|
| L-Histidine hydrochloride monohydrate 135 mg/L | 135 mg/L |

**Component 6:**

| | |
|---|---|
| Sodium thiosulfate | 4 g/L |

**Component 7:**

| | |
|---|---|
| Pyridoxine hydrochloride | 2 mg/L |

**Component 8:**

| | |
|---|---|
| Glucose | 40 g/L |

For these components, there are prepared stock solutions of 10-fold concentration (Components 1), 1000-fold concentration (Components 2), 100/6-fold concentration (Components 3), 100-fold concentration (Component 5), 1750/3-fold concentration (Component 6), 1000-fold concentration (Component 7), and 20-fold concentration (Component 8), they are mixed at the time of use, and the defined volume is obtained with sterilized water to attain the final concentrations. Sterilization is performed by autoclaving at 110°C for 30 minutes (Components 1, 2, 3, 5 and 8), dry heat sterilization at 180°C for 5 hours or longer (Component 4), or filter sterilization (Components 6 and 7). The L-cysteine production culture can be performed as follows. Each L-cysteine-producing strain is spread on the LB agar medium to perform pre-culture overnight at 34°C, and then cells corresponding to about 7 cm on the plate are scraped with an inoculation loop of 10-µl size (NUNC Blue Loop), and inoculated into 2 ml of the L-cysteine production medium contained in a large test tube (internal diameter: 23 mm, length: 20 cm) so as to make cell amounts at the time of the start of the culture substantially the same. Culture is performed at 32°C with shaking, and terminated when glucose contained in the medium is completely consumed (21 to 24 hours). Cysteine-related compounds produced in the medium are quantified by the method described by Gaitonde, M.K. (Biochem. J., 104(2):627-33, Aug. 1967).

### (1-2) Construction of cysPTWA gene expression-enhanced strain of EY19(s) strain

Then, the promoter locating upstream of the *cysPTWA* gene cluster on the chromosome of the EY19(s) strain was replaced with the aforementioned potent promoter Pnlp8 to construct EYP197(s) strain of which expression of *cysPTWA* genes was enhanced. The *cysPTWA* genes are genes coding for the sulfate/thiosulfate transport system proteins. The construction procedure is shown below.

First, a DNA fragment containing the nlp8 promoter region of about 300 bp was obtained by PCR using pMIV-Pnlp8-YeaS7 as the template as well as the primers P1 and P2. The PCR cycle was as follows: 95°C for 3 minutes, then 2 cycles of 95°C for 60 seconds, 50°C for 30 seconds, and 72°C for 40 seconds, 20 cycles of 94°C for 20 seconds, 59°C for 20 seconds, and 72°C for 15 seconds, and 72°C for 5 minutes as the final cycle.

The amplified DNA fragment containing the nlp8 promoter was treated with the Klenow fragment, and then inserted into the plasmid pMW118-(λattL-KmR-λattR) (WO2006/093322A2) which was digested with *Xba*I and then treated with the Klenow fragment, thereby to obtain a plasmid pMW-Km-Pnlp8. By PCR using pMW-Km-Pnlp8 as the template as well as primer P13 (tccgctcacg atttttttca tcgctggtaa ggtcatttat cccccaggaa aaattggtta, SEQ ID NO: 39) and primer P14 (tttcacaccg ctcaaccgca gggcataacc ggcccttgaa gcctgctttt ttatactaag ttg, SEQ ID NO: 40), a DNA fragment of about 1.6 kb containing the Km-Pnlp8 cassette was amplified. The PCR cycle for this amplification was as follows: 95°C for 3 minutes, then 2 cycles of 95°C for 60 seconds, 50°C for 30 seconds, and 72°C for 40 seconds, 30 cycles of 94°C for 20 seconds, 54°C for 20 seconds, and 72°C for 90 seconds, and 72°C for 5 minutes as the final cycle. On each of the primers, a sequence on the chromosome serving as a target for inserting an objective fragment by the λ-dependent integration (the method called "Red-driven integration" (Proc. Natl. Acad. Sci. USA, 2000, vol. 97, No. 12, pp.6640-6645)) (in this case, a sequence near the promoter of *cysPTWA*) was designed. Therefore, if the obtained DNA fragment is inserted into the objective strain by the λ-dependent integration, there is formed a structure that Km-Pnlp8 is inserted immediately before the *cysPTWA* gene on the chromosome, and the *cysPTWA* gene is ligated with the nlp8 promoter. The nucleotide sequence of the *cysPTWA* gene cluster is shown in SEQ ID NO: 41, and the amino acid sequences encoded by the *cysP, cysT* and *cysW* genes are shown in SEQ ID NOS: 42 to 44, respectively. The nucleotide sequence of the *cysA* gene and the amino acid sequence encoded by this gene are shown in SEQ ID NOS: 45 and 46, respectively. The *cysT* gene is synonymous with the *cysU* gene.

As the host strain for the λ-dependent integration, the *P. ananatis* SC17(0)/RSF-Red-TER strain was used. The *P*. *ananatis* SC17(0)/RSF-Red-TER strain is a host strain for efficiently performing the λ-dependent integration, and it is a strain obtained by introducing the helper plasmid RSF-Red-TER which expresses *gam, bet,* and exo genes of A phage (henceforth referred to as "λ Red genes") into the SC17(0) strain, which is a *P. ananatis* strain resistant to λ phage Red gene products (WO2008/075483). The SC17(0) strain was deposited at the Russian National Collection of Industrial Microorganisms (VKPM), GNII Genetika (address: Russia, 117545 Moscow, 1 Dorozhny proezd. 1) on September 21, 2005 with an accession number of VKPM B-9246. A method for constructing the RSF-Red-TER plasmid is disclosed in detail in WO2008/075483.

The aforementioned SC17(0)/RSF-Red-TER strain was cultured with addition of IPTG for inducing expression of the λ Red genes to prepare cells for electroporation. The aforementioned DNA fragment containing the Km-Pnlp8 cassette was introduced into these cells by electroporation, and a recombinant strain in which the nlp8 promoter was inserted upstream of the *cysPTWA* genes by the λ-dependent integration was obtained by using the kanamycin resistance as a marker. By PCR using the chromosomal DNA of the obtained strain as the template, as well as primer P15 (ctttgtccct ttagtgaagg, SEQ ID NO: 47) and primer P16 (agctgatcta gaagctgact cgagttaatg gcctcccaga cgac, SEQ ID NO: 48) as the primers, it was confirmed that the objective structure, Km-Pnlp8-*cysPTWA*, was formed, and this strain was designated SC17(0)-Pnlp8-PTWA strain.

Then, the chromosomal DNA of the SC17(0)-Pnlp8-PTWA strain was purified, and 10 µg of this chromosomal DNA was introduced into the EY19(s) strain by electroporation to obtain a kanamycin resistant strain. Amplification was performed by PCR using the chromosomal DNA of the obtained strain as the template as well as the primers P15 and P16 as the primers to confirm that the structure of Km-Pnlp8-*cysPTWA* had been introduced into the chromosome of the EY19(s) strain. The strain obtained as described above was designated as EYP197 strain. Furthermore, the kanamycin resistance gene was removed from the chromosome by using pMT-Int-Xis2 as described above, and the strain that became kanamycin sensitive was designated as EYP197(s) strain.

### (1-3) Introduction of mutant type 3-phosphoglycerate dehydrogenase gene (serA348) into EYP197(s) strain

The *serA348* gene was introduced into the chromosome of the EYP197(s) strain to construct EYPS1976(s) strain. The *serA348* gene is a mutant type gene of *serA,* and it codes for a mutant type 3-phosphoglycerate dehydrogenase in which the asparagine residue at position 348 of the wild-type 3-phosphoglycerate dehydrogenase of *Pantoea ananatis* is replaced with alanine residue, so that it is desensitized to feedback inhibition by serine (J. Biol. Chem., 1996, 271 (38):23235-8). The construction procedure is shown below.

The nucleotide sequence of the wild-type *serA* gene derived from *Pantoea ananatis* is shown in SEQ ID NO: 49, and the amino acid sequence encoded by this gene is shown in SEQ ID NO: 50. In order to obtain a 3'-end side DNA fragment of the *serA* gene into which the aforementioned mutation (N348A) was introduced, PCR was performed by using the chromosomal DNA of the SC17 strain as the template as well as primer P17 (agctgagtcg acatggcaaa ggtatcactg gaa, SEQ ID NO: 51) and primer P18 (gagaacgccc gggcgggctt cgtgaatatg cagc, SEQ ID NO: 52) as the primers (95°C for 3 minutes, then 2 cycles of 95°C for 60 seconds, 50°C for 30 seconds, and 72°C for 40 seconds, 25 cycles of 94°C for 20 seconds, 60°C for 20 seconds, and 72°C for 60 seconds, and 72°C for 5 minutes as the final cycle). Similarly, in order to obtain a 5'-end side DNA fragment into which the aforementioned mutation (N348A) was introduced, PCR was performed by using the chromosomal DNA of the SC17 strain as the template as well as primer P19 (agctgatcta gacgtgggat cagtaaagca gg, SEQ ID NO: 53) and primer P20 (aaaaccgccc gggcgttctc ac, SEQ ID NO: 54) as the primers (95°C for 3 minutes, then 2 cycles of 95°C for 60 seconds, 50°C for 30 seconds, and 72°C for 40 seconds, 20 cycles of 94°C for 20 seconds, 60°C for 20 seconds, and 72°C for 20 seconds, and 72°C for 5 minutes as the final cycle). Both the obtained PCR fragments were treated with the restriction enzyme *Sma*I, and ligated by using a DNA ligase to obtain a DNA fragment corresponding to the full length of mutant *serA* gene including the objective mutation (N348A). PCR was performed by using this DNA fragment as the template as well as the primers P17 and P19 as the primers (95°C for 3 minutes, then 2 cycles of 95°C for 60 seconds, 50°C for 30 seconds, and 72°C for 40 seconds, 15 cycles of 94°C for 20 seconds, 60°C for 20 seconds, and 72°C for 75 seconds, and 72°C for 5 minutes as the final cycle). The *Sal*I and *Xba*I sites were designed at the 5'-ends of the P17 and P19 primers, respectively. The obtained fragment was treated with *Sal*I and *Xba*I, and then inserted into pMIV-Pnlp8-ter similarly treated with *Sal*I and *Xba*I, thereby to prepare pMIV-Pnlp8-serA348 in which the *serA348* gene was cloned.

The constructed pMIV-Pnlp8-serA348 carries the attachment sites of Mu phage originated from pMIV-5JS. Therefore, by using the helper plasmid pMH10 having the Mu transposase gene, the cassette of Pnlp8-serA348-rrnB terminator including the chloramphenicol resistance marker existing on pMIV-Pnlp8-serA348 between the attachment sites of Mu phage can be inserted into the chromosome of the host, as described above.

pMIV-Pnlp8-serA348 and pMH10 were introduced into the SC17(0) strain in the same manner as that used for the introduction of the cassette of PompC-cysE5-Pnlp8-YeaS-rrnB terminator in (1-1) to obtain a strain in which the cassette of Pnlp8-serA348-rrnB terminator was inserted into the chromosome. By PCR using the primers P1 and P19, it was confirmed that the objective cassette was inserted into the chromosomes of the obtained strains. The 3-phosphoglycerate dehydrogenase activity in cell extracts of 50 clones thus obtained was measured, and a strain which showed the highest activity was selected, and designated as SC17int-serA348 strain. Then, 10 µg of the chromosomal DNA of the SC17int-serA348 strain was introduced into the EYP197(s) strain by electroporation to obtain a chloramphenicol resistant strain, and by PCR using the primers P1 and P19, it was confirmed that the structure of Pnlp8-serA348 had been introduced together with the chloramphenicol resistance gene into the chromosome of the EYP197(s) strain. The strain obtained as described above was designated as EYPS1976 strain. By the aforementioned method for removing marker using pMT-Int-Xis2, the chloramphenicol resistance gene was removed from the chromosome, and the strain that became chloramphenicol sensitive was designated as EYPS1976(s) strain.

### (1-4) Introduction of O-acetyl-L-serine sulfhydrylase B gene (cysM) into EYPS1976(s) strain

The O-acetyl-L-serine sulfhydrylase B gene (*cysM*) was introduced into the chromosome of the EYPS1976(s) strain to construct EYPSint-1M(s) strain. The construction procedure is shown below.

First, by PCR using the plasmid pMIV-Pnlp4-ter constructed in Example 1, (2-2) as the template as well as primer P9 (agctgaaagc ttgcatgcac gcgtggcgat ctggcctgac tgc, SEQ ID NO: 32) and primer P21 (agctgagtcg acnnngtggt ggcaaccttt aaaaaactg ("n" means that the corresponding residue can be any of a, t, g and c), SEQ ID NO: 55) as the primers (95°C for 5 minutes, then 27 cycles of 94°C for 20 seconds, 59°C for 20 seconds, and 72°C for 20 seconds, and 72°C for 5 minutes as the final cycle), a DNA fragment in which the -7 to -9 region contained in the 5'-end side of the nlpD promoter was randomized was obtained. The "-7 to -9 region" means the positions of 7th to 9th nucleotides on the 5' side from the transcription initiation site of the nlpD promoter. The obtained fragments each were treated with *Sal*I and *Pae*I, and inserted into pMIV-Pnlp0-ter treated with the same enzymes to obtain plasmids in each of which Pnlp0 of pMIV-Pnlp0-ter was replaced with mutant type Pnlp. One of those plasmids was used as pMIV-Pnlp1-ter. The nucleotide sequence of the *Pae*I*-Sal*I fragment of the Pnlp1 promoter inserted into this plasmid is as shown in SEQ ID NO: 56.

The *cysM* gene cloned from the *E. coli* MG1655 strain was incorporated into pMIV-Pnlp1-ter. Specifically, a *cysM* gene fragment was amplified by PCR using the genome of the *E. coli* MG1655 strain as the template as well as primer P22 (agctgagtcg acgtgagtac attagaacaa acaat, SEQ ID NO: 57) and primer P23 (agctgatcta gaagtctccg atgctattaa tcc, SEQ ID NO: 58) as the primers (95°C for 5 minutes, then 30 cycles of 98°C for 5 seconds, 50°C for 10 seconds, and 72°C for 60 seconds, and 72°C for 2 minutes as the final cycle). The DNA fragment obtained as described above was treated with *Sal*I and *Xba*I, and inserted into pMIV-Pnlp1-ter treated with the same enzymes to prepare a plasmid pMIV-Pnlp1-CysM in which the *cysM* gene of the *E. coli* MG1655 strain was cloned. The nucleotide sequence of the *cysM* gene of the *E*. *coli* MG1655 strain is shown in SEQ ID NO: 35, and the amino acid sequence encoded by this gene is shown in SEQ ID NO: 36.

By using pMIV-Pnlp1-CysM constructed as described above, a *P. ananatis* SC17 strain in which a cassette comprising the *cysM* gene was inserted into the chromosome was obtained according to the aforementioned method using the helper plasmid pMH10, and designated as SC17int-1M. The chromosomal DNA was extracted from SC17int-1M. This chromosomal DNA in an amount of 10 µg was introduced into the EYP1976(s) strain by electroporation to obtain a chloramphenicol resistant strain, and the obtained strain was designated as EYPSint-1M. Further, the chloramphenicol resistance gene was removed from the chromosome by the aforementioned method for eliminating marker using pMT-Int-Xis2, and the strain that became chloramphenicol sensitive was designated as EYPSint-1M(s) strain.

### (1-5) Construction of strain deficient in cysteine decomposition system gene d0191 from EYPSint-1M strain

The *d0191* gene coding for a protein having the cysteine desulfhydrase activity was deleted in the EYPSint-1M(s) strain to construct EYPSint-1MΔd0191(s) strain. The construction procedure is shown below.

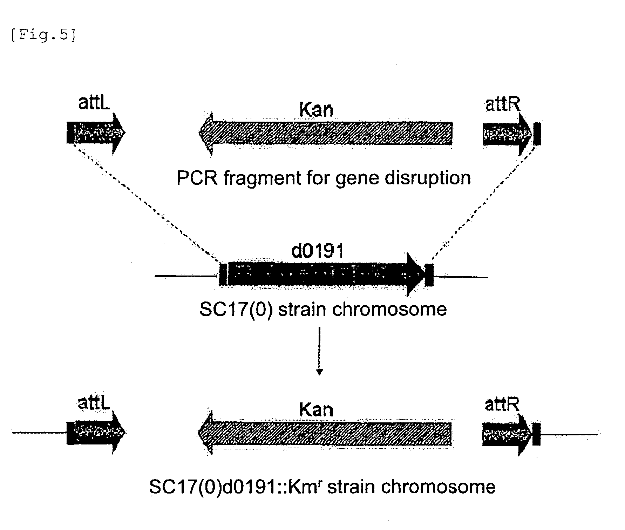

The *d0191* gene was deleted by the λ-dependent integration described in (1-2). The outline thereof is shown in Fig. 5. A DNA fragment having a structure in which the kanamycin resistance gene was present between the homologous sequences of the both ends of the *d0191* gene (PCR fragment for gene disruption, Fig. 5) was obtained by PCR. As the primers, primer Dd0191-FW (CCGTGTCTGAAGCCTATTTTGCCCGCCTGCTGGGCTTGCCTTTTATTGCCTGAAGCCT GCTTTTTTATACTAAGTTGGCA, SEQ ID NO: 59), and primer Dd0191-RV (CTAGCCCAGTTCGCGCTGCCAGGGCGTAATATCGCCAATGTGCTCGGCAACGCTCAAG TTAGTATAAAAAAGCTGAACGA, SEQ ID NO: 60) were used, and as the template, the plasmid pMW118-(λattL=Kmr-λattR) was used. PCR was performed by using LA-Taq polymerase (Takara) in a standard reaction mixture composition described in the protocol with a PCR cycle of 94°C for 5 minutes, and following 30 cycles of 98°C for 5 seconds, 55°C for 5 seconds, and 72°C for 2 minutes and 30 seconds. The obtained DNA fragment was introduced into the *P. ananatis* SC17(0)/RSF-Red-TER strain by electroporation to obtain a kanamycin resistant strain. It was confirmed that the obtained kanamycin resistant strain was a *d0191* gene-disrupted strain in which the *d0191* gene was replaced with the kanamycin resistant cassette by the homologous sequences of the *d0191* gene region existing on the both ends of the DNA fragment (SC17(0)d0191::Kmr strain, Fig. 5).

The chromosomal DNA was extracted from the SC17(0)d0191::Kmr strain. This chromosomal DNA in an amount of 10 μg was introduced into the EYPSint-1M(s) strain by electroporation to obtain a kanamycin resistant strain, and the obtained strain was designated as EYPSint-1MΔd0191. Further, the kanamycin resistance gene was removed from the chromosome by the aforementioned method for eliminating marker using pMT-Int-Xis2, and the strain that became kanamycin sensitive was designated as EYPSint-1MΔd0191(s) strain. This strain was used in the following experiment as a *P. ananatis* cysteine-producing bacterium.

### (2) Cysteine production by P. ananatis overexpressing cysG gene or cysGJI genes using thiosulfate as sulfur source

There were constructed strains of the above-obtained *P. ananatis* cysteine-producing bacterium, EYPSint-1MΔd0191(s), into which the *cysG* overexpression plasmids pMIV-Pnlp23-cysG (Pa) and pMIV-Pnlp23-*cysG* (Ec), the *cysG* and *cysJI* coexpression plasmids pMIV-Pnlp23-cysGJI (Pa) and pMIV-Pnlp23-cysGJI (Ec), and pMIV-5JS as a control were each introduced. Cysteine production culture was performed with these strains, and cysteine production amounts were compared. For the cysteine production culture, a cysteine production medium having the following composition containing glucose as a carbon source and thiosulfate as a sulfur source was used.

### [Cysteine production medium] (concentrations of the components are final concentrations)

**Components 1:**

| | |
|---|---|
| NH₄Cl | 12.1 g/L |
| KH₂PO₄ | 1.5 g/L |
| MgSO₄·7H₂O | 1 g/L |
| Thiamine hydrochloride | 0.1 mg/L |

**Components 2:**

| | |
|---|---|
| FeSO₄·7H₂O | 1.7 mg/L |
| Na₂MoO₄·2H₂O | 0.15 mg/L |
| CoCl₂·6H₂O | 0.7 mg/L |
| MnCl·4H₂O | 1.6 mg/L |
| ZnSO₄·7H₂O | 0.3 mg/L |
| CuSO4·5H₂O | 0.25 mg/L |

**Components 3:**

| | |
|---|---|
| Tryptone | 0.6 g/L |
| Yeast extract | 0.3 g/L |
| NaCl | 0.6 g/L |

**Component 4:**

| | |
|---|---|
| Calcium carbonate | 20 g/L |

**Component 5:**

| | |
|---|---|
| L-Histidine hydrochloride monohydrate | 135 mg/L |

**Component 6:**

| | |
|---|---|
| Sodium thiosulfate | 0.6 g/L |

**Component 7:**

| | |
|---|---|
| Pyridoxine hydrochloride | 2 mg/L |

**Component 8:**

| | |
|---|---|
| Glucose | 20 g/L |

For these components, there were prepared stock solutions of 10-fold concentration (Components 1), 1000-fold concentration (Components 2), 100/6-fold concentration (Components 3), 100-fold concentration (Component 5), 1750/3-fold concentration (Component 6), 1000-fold concentration (Component 7), and 20-fold concentration (Component 8), they were mixed at the time of use, and the defined volume was obtained with sterilized water to attain the final concentrations. Sterilization was performed by autoclaving at 110°C for 30 minutes (Components 1, 2, 3, 5 and 8), dry heat sterilization at 180°C for 5 hours or longer (Component 4), or filter sterilization (Components 6 and 7). The L-cysteine production culture was performed as follows. Each L-cysteine-producing strain was spread on the LB agar medium to perform pre-culture overnight at 34°C, and then cells corresponding to about 7 cm on the plate were scraped with an inoculation loop of 10-µl size (NUNC Blue Loop), and inoculated into 2 ml of the L-cysteine production medium contained in a large test tube (internal diameter: 23 mm, length: 20 cm) so as to make cell amounts at the time of the start of the culture substantially the same. Culture was performed at 32°C with shaking, and terminated when glucose contained in the medium was completely consumed (21 to 24 hours). L-cysteine produced in the medium was quantified by the method described by Gaitonde, M.K. (Biochem. J., 104(2):627-33, Aug. 1967). In order to allow the strains to harbor the plasmids, chloramphenicol (25 mg/L) was added over the entire culture period. The experiment was performed in quadruplicate·for each strain, and the concentrations (g/L) of the accumulated cysteine are shown in Table 2.

It was revealed that, by enhancing expression of the *cysG* gene coding for the synthetase for siroheme, which is a cofactor of sulfite reductase, and further, by enhancing expression of the *cysJI* genes coding for the subunits of sulfite reductase in addition to the *cysG* gene, production of cysteine by fermentation in *P. ananatis* using thiosulfate as a sulfur source could be improved.

[Table 2]

**Table 2: Effect of enhancement of expression of genes involved in sulfite reduction in P. ananatis cysteine-producing bacterium, EYPSint-1MΔd0191(s)**

| Strain | L-cysteine (g/L) |
|---|---|
| EYPSint-1MΔd0191(s)/pMIV-5JS | 0.26 ± 0.01 |
| EYPSint-1MΔd0191(s)/pMIV-cysG (Pa) | 0.49 ± 0.01 |
| EYPSint-1MΔd0191(s)/pMIV-cysGJI (Pa) | 0.54 ± 0.03 |
| EYPSint-1MΔd0191(s)/pMIV-cysG (EC) | 0.49 ± 0.05 |
| EYPSint-1MΔd0191(s)/pMIV-cysGJI (EC) | 0.48 ± 0.03 |

### Industrial Applicability

According to the present invention, L-cysteine-producing ability of a bacterium can be improved when thiosulfate is used as a sulfur source. Further, according to the present invention, L-cysteine, a related substance thereof, or a mixture thereof can be efficiently produced when thiosulfate is used as a sulfur source.

Explanation of Sequence Listing
SEQ ID NO: 1: Nucleotide sequence of Pnlp0
SEQ ID NO: 2: Nucleotide sequence of Pnlp23
SEQ ID NOS: 3 and 4: Primers for amplification of *P*. *ananatis cysG* gene
SEQ ID NOS: 5 and 6: Primers for amplification of *P*. *ananatis cysJI* genes
SEQ ID NO: 7: Primer for amplification of *P*. *ananatis cysGJI* genes
SEQ ID NOS: 8 and 9: Primers for amplification of *E. coli cysG* gene
SEQ ID NOS: 10 and 11: Primers for amplification of *E. coli cysJI* genes
SEQ ID NO: 12: Primer for amplification of *E. coli cysGJI* genes
SEQ ID NOS: 13 to 20: Primers P1 to P8
SEQ ID NO: 21: Nucleotide sequence of Pnlp8
SEQ ID NOS: 22 and 23: Primers for amplification of *E. coli ydeD* gene
SEQ ID NOS: 24 to 27: Primers for amplification of *cysEX* gene
SEQ ID NOS: 28 and 29: Primers for amplification of *serA*5 gene
SEQ ID NOS: 30 and 31: Primers for amplification of promoter PompA
SEQ ID NOS: 32 and 33: Primers P9 and P10
SEQ ID NO: 34: Nucleotide sequence of Pnlp4
SEQ ID NO: 35: Nucleotide sequence of *E. coli cysM* gene
SEQ ID NO: 36: Amino acid sequence of *E. coli* CysM protein
SEQ ID NOS: 37 to 40: Primers P11 to P14
SEQ ID NO: 41: Nucleotide sequence of *E*. *coli cysPTWA* gene cluster
SEQ ID NO: 42: Amino acid sequence of *E*. *coli* CysP protein
SEQ ID NO: 43: Amino acid sequence of *E. coli* CysT protein
SEQ ID NO: 44: Amino acid sequence of *E*. *coli* CysW protein
SEQ ID NO: 45: Amino acid sequence of *E*. *coli cysA* gene
SEQ ID NO: 46: Amino acid sequence of *E*. *coli* CysA protein
SEQ ID NOS: 47 and 48: Primers P15 and P16
SEQ ID NO: 49: Nucleotide sequence of *P. ananatis serA* gene
SEQ ID NO: 50: Amino acid sequence of *P. ananatis* SerA protein
SEQ ID NOS: 51 to 55: Primers P17 to P21
SEQ ID NO: 56: Nucleotide sequence of Pnlp1
SEQ ID NOS: 57 and 58: Primers P22 and P23
SEQ ID NOS: 59 and 60: Primers for amplification of *P*. *ananatis d0191* gene
SEQ ID NO: 61: Nucleotide sequence of *E. coli cysJ* gene
SEQ ID NO: 62: Amino acid sequence of *E. coli* CysJ protein
SEQ ID NO: 63: Nucleotide sequence of *P. ananatis cysJ* gene
SEQ ID NO: 64: Amino acid sequence of *P. ananatis* CysJ protein
SEQ ID NO: 65: Nucleotide sequence of *E. coli cysI* gene
SEQ ID NO: 66: Amino acid sequence of *E. coli* CysI protein
SEQ ID NO: 67: Nucleotide sequence of *P. ananatis cysI* gene
SEQ ID NO: 68: Amino acid sequence of *P. ananatis* CysI protein
SEQ ID NO: 69: Nucleotide sequence of *E. coli cysG* gene
SEQ ID NO: 70: Amino acid sequence of *E. coli* CysG protein
SEQ ID NO: 71: Nucleotide sequence of *P. ananatis cysG* gene
SEQ ID NO: 72: Amino acid sequence of *P. ananatis* CysG protein
SEQ ID NO: 73: Nucleotide sequence of Pnlp0 including ligation site for downstream gene
SEQ ID NO: 74: Nucleotide sequence of Pnlp8 including ligation site for downstream gene

## Claims

1. A method for producing L-cysteine, a related substance thereof, or a mixture thereof, which comprises:
culturing a bacterium belonging to the family *Enterobacteriaceae* in a medium containing thiosulfate, which bacterium has L-cysteine-producing ability and is modified so that expression of the *cysG* gene is increased by increasing copy number of the gene, or by modifying an expression control sequence of the gene; and
collecting L-cysteine, a related substance thereof, or a mixture thereof accumulated in the medium,
wherein the related substance is selected from the group consisting of L-cystine, S-sulfocysteine, a thiazolidine derivative, a hemithioketal, γ-glutamylcysteine, glutathione, cystathionine, homocysteine, L-methionine, and S-adenosylmethionine.

2. The method according to claim 1, wherein the bacterium is further modified so that expression of a gene selected from the group consisting of the *cysJ* gene and the *cysI* gene by increasing copy number of the gene, or by modifying an expression control sequence of the gene.

3. The method according to claim 2, wherein expression of the *cysG* gene, the *cysJ* gene, and the *cysI* gene is increased.

4. The method according to any one of claims 1 to 3, wherein the bacterium is a bacterium belonging to the genus *Pantoea.*

5. The method according to claim 4, wherein the bacterium is *Pantoea ananatis.*

6. The method according to any one of claims 1 to 3, wherein the bacterium is a bacterium belonging to the genus *Escherichia.*

7. The method according to claim 6, wherein the bacterium is *Escherichia coli.*

8. The method according to any one of claims 1 to 7, wherein the bacterium further has a characteristic selected from the group consisting of the following characteristics (1) and (2):
(1) activity of an enzyme involved in the biosynthesis of L-cysteine is enhanced,
(2) expression of a gene coding for a protein involved in secretion of L-cysteine is enhanced.

9. The method according to any one of claims 1 to 8, wherein the related substance is L-cystine or a thiazolidine derivative.

## Patentansprüche

1. Verfahren zum Herstellen von L-Cystein, einer damit verwandten Substanz oder eines Gemisches davon, welches umfasst:
das Kultivieren eines Bakteriums der Familie *Enterobacteriaceae* in einem Thiosulfat enthaltenden Medium, wobei das Bakterium die Fähigkeit zur Produktion von L-Cystein aufweist und so modifiziert ist, dass die Expression des cysG-Gens erhöht ist, indem die Kopienzahl des Gens erhöht ist oder die Expressionskontrollsequenz des Gens modifiziert ist; und
das Gewinnen von L-Cystein, einer damit verwandten Substanz oder eines Gemisches davon, das in dem Medium angehäuft ist, wobei die verwandte Substanz aus der Gruppe ausgewählt ist, die aus L-Cystein, S-Sulfocystein, einem Thiazolidinderivat, einem Hemithioketal, γ-Glutamylcystein, Glutathion, Cystathion, Homocystein, L-Methionin und S-Adenosylmethionin besteht.

2. Verfahren nach Anspruch 1, wobei das Bakterium außerdem so modifiziert ist, dass die Expression eines Gens, das aus der Gruppe ausgewählt ist, die aus dem cysJ-Gen und dem cysI-Gen besteht, erhöht ist, indem die Kopienzahl des Gens erhöht ist oder eine Expressionskontrollsequenz des Gens modifiziert ist.

3. Verfahren nach Anspruch 2, wobei die Expression des cysG-Gens, des cysJ-Gens und des cysI-Gens erhöht ist.

4. Verfahren nach einem der Ansprüche 1 bis 3, wobei das Bakterium ein Bakterium der Gattung *Pantoea* ist.

5. Verfahren nach Anspruch 4, wobei das Bakterium *Pantoea Ananatis* ist.

6. Verfahren nach einem der Ansprüche 1 bis 3, wobei das Bakterium ein Bakterium der Gattung *Escherichia* ist.

7. Verfahren nach Anspruch 6, wobei das Bakterium *Escherichia coli* ist.

8. Verfahren nach einem der Ansprüche 1 bis 7, wobei das Bakterium außerdem eine Eigenschaft aufweist, die aus der Gruppe ausgewählt ist, die aus den folgenden Eigenschaften (1) und (2) besteht:
(1) die Aktivität eines Enzyms, das an der Biosynthese von L-Cystein beteiligt ist, ist erhöht,
(2) die Expression eines Gens, das für ein an der Ausscheidung von L-Cystein beteiligtes Protein kodiert, ist erhöht.

9. Verfahren nach einem der Ansprüche 1 bis 8, wobei die verwandte Substanz L-Cystin oder ein Thiazolidinderivat ist.

## Revendications

1. Procédé de production de L-cystéine, d'une substance liée de celle-ci, ou d'un mélange de celles-ci, lequel comprend :
la culture d'une bactérie appartenant à la famille *Enterobacteriaceae* dans un milieu contenant du thiosulfate, laquelle bactérie présente une aptitude à produire de la L-cystéine et est modifiée de sorte que l'expression du gène *cysG* est élevée par augmentation de nombre de copies du gène ou par modification d'une séquence de contrôle d'expression du gène ; et
recueil de L-cystéine, d'une substance liée à celle-ci, ou d'un mélange de celles-ci accumulé dans le milieu,
dans lequel la substance liée est choisie dans le groupe constitué de L-cystine, S-sulfocystéine, d'un dérivé de thiazolidine, d'un hémithiocétal, de γ-glutamylcystéine, de glutathionne, de cystathionine, d'homocystéine, de L-méthionine, et S-adénosylméthionine.

2. Procédé selon la revendication 1, dans lequel la bactérie est encore modifiée de sorte que l'expression d'un gène choisi dans le groupe constitué du gel *cisJ* et du gène *cisI* en élevant le nombre de copies du gène ou en modifiant une séquence de contrôle d'expression du gène.

3. Procédé selon la revendication 2, dans lequel l'expression du gène *cisG,* du gène *cisJ,* et du gène *cisI* est élevée.

4. Procédé selon l'une quelconque des revendications 1 à 3, dans lequel la bactérie est une bactérie appartenant au genre *Pantoea.*

5. Procédé selon la revendication 4, dans lequel la bactérie est *Pantoea ananatis*

6. Procédé selon l'une quelconque des revendications 1 à 3, dans lequel la bactérie est une bactérie appartenant au genre *Escherichia.*

7. Procédé selon la revendication 6, dans lequel la bactérie est *Escherichia.*

8. Procédé selon l'une quelconque des revendications 1 à 7, dans lequel la bactérie présente de plus une caractéristique choisie dans le groupe constitué des caractéristiques (1) et (2) :
(1) l'activité d'une enzyme impliquée dans la biosynthèse L-cystéine est promue,
(2) l'expression d'un gène codant pour une protéine impliquée dans la sécrétion de L-cystéine est promue.

9. procédé selon l'une quelconque des revendications 1 à 8, dans lequel la substance liée est la L-cystine ou un dérivé de thiazolidine.
